# EUROPEAN PATENT APPLICATION

(11) **EP 4 578 863 A1**
(43) Date of publication of application: **02.07.2025**
(21) Application number: 23869867.4
(22) Date of filing: 30.06.2023
(51) Int. Cl.: C07F 5/02, C09K 11/06

(54) **ORGANIC COMPOUND AND PREPARATION METHOD THEREFOR, AND USE OF ORGANIC COMPOUND**

(30) Priority: 30.09.2022 CN 202211217040
(71) Applicant: Huawei Technologies Co., Ltd., Shenzhen, Guangdong 518129 (CN); Zhejiang Brilliant Optoelectronic Technology Co., Ltd., Taizhou, Zhejiang 318020 (CN)
(72) Inventor: GAN, Lin, Shenzhen, Guangdong 518129 (CN); CHEN, Huaijun, Taizhou, Zhejiang 318020 (CN); WU, Jiang, Shenzhen, Guangdong 518129 (CN); TAN, Jiahui, Taizhou, Zhejiang 318020 (CN); PAN, Junyou, Taizhou, Zhejiang 318020 (CN); CHEN, Jinpeng, Taizhou, Zhejiang 318020 (CN)
(74) Representative: Körber, Martin Hans
(86) International application number: PCT/CN2023/105094
(87) International publication number: WO 2024/066619

(57) **Abstract**

This application provides an organic compound. The organic compound has a structural formula 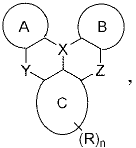 where at least one of a ring A, ring A-Y, a ring B, ring B-Z, and R has a structure 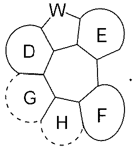 This application further provides a method for preparing the organic compound and use thereof. The organic compound is used in an organic device. The device using the organic compound has high light-emitting efficiency, a narrow FWHM on a luminescence spectrum, a long service life, and the like.

## Description

### CROSS-REFERENCE TO RELATED APPLICATIONS

This application claims priority to Chinese Patent Application No. 202211217040.2, filed with the China National Intellectual Property Administration on September 30, 2022 and entitled "ORGANIC COMPOUND AND PREPARATION METHOD THEREOF, AND USE OF ORGANIC COMPOUND", which is incorporated herein by reference in its entirety.

### TECHNICAL FIELD

This application relates to the field of organic light-emitting material technologies, and in particular, to an organic compound, a method for preparing the organic compound, and use of the organic compound.

### BACKGROUND

Organic semiconductor materials have great potential in optoelectronic devices, especially in organic light-emitting diodes (OLEDs), due to their variety in chemical synthesis, low manufacturing costs in large-scale production, excellent optical and electrical properties, and the like.

A common organic electroluminescent device (for example, an OLED light-emitting device) has a multi-layer structure including two electrode film layers and a plurality of organic material layers such as a hole injection layer, a hole transport layer, a light-emitting layer, an electron transport layer, and an electron injection layer that are stacked between the two electrode film layers. When a voltage is applied to electrodes at both ends of the OLED light-emitting device as an electroluminescent device, holes are injected into the organic material layers from a positive electrode, and electrons are injected into the organic material layers from a negative electrode. An exciton is formed when an injected hole and an electron meet. Light is emitted when the exciton returns to a ground state through transition. This type of organic electroluminescent devices are characterized by self-luminescence, high brightness, high efficiency, low drive voltages, wide angles of view, high contrast, high responsivity, and the like.

Existing light-emitting materials for organic light-emitting displays mainly include fluorescent materials, phosphorescent materials, thermally activated delayed fluorescence (TADF) materials, and the like. However, the foregoing conventional light-emitting materials have at least the following disadvantages: a broad full width at half maximum (FWHM) on a luminescence spectrum, low color purity for display effect of a device, difficulty in providing deep blue light, failure in meeting requirements of display techniques for a high color rendering index, and low light-emitting efficiency of the device, which are not conducive to achieving low power consumption, a long service life, and the like of the display device.

### SUMMARY

In view of this, to resolve at least one of the foregoing disadvantages, it is necessary to provide an organic compound, a method for preparing the organic compound, and use of the organic compound. The organic compound is used in an organic device, and therefore the organic device can have high light-emitting efficiency, a narrow FWHM on a luminescence spectrum, a long service life, and the like.

A first aspect of embodiments of this application provides an organic compound, where the organic compound has a structural formula shown in formula (I): 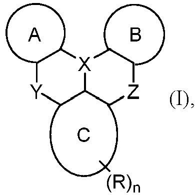 where at least one of a ring A, a ring A-Y structure, a ring B, a ring B-Z structure, and R has a structure shown in formula (II): 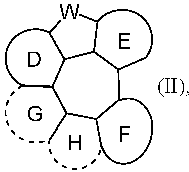 where
a ring G and a ring H in the structure shown in formula (II) are absent or at least one of the ring G and the ring H is present;
the ring A, the ring B, a ring C, a ring D, a ring E, a ring F, the ring G, and the ring H are each independently selected from substituted or unsubstituted aryl, substituted or unsubstituted heteroaryl, or a substituted or unsubstituted fused-ring structure;
W is selected from C(R₁, R₂), N-R₃, P, P=O, or Al;
X is selected from B, N, P, P=O, or Al;
Y and Z are each independently selected from C=O, N-R₄, O, S, Se, P, P=O, or P=S, and when Y and Z are both present, at least one of Y and Z is N-R₄;
R and R₁ to R₄ are each independently selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms; and
n is an integer greater than or equal to 0.

In embodiments of this application, a new organic compound based on a boron-nitrogen-ring-like structure is synthesized. The site X is at a para-position of the sites Y, Z, and W separately on a same conjugated six-membered ring, so that X with Y, Z, and W separately generates hyperconjugation effect with multiple conjugation effects, achieving localization of a highest occupied molecular orbital (Highest Occupied Molecular Orbital, HOMO) and a lowest unoccupied molecular orbital (Lowest Unoccupied Molecular Orbital, LUMO) on the ring A, the ring B, and the ring C, resulting in efficient reverse intersystem crossing, thereby improving light-emitting efficiency (up to 100%) of the new organic compound. A structure shown in the definition formula (I) without the structure shown in formula (II) introduced is a basic ring structure. In this application, by three methods: (1) introducing the ring structure shown in formula (II) into a framework of the basic ring structure, and/or (2) introducing the ring structure shown in formula (II) as a modifying group into the basic ring structure on a specific site, based on the organic compound, a color of light is adjusted, a full width at half maximum (FWHM) on a luminescence spectrum is narrowed, light-emitting efficiency is improved, a luminescence lifetime is prolonged, and the like.

For (1), the ring structure shown in formula (II) is introduced into the framework of the basic ring structure, making the ring structure shown in formula (II) a part of the framework of the basic ring structure on a rigid conjugation plane. In addition, introduction of the ring structure shown in formula (II) can increase a conjugation length of the entire molecule. An electron cloud on the ring structure shown in formula (II) is also involved in super-resonance effect of the entire molecule. This can effectively reduce an FWHM on a luminescence spectrum of the organic compound, and can cause a red shift to a sufficient degree on the spectrum of the organic compound relative to that of the basic ring structure, to adjust a color of light from a blue light region to a green light region to emit green light with a high color rendering index. Moreover, the ring D, the ring E, the ring F, the ring G, and W in the ring structure shown in formula (II) are fused to form rings, which reduces a number of free radicals in the ring structure shown in formula (II), effectively suppressing vibrational and rotational freedom of the ring structure shown in formula (II), improving planarity of the molecules of the organic compound, and further narrowing the FWHM on the luminescence spectrum of the organic compound.

For (2), because the ring structure shown in formula (II) is introduced as the modifying group into the basic ring structure on the specific site, a frontier orbital energy level of the organic compound can be effectively adjusted, and multiple resonance effect of the basic ring structure on the rigid conjugation plane can be enhanced based on a strong electron-donating ability of the ring structure shown in formula (II). In addition, rotation and vibration of the ring structure shown in formula (II) can be effectively suppressed, so that a color of light is adjusted and an FHWM on a luminescence spectrum is narrowed, thereby implementing highly efficient narrow-FHMW blue light emission.

With reference to the first aspect, in some embodiments, the ring A, the ring B, the ring C, the ring D, the ring E, the ring F, the ring G, and the ring H are each independently selected from substituted or unsubstituted C6-C30 aryl, substituted or unsubstituted C5-C30 heteroaryl, or a substituted or unsubstituted C10-C30 fused-ring structure.

With reference to the first aspect, in some embodiments, each time when R₄ is present, R₄ is linked to or fused with the ring A or ring B adjacent thereto to form a ring.

By bonding and fusing the substituent (R₄) on at least one of Y and Z with the adjacent ring A or ring B to form a ring, a conjugation length of the organic compound can be further increased, vibrational and rotational freedom of free substituents on Y, Z, the ring A, and the ring B can be further effectively suppressed, and planarity of the molecules of the organic compound can be further improved, thereby further improving light-emitting efficiency, and further narrowing an FWHM on a luminescence spectrum of the organic compound.

With reference to the first aspect, in some embodiments, each time when at least two substituents are present in the ring A, the ring B, the ring C, the ring D, the ring E, the ring F, the ring G, and the ring H, at least two adjacent substituents in the substituents are linked or fused together to form a ring.

At least two adjacent substituents on the ring A, the ring B, the ring C, the ring D, the ring E, the ring F, and the ring G are linked or fused together and jointly form a ring structure (such as an aryl ring or a heteroaryl ring or a fused-ring structure) with parent nuclei linked to these substituents, and a plurality of structures such as aryl, heteroaryl, or fused rings with small conjugated systems are bonded and fused using substituents located on these ring structures to form a ring. This can further increase a conjugation length, further reduce a number of ring structures of free aryl, heteroaryl, or the like, and effectively suppress vibrational and rotational freedom of the ring structures of free aryl, heteroaryl, or the like, further improving planarity of the molecules. In this case, an overlap between orbitals in a ground state and an excited state increases, a probability of migration increases, light-emitting efficiency increases, and conjugation extension causes a luminescence wavelength to be longer, and is conducive to further narrowing an FWHM on a luminescence spectrum of the new organic compound.

With reference to the first aspect, in some embodiments, the substituents are each independently selected from one or a combination of the following: aryl, heteroaryl, a fused ring, linear alkyl, branched alkyl, alicyclic hydrocarbyl, alkoxy, allyl, cyano, halogen, hydrogen, and deuterium.

With reference to the first aspect, in some embodiments, the substituents are each independently selected from one or a combination of the following: C6-C30 aryl, C5-C30 heteroaryl, a C10-C30 fused ring, C1-C8 linear alkyl, C1-C8 branched alkyl, C3-C10 alicyclic hydrocarbyl, C1-C8 alkoxy, allyl, cyano, halogen, hydrogen, and deuterium.

With reference to the first aspect, in some embodiments, formula (II) has a structure shown in formula (III), formula (IV), or formula (V):

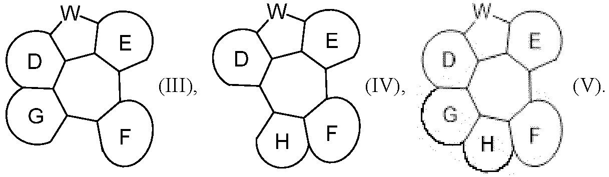

With reference to the first aspect, in some embodiments, the organic compound has a structure shown in formula (VI), formula (VII), formula (VIII), formula (IX), formula (X), formula (XI), formula (XII), formula (XIII), or formula (XIV): 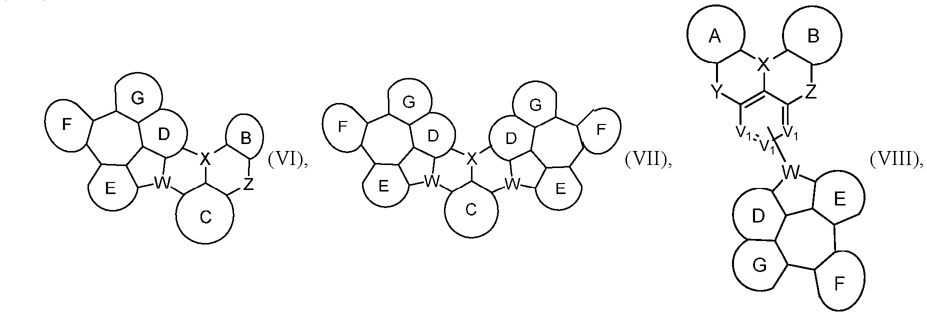 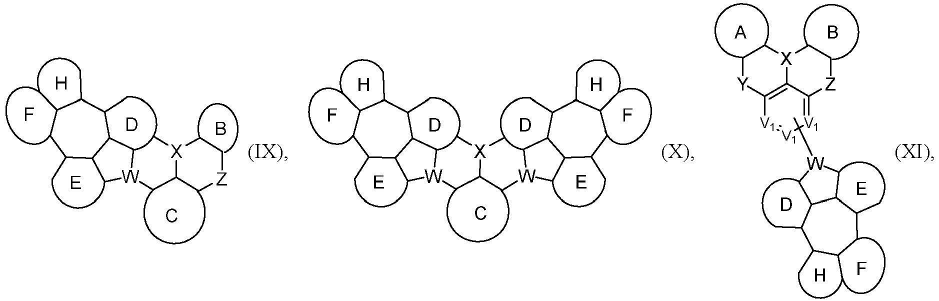 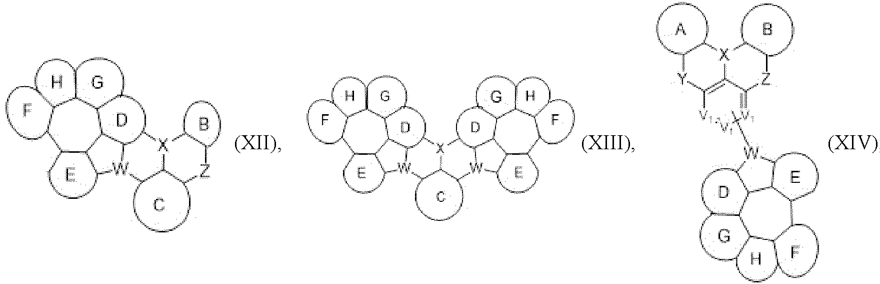 , where
V₁ is selected from C-R₅ or N-R₆; and
R₅ and R₆ are each independently selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms.

With reference to the first aspect, in some embodiments, the ring A, the ring B, the ring C, the ring D, the ring E, the ring F, the ring G, and the ring H are each independently selected from one or a combination of the following structures: 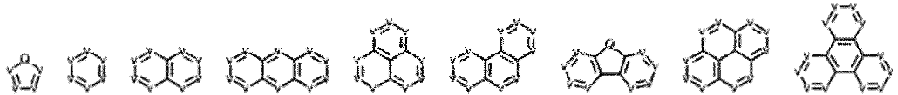
where V is selected from C-R₇ or N-R₈;
Q is selected from B-R₉, C(=O), N-R₁₀, O, S, P, P=O, or P=S; and
R₇ to R₁₀ are each independently selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms.

With reference to the first aspect, in some embodiments, a compound shown in formula (III) is 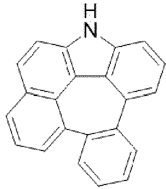 ; a compound shown in formula (IV) is 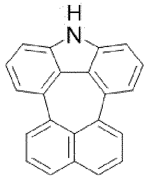 ; and a compound shown in formula (V) is

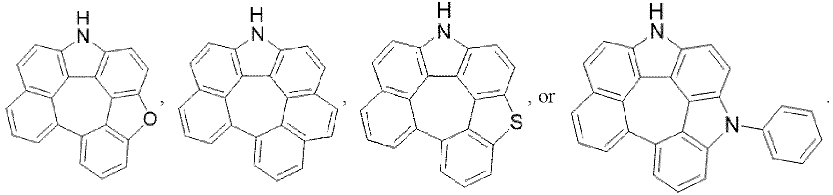

A second aspect of embodiments of this application provides a method for preparing an organic compound, where the preparation method includes the following steps:
substituting X₂ or X₃ in a compound 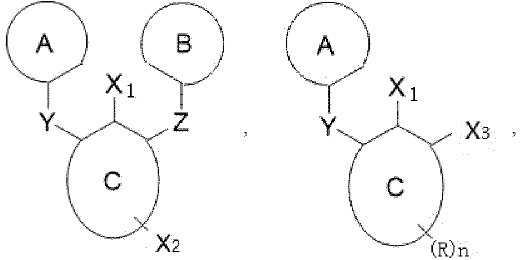 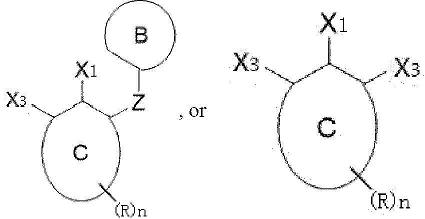 with a compound 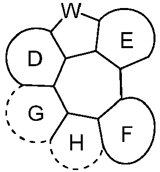 to obtain an intermediate 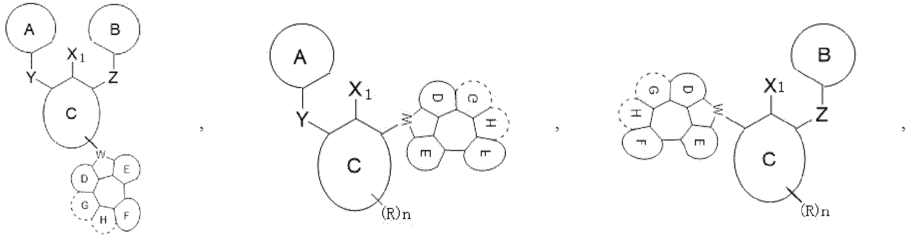 or 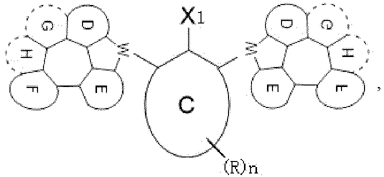 where X₁, X₂, and X₃ are each independently selected from a halogen atom;
a ring A, a ring B, a ring C, a ring D, a ring E, a ring F, a ring G, and a ring H are each independently selected from substituted or unsubstituted aryl, substituted or unsubstituted heteroaryl, or a substituted or unsubstituted fused-ring structure;
W is selected from C(R₁, R₂), N-R₃, P, P=O, or Al;
Y and Z are each independently selected from C=O, N-R₄, O, S, Se, P, P=O, or P=S, and when Y and Z are both present, at least one of Y and Z is N-R₄;
R and R₁ to R₄ are each independently selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms, and
n is an integer greater than or equal to 0; and
reacting the intermediate 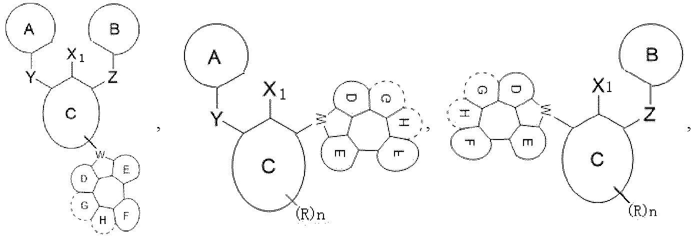 or 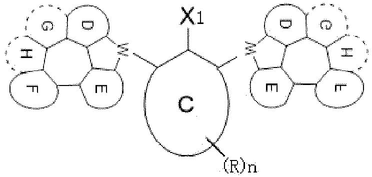 with an X-containing compound to obtain the organic compound 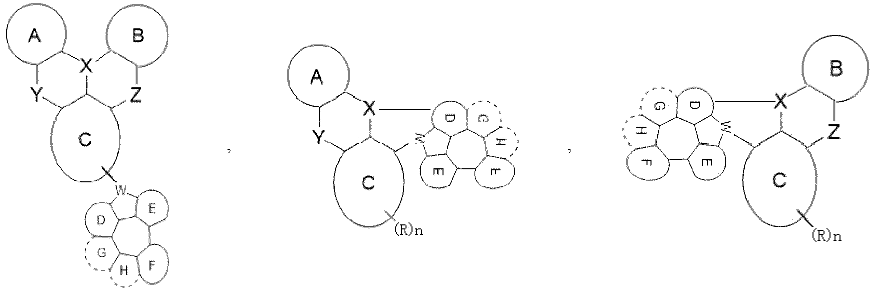 , or 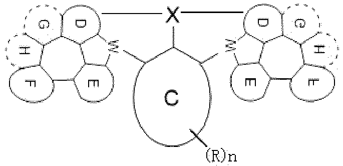 , where X is selected from B, N, P, P=O, or Al.

A third aspect of embodiments of this application provides another method for preparing an organic compound, where the preparation method includes the following steps:
substituting X₂ and X₃ in a compound 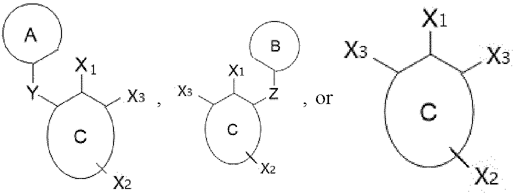 with a compound 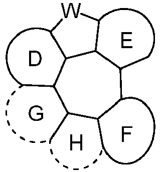 to obtain an intermediate 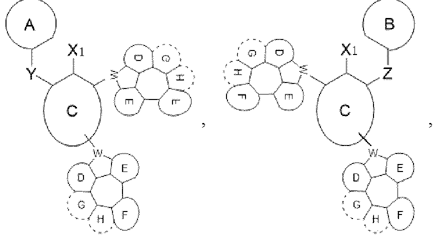 or 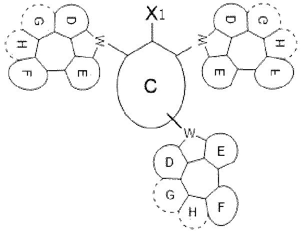 , where X₁, X₂, and X₃ are each independently selected from a halogen atom;
a ring A, a ring B, a ring C, a ring D, a ring E, a ring F, a ring G, and a ring H are each independently selected from substituted or unsubstituted aryl, substituted or unsubstituted heteroaryl, or a substituted or unsubstituted fused-ring structure;
W is selected from C(R₁, R₂), N-R₃, P, P=O, or Al;
Y and Z are each independently selected from C=O, N-R₄, O, S, Se, P, P=O, or P=S, and when Y and Z are both present, at least one of Y and Z is N-R₄; and
R and R₁ to R₄ are each independently selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms; and
reacting the intermediate 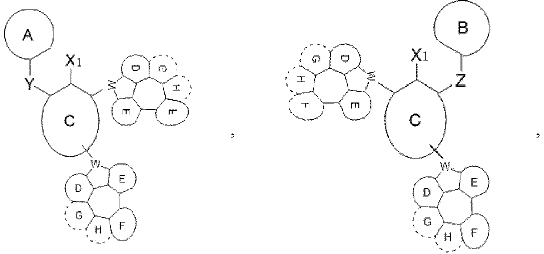 or 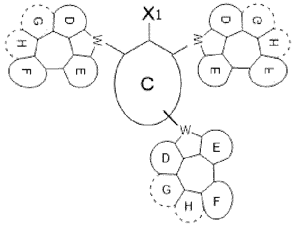 with an X-containing compound to obtain the organic compound 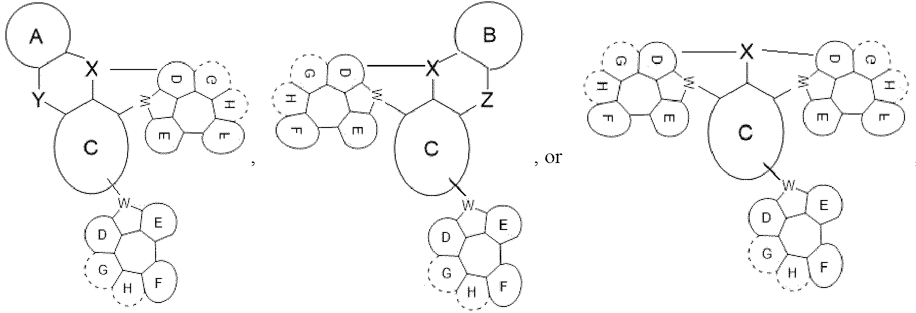 , where X is selected from B, N, P, P=O, or Al.

A fourth aspect of embodiments of this application provides a polymer, where the polymer includes at least one first repeating unit, and the first repeating unit is derived from at least one organic compound according to the first aspect of embodiments of this application or the organic compound prepared by using the method for preparing the organic compound according to the second aspect or the third aspect of embodiments of this application.

With reference to the fourth aspect, in some embodiments, the polymer further includes at least one second repeating unit different from the first repeating unit.

With reference to the fourth aspect, in some embodiments, the second repeating unit is selected from at least one of the following repeating units: 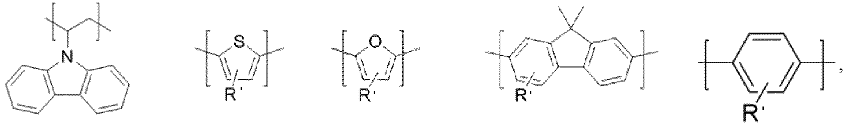 where
R' is selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms.

A fifth aspect of embodiments of this application provides another polymer, where the polymer includes a polymer molecule main chain and a branched chain linked to the polymer molecule main chain, and the branched chain is derived from the organic compound according to the first aspect of embodiments of this application or the organic compound prepared by using the method for preparing the organic compound according to the second aspect or the third aspect of embodiments of this application.

With reference to the fifth aspect, in some embodiments, the polymer includes at least one of the following repeating units: 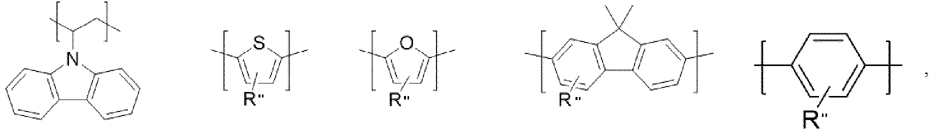 where
R" is selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms.

A sixth aspect of embodiments of this application provides a composition, where the composition includes an organic solvent and at least one organic compound according to the first aspect of embodiments of this application, or the organic compound prepared by using the method for preparing the organic compound according to the second aspect or the third aspect of embodiments of this application, or at least one polymer according to the fourth aspect of embodiments of this application, or at least one polymer according to the fifth aspect.

A seventh aspect of embodiments of this application provides a mixture, including an organic functional material and at least one organic compound according to the first aspect of embodiments of this application, or the organic compound prepared by using the method for preparing the organic compound according to the second aspect or the third aspect of embodiments of this application, or at least one polymer according to the fourth aspect of embodiments of this application, or at least one polymer according to the fifth aspect, or at least one composition according to the sixth aspect of embodiments of this application, where the organic functional material is selected from at least one of a hole injection material, a hole transport material, an electron transport material, an electron injection material, an electron blocking material, a hole blocking material, a light-emitting body, and a host material.

An eighth aspect of embodiments of this application provides a device, including at least one functional layer and two electrodes, where the at least one functional layer is located between the two electrodes, and the at least one functional layer includes at least one organic compound according to the first aspect of embodiments of this application, or the organic compound prepared by using the method for preparing the organic compound according to the second aspect or the third aspect of embodiments of this application, or at least one polymer according to the fourth aspect of embodiments of this application, or at least one polymer according to the fifth aspect, or at least one composition according to the sixth aspect of embodiments of this application, or at least one mixture according to the seventh aspect of embodiments of this application.

With reference to the eighth aspect, in some embodiments, the device is an organic light-emitting device, the at least one functional layer includes a light-emitting layer, and a guest material of the light-emitting layer includes at least one organic compound according to the first aspect of embodiments of this application, or the organic compound prepared by using the method for preparing the organic compound according to the second aspect or the third aspect of embodiments of this application, or at least one polymer according to the fourth aspect of embodiments of this application, or at least one polymer according to the fifth aspect.

In this application, the organic compound is used in an organic light-emitting device, and therefore the organic light-emitting device can have high light-emitting efficiency, high color purity, high device stability, a long device service life, and the like.

A ninth aspect of embodiments of this application provides a display apparatus, where the display apparatus includes a control system and at least one device according to the eighth aspect of embodiments of this application, where the control system is configured to control the device.

A tenth aspect of embodiments of this application provides an electronic device, where the electronic device includes a housing and the display apparatus according to the ninth aspect of embodiments of this application disposed on the housing.

### BRIEF DESCRIPTION OF DRAWINGS

FIG. 1 is a diagram of a structure of an organic device according to an embodiment of this application;
FIG. 2 is a diagram of a structure of a display apparatus according to an embodiment of this application; and
FIG. 3 is a diagram of a structure of an electronic device according to an embodiment of this application.

### DESCRIPTION OF REFERENCE NUMERALS OF MAIN ELEMENTS

| | |
|---|---|
| Organic device | 100 |
| Substrate | 101 |
| Anode | 102 |
| Hole injection layer | 103 |
| Hole transport layer | 104 |
| Light-emitting layer | 105 |
| Electron transport layer | 106 |
| Electron injection layer | 107 |
| Cathode | 108 |
| Display apparatus | 200 |
| Control system | 210 |
| Electronic device | 300 |
| Housing | 310 |

### DESCRIPTION OF EMBODIMENTS

In an organic light-emitting display, three primary colors of light, that is, red, green, and blue, are mixed to express various colors. However, the three primary colors with respective low color purity produce irreproducible colors, and consequently picture quality of the display is greatly reduced. In this case, in commercially available displays, color purity is improved by removing undesired colors from a luminescence spectrum using an optical filter. In this way, if an original spectral width is broad, a proportion of removal increases. Therefore, actual light-emitting efficiency is greatly reduced even in a high light-emitting efficiency case. For example, a full width at half maximum (FWHM) on a blue luminescence spectrum of a commercially available smartphone is about 20 nm to 25 nm, but a value of an FWHM of a common fluorescent material is about 40 nm to 60 nm, a value of an FWHM of a phosphorescent material is about 60 nm to 90 nm, and a value of an FWHM of a TADF material is about 70 nm to 100 nm. In a case that a fluorescent material is used, since the FWHM is narrow, it is sufficient to remove only some undesired colors. However, in a case that a phosphorescent material or a TADF material is used, more than half of colors need to be removed, and consequently actual light-emitting efficiency is greatly reduced.

To resolve disadvantages of conventional light-emitting materials, such as a broad FWHM on a luminescence spectrum, difficulty in providing deep blue light for display effect of a device, failure in meeting requirements of display techniques for a high color rendering index, and low light-emitting efficiency of the device, which are not conducive to achieving low power consumption and a long service life of the display device, it is desired to develop a light-emitting material with both high light-emitting efficiency and high color purity. In this research background, a light-emitting material of a boron-nitrogen ring compound type is developed.

It is found from research that the light-emitting material of the boron-nitrogen ring compound type provides multiple resonance effect in molecular design. To be specific, a molecular structure is maintained in a special planar rigid conjugated structure, and specific unoccupied orbitals that are on boron atoms and that can be involved in electron cloud conjugation and lone-pair electrons that are on nitrogen atoms and that can be involved in electron cloud conjugation result in mutual enhancement through conjugation effect based on their different electronegativities, forming an intramolecular short-range charge transfer state, thereby implementing highly efficient light emission with thermally activated delayed fluorescence (TADF) properties. In addition, compared with another light-emitting material (for example, a fluorescent material), the planar structure of the boron-nitrogen ring compound is characterized by a high degree of merging of energy levels in molecular vibration modes, so that an FWHM on a luminescence spectrum of the boron-nitrogen ring compound is significantly narrower than that of another type of light-emitting material (for example, a fluorescent material), which is conducive to achieving high color purity. Due to high device efficiency, high color purity, and potentially high device stability, the boron-nitrogen ring compound material is highly valued by academia and the industry, and is one of the most popular topics in the field of light-emitting materials for OLED devices.

However, an FWHM on a luminescence spectrum and light-emitting efficiency of the light-emitting material of the boron-nitrogen ring compound type usually depend on whether vibrational and rotational freedom of groups in the molecules can be effectively suppressed. For example, for currently typical light-emitting materials of a boron-nitrogen ring compound type (with structures shown in the following formulas a to e), as there is a large torsion angle between a phenyl group linked to a nitrogen atom and a boron-nitrogen rigid conjugation plane, the phenyl group is not directly involved in super-resonance effect of the boron-nitrogen rigid conjugation plane, and cannot be directly involved in a light-emitting process of the boron-nitrogen ring compound. Moreover, such phenyl group usually has large vibrational and rotational degrees of freedom, which has adverse effect on narrowing an FWHM on a luminescence spectrum of the boron-nitrogen ring compound. Therefore, the light-emitting material of the boron-nitrogen ring compound type still has room for improvement in achieving higher light-emitting efficiency, a narrower FWHM, and a longer device service life.

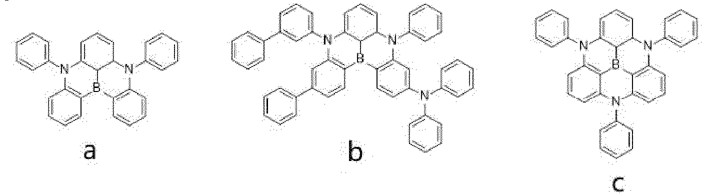

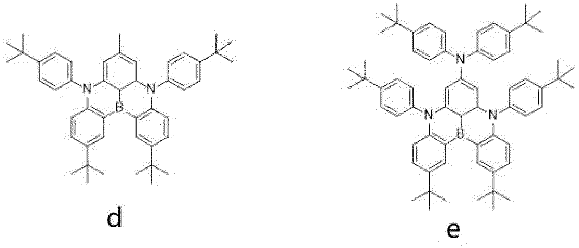

To resolve the foregoing problems, this application provides a new organic compound. The organic compound may be used as an organic light-emitting material in an organic light-emitting device, but is not limited thereto. The organic compound is optimized and selected, so that the organic compound has at least high light-emitting efficiency, a narrow FWHM on a luminescence spectrum, and a long luminescence lifetime.

The following further describes this application in detail with reference to specific embodiments. Unless otherwise specified, a data range involved in this application should include an end value.

In addition, the following describes some terms in this application.

In this application, the term "substituted" indicates that a hydrogen atom in a substituted group is substituted with a substituent.

In this application, when a same substituent is present for a plurality of times, the same substituent may be separately selected from different groups.

In this application, the term "substituted or unsubstituted" indicates that a defined group may be substituted or may not be substituted. When a defined group is substituted with a substituent, it should be understood that the defined group is optionally substituted with a substituent acceptable in the art, and the substituent may be further substituted with a substituent acceptable in the art.

In this application, the term "number of ring atoms" indicates a number of atoms that bond together into a ring to obtain a structural compound (such as a monocyclic compound, a fused-ring compound, a crosslinked compound, a carbocyclic compound, or a heterocyclic compound). When the ring is substituted with a substituent, atoms included in the substituent are not included in the ring atoms. The same applies to the term "number of ring atoms" described below unless otherwise specified. For example, a number of ring atoms of a benzene ring is 6, a number of ring atoms of a naphthalene ring is 10, and a number of ring atoms of a thienyl group is 5.

In this application, the term "adjacent substituents" indicates that substituted groups are located on a same ring and adjacent to each other. For example, in the following formula (I), Z and the ring B or the ring C have a common ring, and a substituent on Z and a substituent that is close to Z and that is on the ring B or the ring C are "adjacent substituents" that can be bonded and fused to form a ring. Substituted groups located on different rings, even if substituents on the two substituted groups are adjacent, do not meet the definition of "adjacent substituents", and can be neither bonded nor fused to form a ring. For example, one substituent linked to the ring A and one substituent linked to the ring B, even if the two substituents are adjacent, can be neither bonded nor fused to form a ring.

In this application, a single bond linked to a substituent running through a corresponding ring indicates that the substituent may be linked to the ring at any position. For example, R in 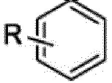 is linked to a benzene ring on any substitutable site.

In this application, when a same group includes a plurality of substituents with a same symbol, the substituents may be the same or different. For example, six R₁ on a benzene ring 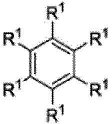 may be the same or different.

The organic compound provided in this application has a structural formula shown in formula (I): 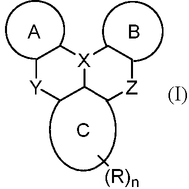 , where at least one of a ring A, a ring A-Y group, a ring B, a ring B-Z group, and R has a structure shown in formula (II): 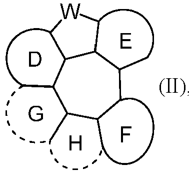 , where
a ring G and a ring H in the structure shown in formula (II) are absent or at least one of the ring G and the ring H is present;
the ring A, the ring B, a ring C, a ring D, a ring E, a ring F, the ring G, and the ring H are each independently selected from substituted or unsubstituted aryl, substituted or unsubstituted heteroaryl, or a substituted or unsubstituted fused-ring structure;
W is selected from C(R₁, R₂), N-R₃, P, P=O, or Al;
X is selected from B, N, P, P=O, or Al;
Y and Z are each independently selected from C=O, N-R₄, O, S, Se, P, P=O, or P=S, and when Y and Z are both present, at least one of Y and Z is N-R₄;
R and R₁ to R₄ are each independently selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms; and
n is an integer greater than or equal to 0.

It may be understood that at least one hydrogen on the foregoing substituted aryl, heteroaryl, alkyl, cycloalkyl, or the like may be further substituted.

For ease of description, in the following, when none of the ring A, the ring A-Y group, the ring B, the ring B-Z group, and R in formula (I) is the ring structure shown in formula (II), the ring structure is referred to as a "basic ring structure" for short. It may be understood that the basic ring structure in this application is a ring structure similar to a boron-nitrogen ring.

For an organic light-emitting material having high light-emitting efficiency, a narrow FWHM on a luminescence spectrum, a long device service life, and the like, the inventors of this application have carried out creative experimental research and synthesized a new organic compound based on a boron-nitrogen-ring-like structure. In the new organic compound, X is an electron acceptor atom or group with an electron-pulling ability, and Y and Z are each independently an electron donor atom or group with an electron-donating ability. The site X is at a para-position of the sites Y, Z, and W separately on a same conjugated six-membered ring, so that X with Y, Z, and W separately generates hyperconjugation effect with multiple conjugation effects, achieving localization of a highest occupied molecular orbital (Highest Occupied Molecular Orbital, HOMO) and a lowest unoccupied molecular orbital (Lowest Unoccupied Molecular Orbital, LUMO) on the ring A, the ring B, and the ring C, resulting in efficient reverse intersystem crossing, thereby improving light-emitting efficiency (up to 100%) of the new organic compound. To further narrow the FWHM on the luminescence spectrum of the basic ring structure, in this application, three methods are used: (1) adding the ring structure shown in formula (II) into a ring structure framework of the basic ring structure, and/or (2) introducing the ring structure shown in formula (II) as a modifying group into the basic ring structure on a specific site. In this way, the new organic compound achieves the following effects: a color of light is adjusted, an FWHM on a luminescence spectrum is narrowed, light-emitting efficiency is improved, and a luminescence lifetime is prolonged.

For the method (1), the ring structure shown in formula (II) is introduced into the ring structure framework of the basic ring structure, making the ring structure shown in formula (II) a part of the ring structure framework on a rigid conjugation plane. In addition, introduction of the ring structure shown in formula (II) can increase a conjugation length of the entire molecule. An electron cloud on the ring structure shown in formula (II) also participates in super-resonance effect of the entire molecule. This can effectively narrow an FWHM on a luminescence spectrum of the new organic compound, and can cause a red shift to a sufficient degree on the spectrum of the new organic compound relative to that of the basic ring structure, to adjust a color of light from a blue light region to a green light region to emit green light with a high color rendering index. Moreover, the ring D, the ring E, the ring F, the ring G, and W in the ring structure shown in formula (II) are fused to form rings, which reduces a number of free radicals (for example, ring structures such as aryl or heteroaryl) in the structure shown in formula (II), effectively suppresses vibrational and rotational freedom of the free radicals in the structure shown in formula (II), improves overall planarity of the molecules of the new organic compound, and further narrows the FWHM on the luminescence spectrum of the new organic compound.

For the method (2), the ring structure shown in formula (II) is introduced as a modifying group into the basic ring structure on the specific site, thereby adjusting a color of emitted light and narrowing an FHWM on a luminescence spectrum of the new organic compound. This is because after the ring structure shown in formula (II) is introduced as the modifying group into the basic ring structure on the specific site, a frontier orbital energy level of the new organic compound can be effectively adjusted, and multiple resonance effect of the new organic compound on the rigid conjugation plane can be enhanced based on a strong electron-donating ability of the ring structure shown in formula (II). In addition, based on structural features of the ring structure shown in formula (II), rotation and vibration of free radicals in the ring structure can be effectively suppressed, so that a color of light is adjusted and an FHWM on a luminescence spectrum is narrowed, thereby implementing highly efficient narrow-FHMW blue light emission. In addition, in terms of a degree of difficulty in synthesis, the specific site on the basic ring structure is selected according to a principle of mutual reduction of steric hindrance, and a group with small steric hindrance is preferably selected as a substitution site. For example, a hydrogen atom on the basic ring structure may be substituted with the ring structure shown in formula (II).

In some embodiments, formula (II) has a structure shown in formula (III), formula (IV), or formula (V):

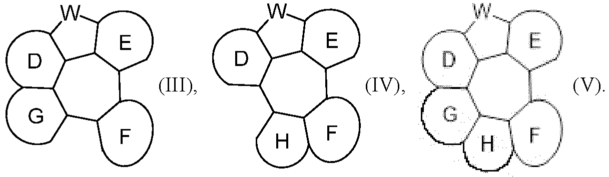

In some embodiments, the ring A, the ring B, the ring C, the ring D, the ring E, the ring F, the ring G, and the ring H are each independently selected from substituted or unsubstituted C6-C30 aryl, substituted or unsubstituted C5-C30 heteroaryl, or a substituted or unsubstituted C10-C30 fused-ring structure.

Further, each time when at least two substituents are present in the ring A, the ring B, the ring C, the ring D, the ring E, the ring F, the ring G, and the ring H, at least two adjacent substituents in the substituents may be bonded or fused together to form a ring, that is, at least two adjacent substituents on the ring A, the ring B, the ring C, the ring D, the ring E, the ring F, the ring G, and the ring H are bonded together and jointly form a ring structure (such as an aryl ring or a heteroaryl ring or a fused-ring structure) with parent nuclei. A plurality of structures such as aryl, heteroaryl, or fused rings with small conjugated systems are bonded and fused using substituents located on these ring structures to form a ring, which can further increase a conjugation length, further reduce a number of ring structures of free aryl, heteroaryl, or the like, and effectively suppress vibrational and rotational freedom of the ring structures of free aryl, heteroaryl, or the like, further improving planarity of the molecules. In this case, an overlap between orbitals in a ground state and an excited state increases, a probability of migration increases, light-emitting efficiency increases, and conjugation extension causes a luminescence wavelength to be longer, and is conducive to further narrowing an FWHM on a luminescence spectrum of the new organic compound.

Further, the substituents that may be present in the ring A, the ring B, the ring C, the ring D, the ring E, the ring F, the ring G, and the ring H are each independently selected from one or a combination of the following: aryl, heteroaryl, a fused ring, linear alkyl, branched alkyl, alicyclic hydrocarbyl, alkoxy, allyl, cyano, halogen, hydrogen, and deuterium. Further, when present, the substituents are each independently selected from one or a combination of the following: C6-C30 aryl, C5-C30 heteroaryl, a C10-C30 fused ring, C1-C8 linear alkyl, C1-C8 branched alkyl, C3-C10 alicyclic hydrocarbyl, C1-C8 alkoxy, allyl, cyano, halogen, hydrogen, and deuterium.

X is selected from B, N, P, P=O, or Al. These atoms or organic groups have strong electron-pulling abilities and can form a stable conjugated system with a plurality of electron donors.

Y and Z are each independently selected from C=O, N-R₄, O, S, Se, P, P=O, or P=S. These atoms or organic groups have strong electron-donating abilities, can form a stable conjugated system with electron acceptors, and can enhance multiple resonance effect of the new organic compound on the rigid conjugation plane. Especially, when at least one of Y and Z is N-R₄, the N atom has both an electron-donating ability and an electron-withdrawing ability. The N atom can form a stable conjugated system with an electron acceptor at a para-position, and can also be bonded and fused with an adjacent substituent on the ring B or the ring C to form a ring, further enhancing planarity of the new organic compound and multiple resonance effect on the rigid conjugation plane.

Further, when N-R₄ is present, R₄ is bonded and fused with the adjacent ring A or ring B to form a ring, that is, R₄ may be bonded and fused with the adjacent ring A or ring B to form a ring. By bonding and fusing the substituent on at least one of Y and Z with the adjacent ring A or ring B to form a ring, specifically with a substituent on the ring A or ring B to form a ring, a conjugation length of the new organic compound can be further increased, vibrational and rotational freedom of free substituents on Y, Z, the ring A, and the ring B can be further effectively suppressed, and planarity of the molecules can be further improved, thereby further improving light-emitting efficiency, and further narrowing an FWHM on a luminescence spectrum of the new organic compound.

In some embodiments, the organic compound has a structure shown in formula (VI), formula (VII), formula (VIII), formula (IX), formula (X), formula (XI), formula (XII), formula (XIII), or formula (XIV): 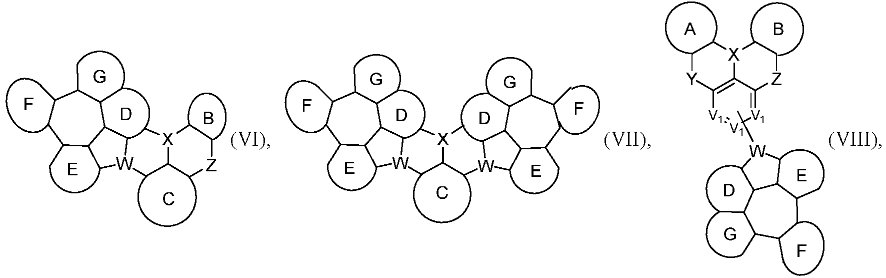 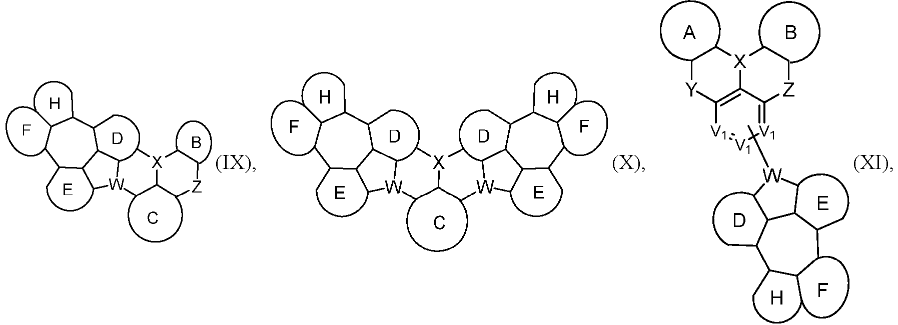 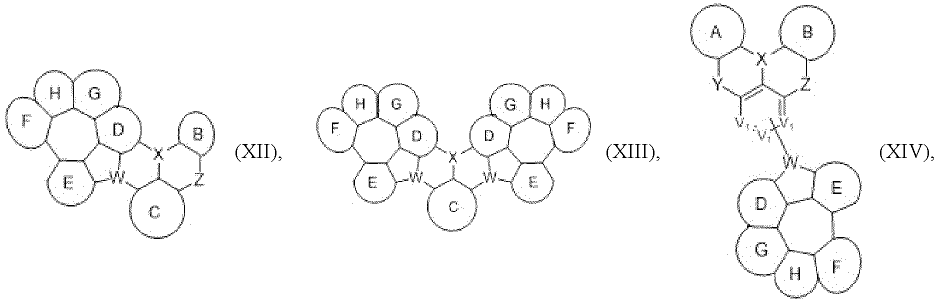 where
V₁ is selected from C-R₅ or N-R₆; and
R₅ and R₆ are each independently selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms.

In formula (VI), formula (VII), formula (IX), formula (X), formula (XII), and formula (XIII), the ring A-Y structure is specifically designed into the fused-ring structure shown in formula (III), formula (IV), or formula (V), or the ring A-Y structure and the ring B-Z structure are both specifically designed into the fused-ring structure shown in formula (III), formula (IV), or formula (V). The site X is at a para-position of the site W on a same conjugated six-membered ring, so that the site X and the site W generate hyperconjugation effect with multiple conjugation effects, thereby improving light-emitting efficiency of the new organic compound. In addition, introduction of the structure shown in formula (III), formula (IV), or formula (V) into the framework of the basic ring structure can improve planarity of the molecules of the organic compound, which is conducive to further narrowing an FWHM on a luminescence spectrum of the new organic compound. In the ring structures shown in formula (VIII), formula (XI), and formula (XIV), the ring C is modified by W at any V₁ position. The ring C has small steric hindrance at the V₁ position, which is conducive to introduction of the ring structure shown in formula (III), formula (IV), or formula (V). In addition, a frontier orbital energy level of the new organic compound can be effectively adjusted, and multiple resonance effect of the basic ring structure on the rigid conjugation plane can be enhanced based on a strong electron-donating ability of the ring structure shown in formula (II). In addition, rotation and vibration of the ring structure shown in formula (III), formula (IV), or formula (V) can be effectively suppressed, so that a color of light is adjusted and an FHWM on a luminescence spectrum is narrowed, thereby implementing highly efficient narrow-FHMW blue light emission.

In some embodiments, a compound shown in formula (III) is 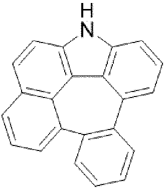 (specifically named as 3H-3-azadibenzo[G,IJ]naphth[2,1,8-CDE]azulene); a compound shown in formula (IV) is 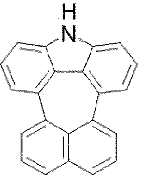 (specifically named as 4H-naphtho[1',8':5,6,7]cyclohepta[1,2,3,4-def]carbazole), and a compound shown in formula (V) is 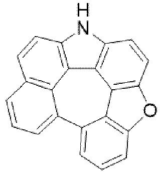 (specifically named as 13H-benzo[6,7]pleiadeno[1,12,11-bede]indole, where the ring G is a benzene ring), 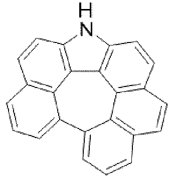 (specifically named as 1H-4-oxa-1-azanaphth[2',1',8':3,4,5]azuleno[1,8,7,6-cdef]fluorene, where the ring G is furan), 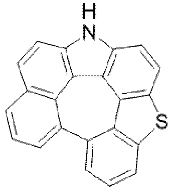 (specifically named as 1H-4-thia-1-azanaphth[2',1',8':3,4,5]azuleno[1,8,7,6-cdef]fluorene, where the ring G is thiophene), or 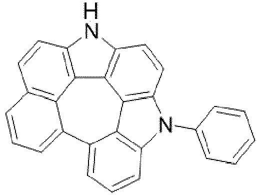 (specifically named as 1,4-diazanaphth[2',1',8':3,4,5]azuleno[1,8,7,6-cdef]fluorene, 1,4-dihydro-4-phenyl-(ACI), where the ring G is indole).

In some embodiments, the ring A, the ring B, the ring C, the ring D, the ring E, the ring F, the ring G, and the ring H are each independently derived from one or a combination of the following structures: 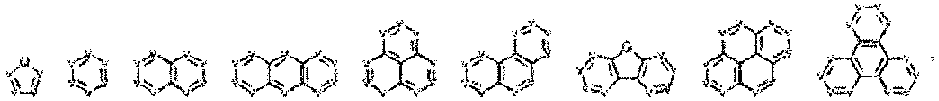 where
when present, V is selected from C-R₇ or N-R₈; when present, Q is selected from B-R₉, C(=O), N-R₁₀, O, S, P, P=O, or P=S; and when present, R₇ to R₁₀ are each independently selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms.

In some embodiments, the organic compound includes but is not limited to any one of the following compounds:

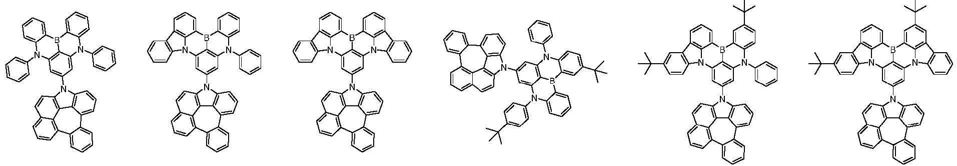

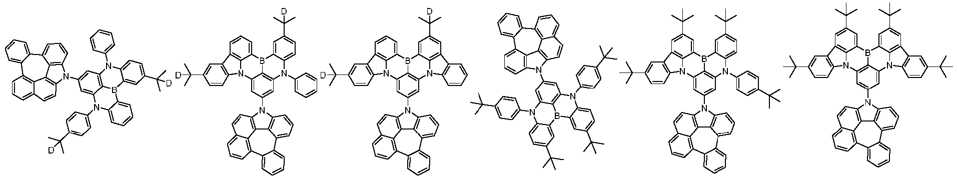

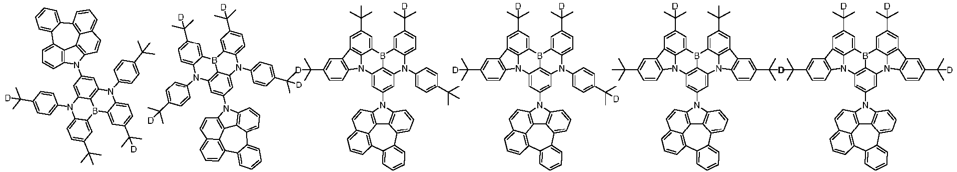

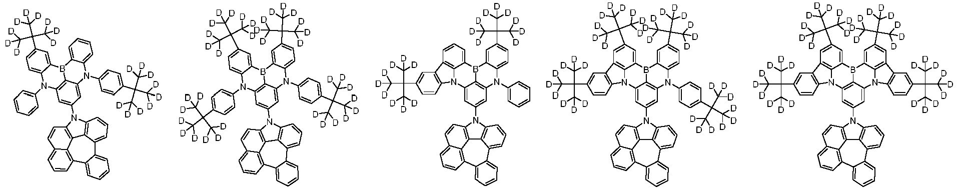

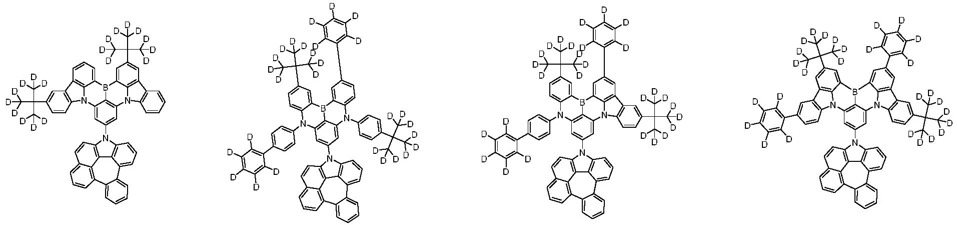

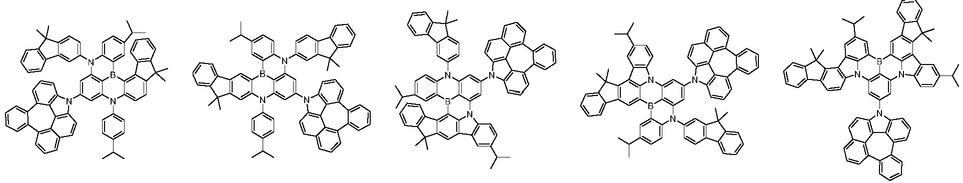

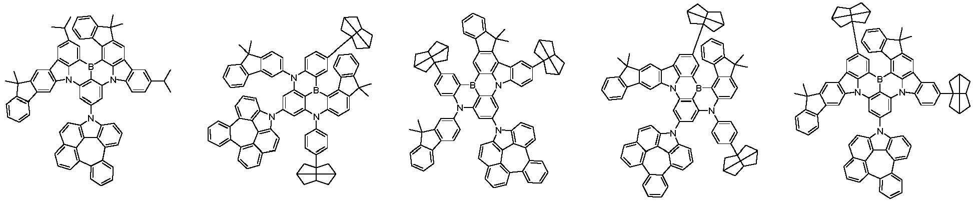

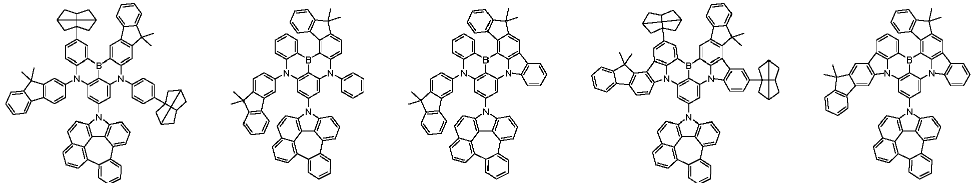

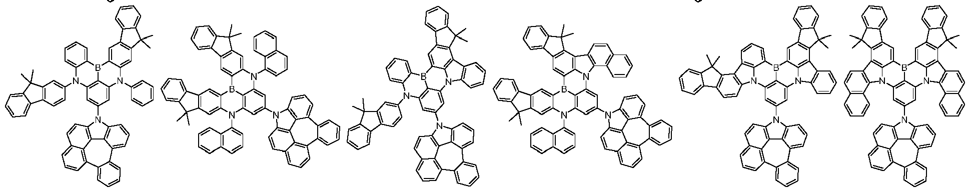

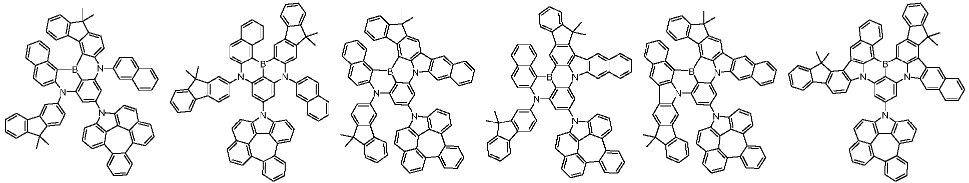

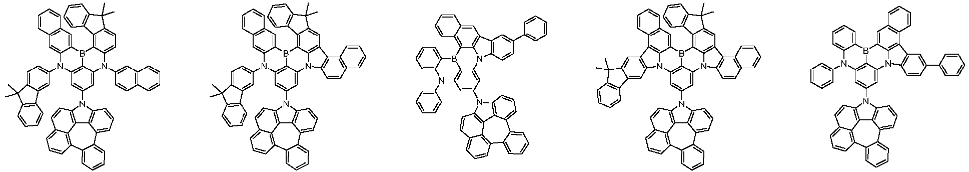

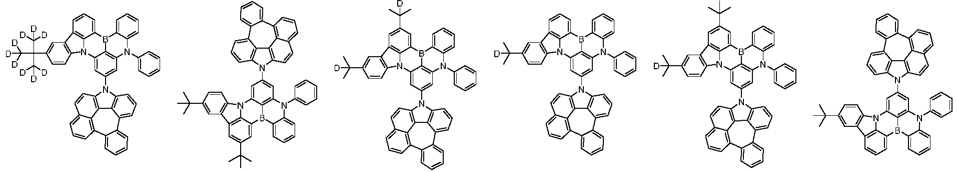

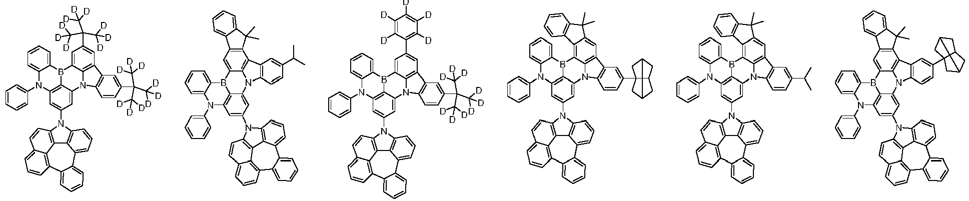

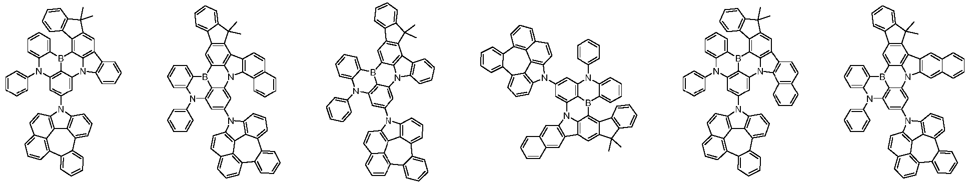

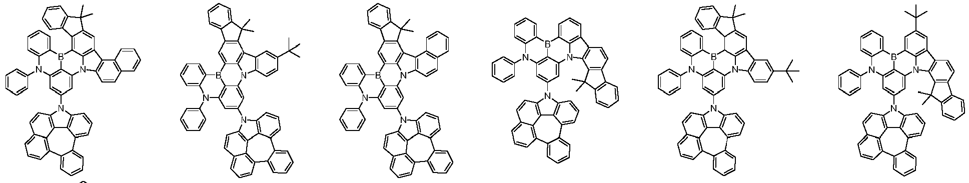

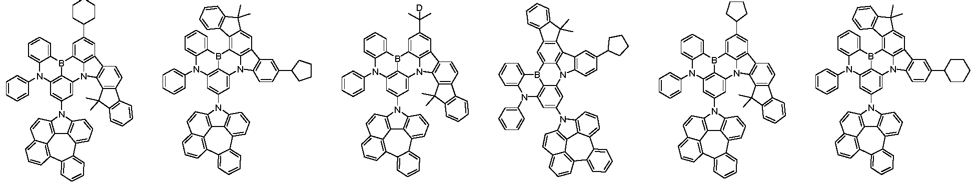

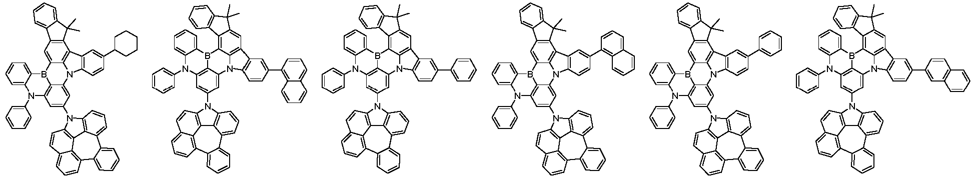

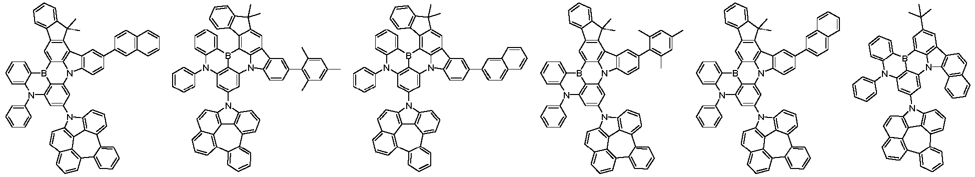

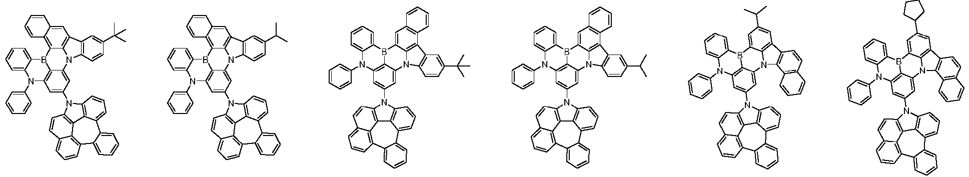

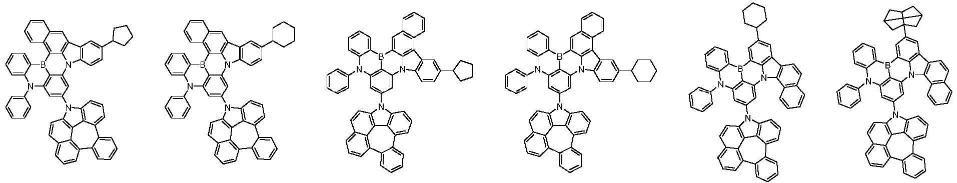

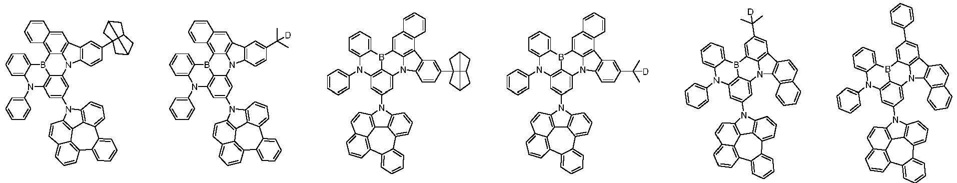

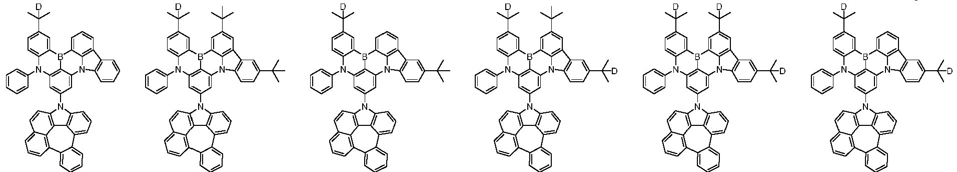

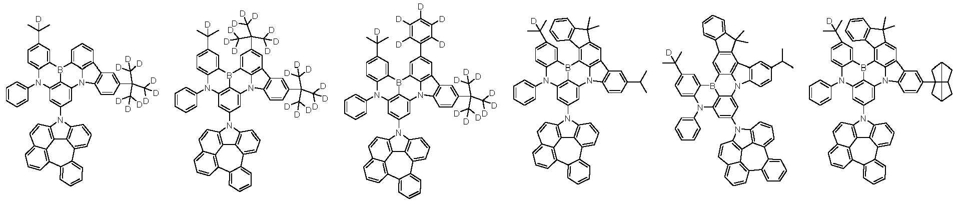

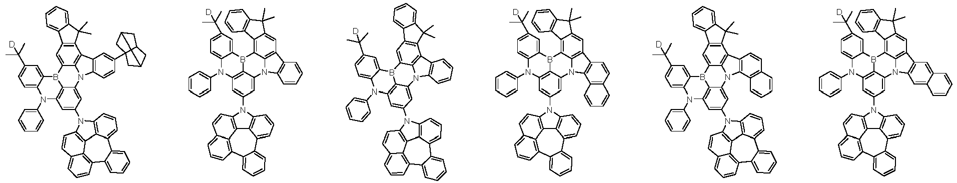

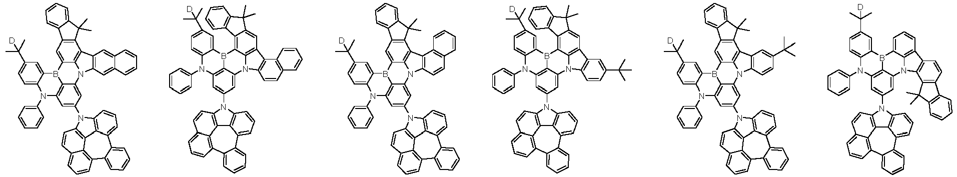

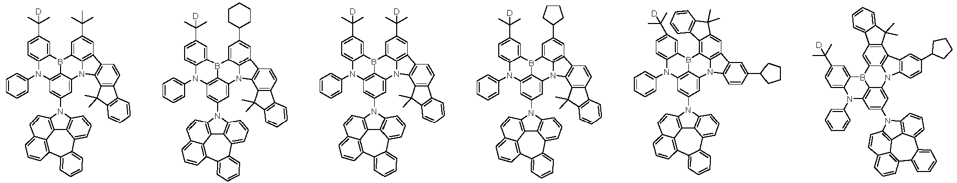

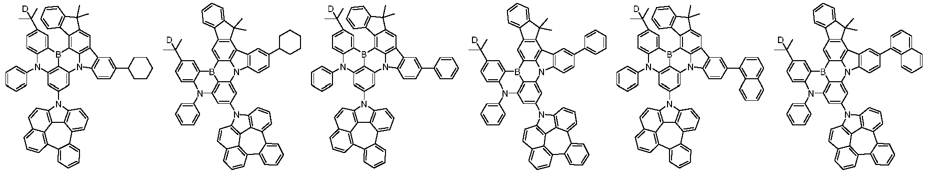

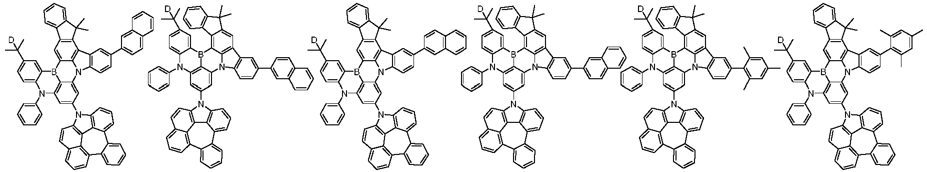

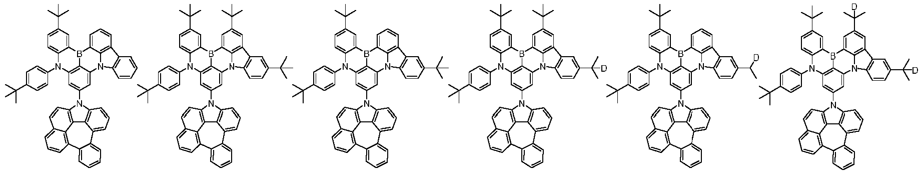

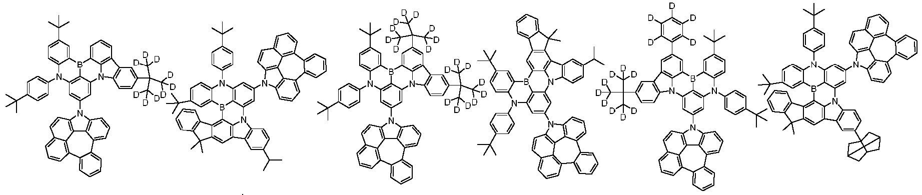

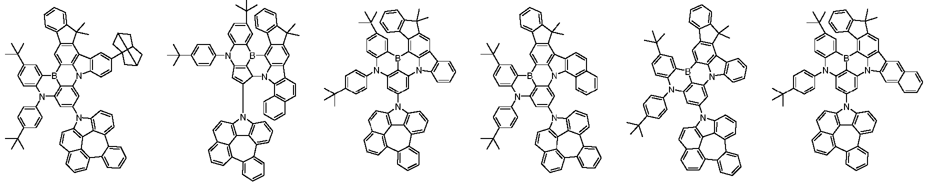

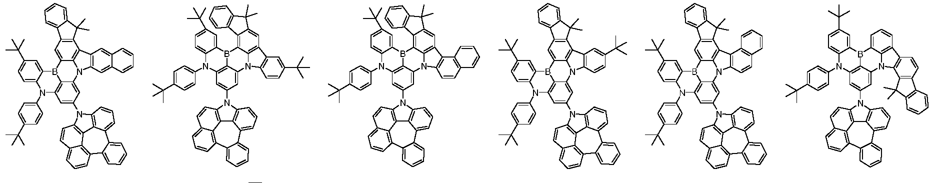

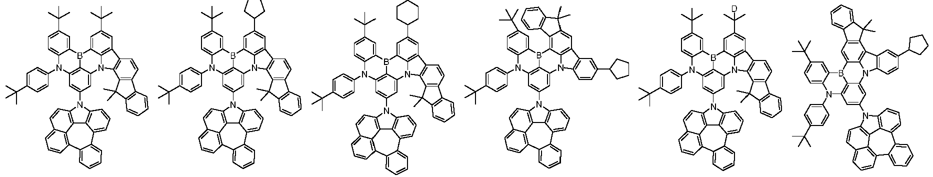

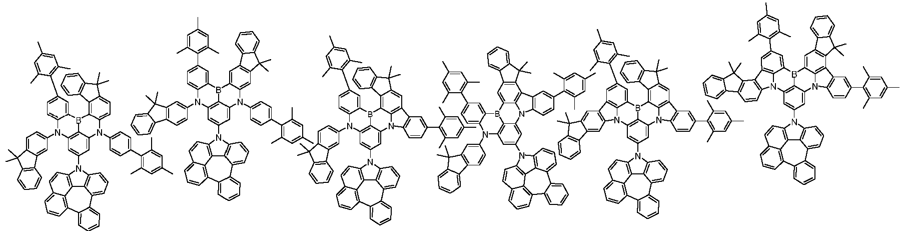

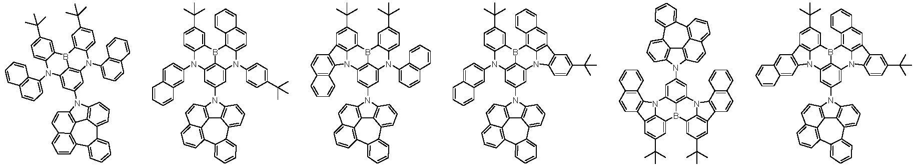

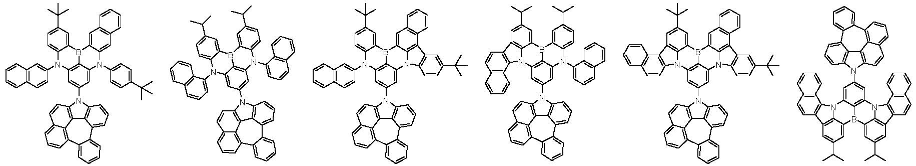

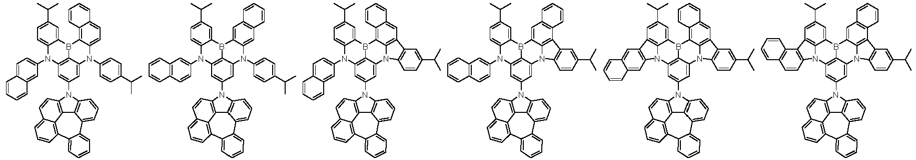

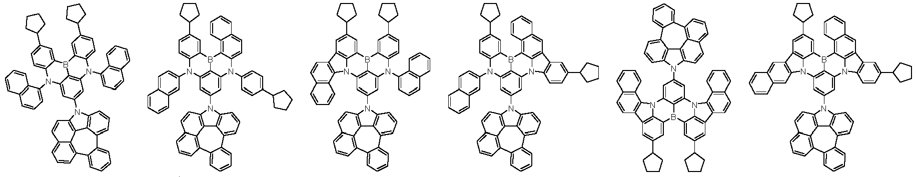

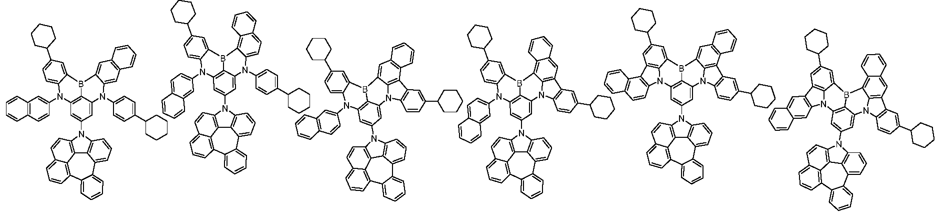

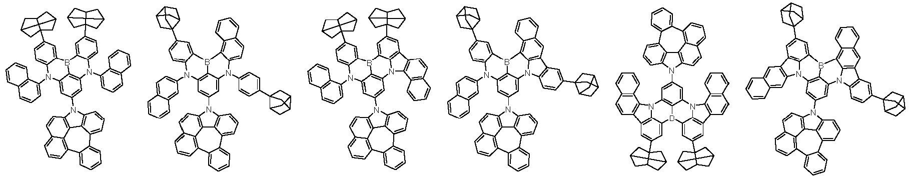

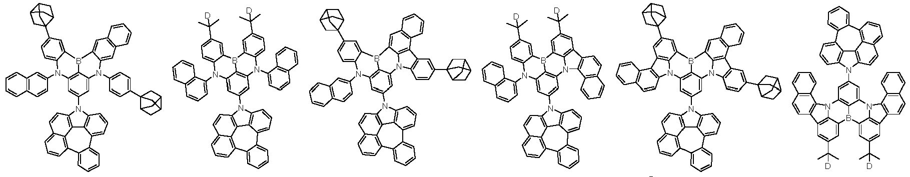

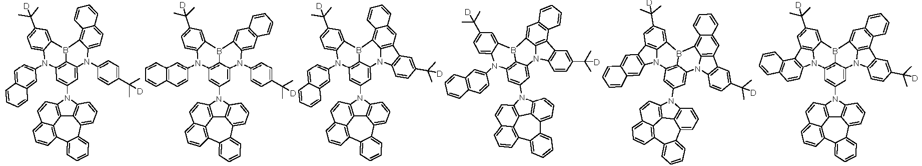

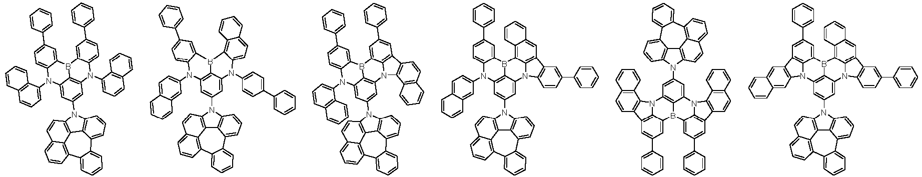

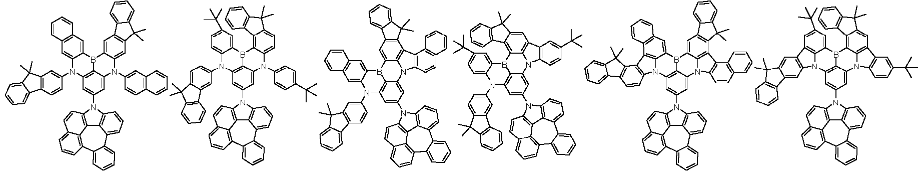

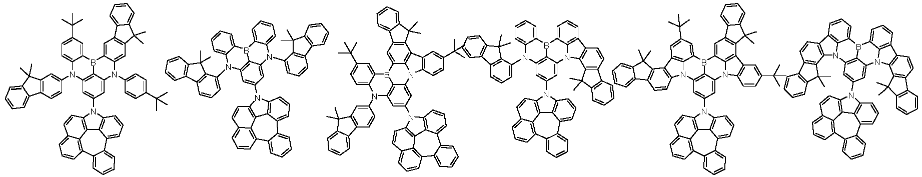

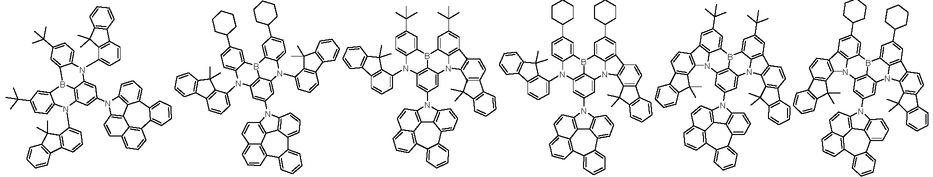

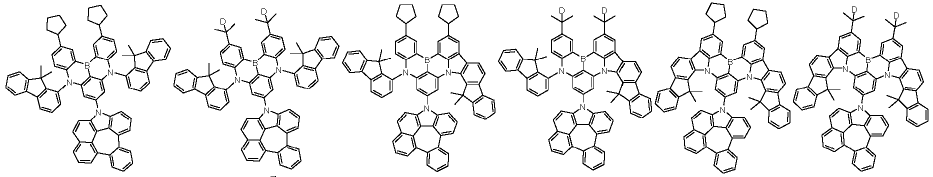

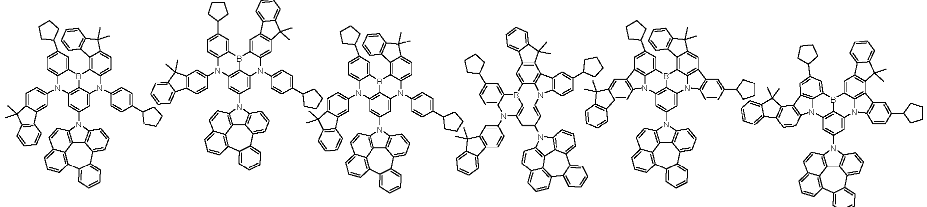

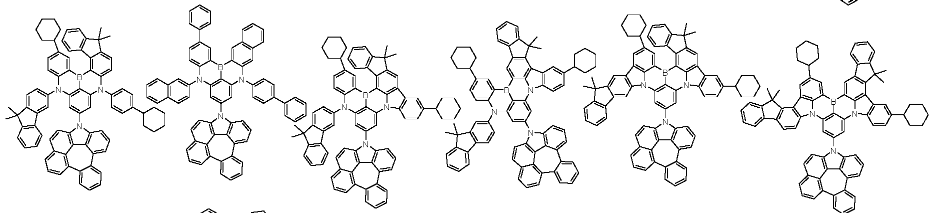

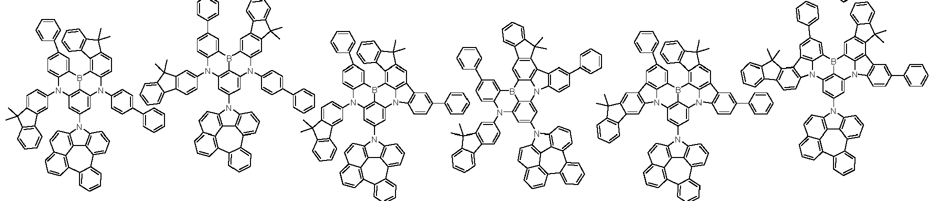

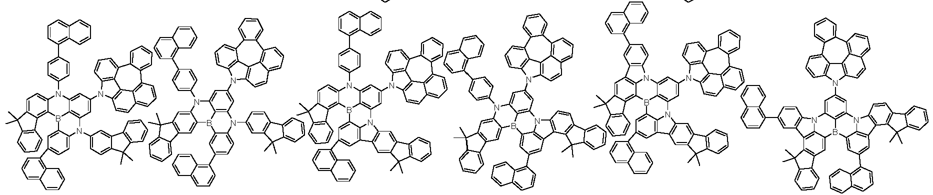

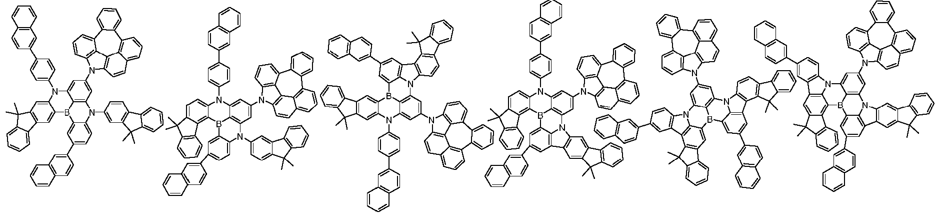

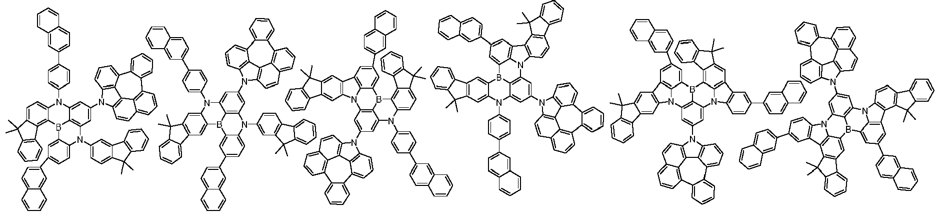

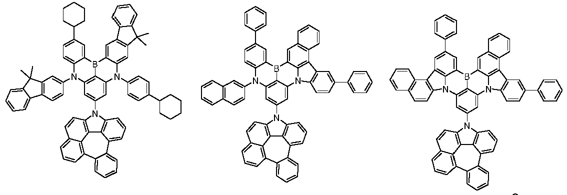

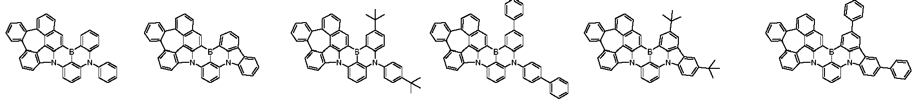

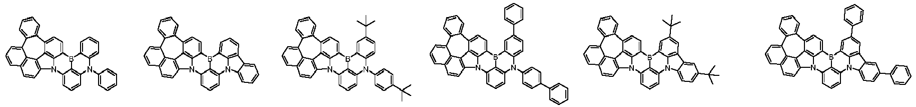

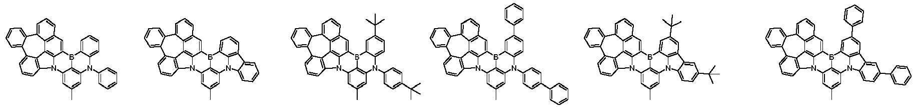

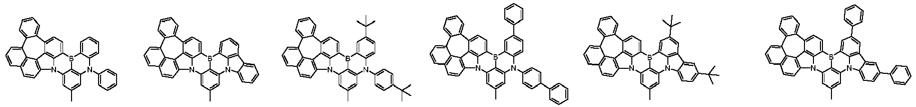

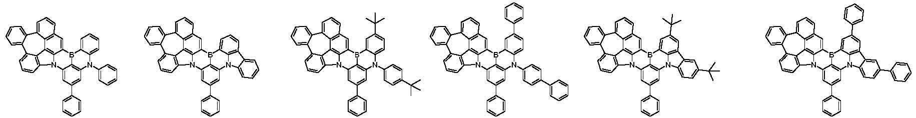

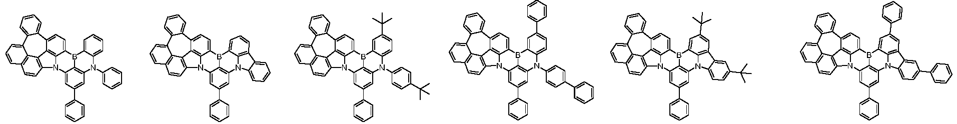

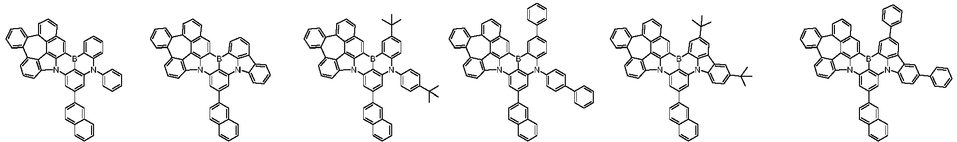

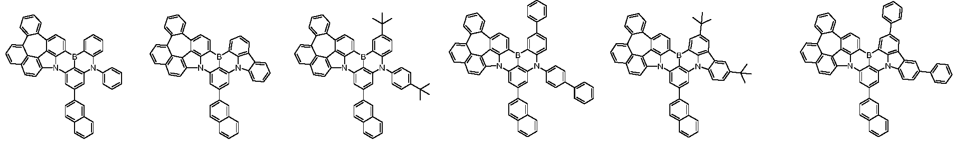

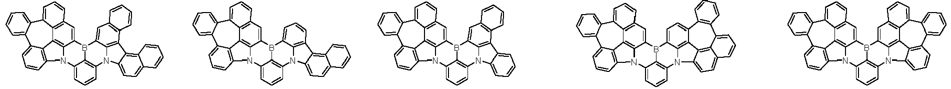

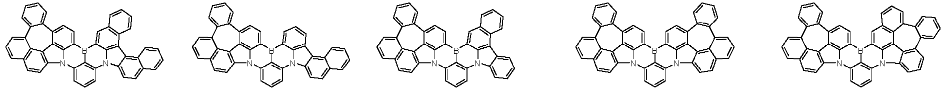

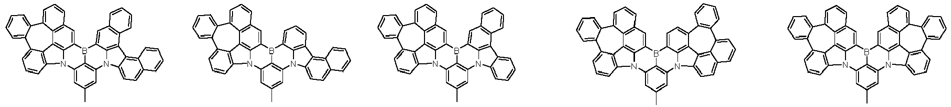

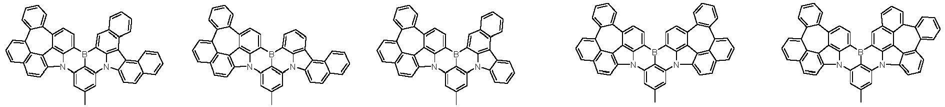

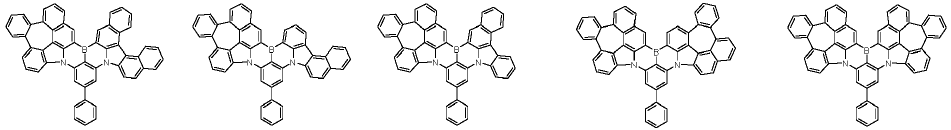

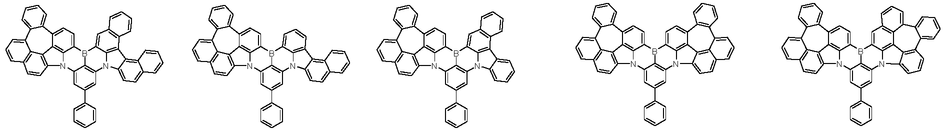

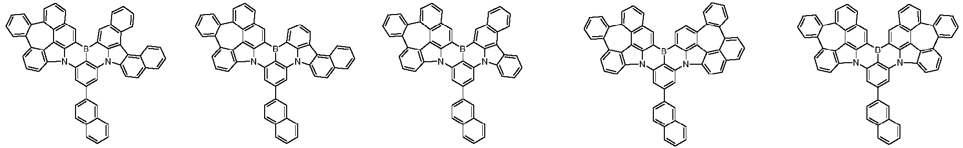

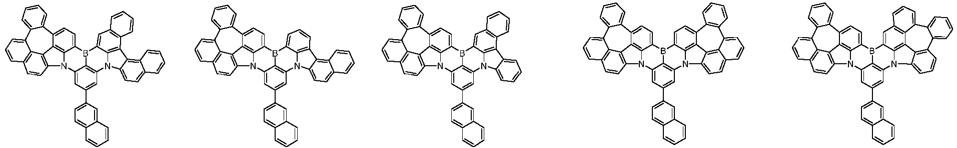

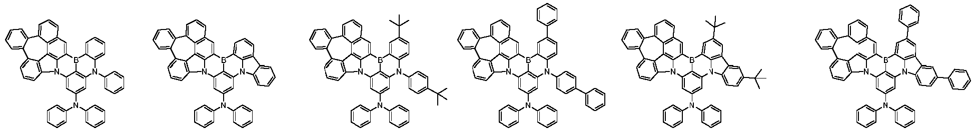

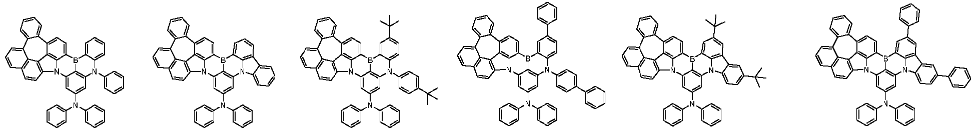

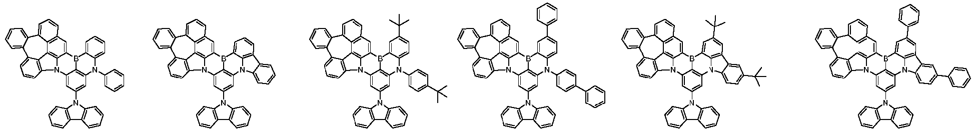

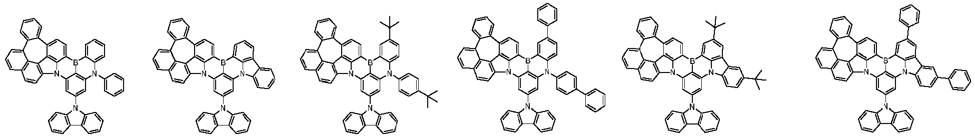

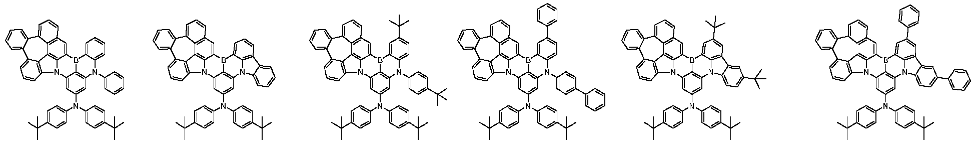

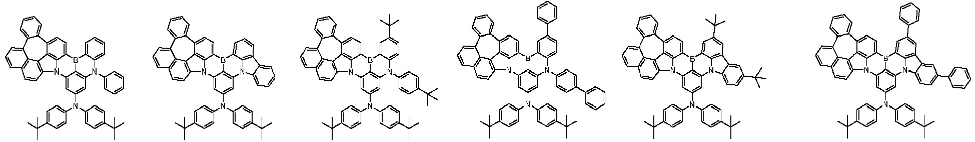

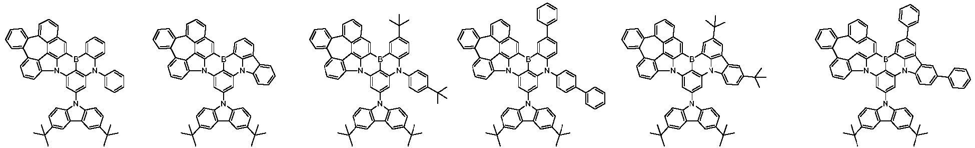

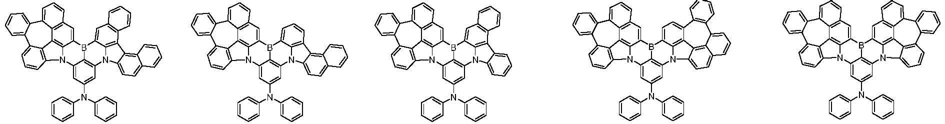

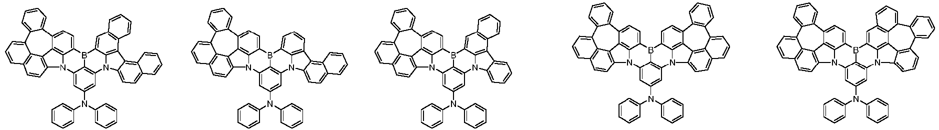

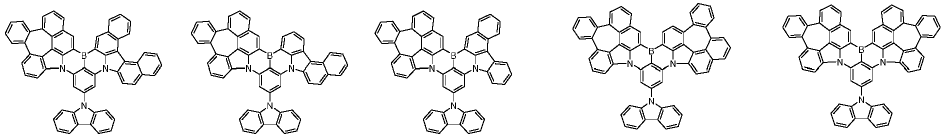

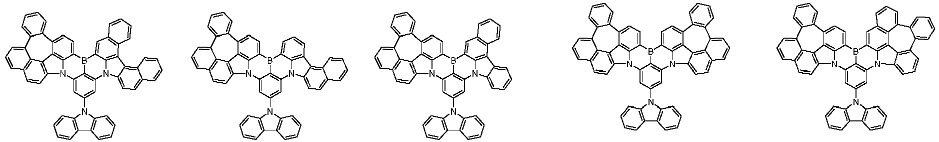

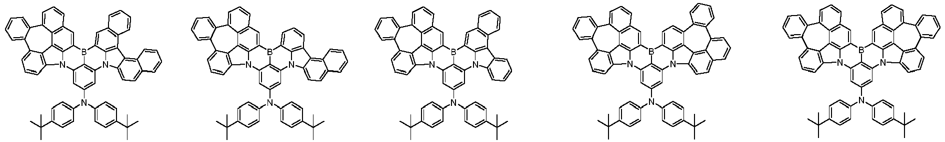

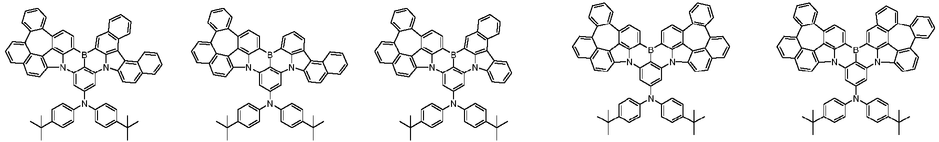

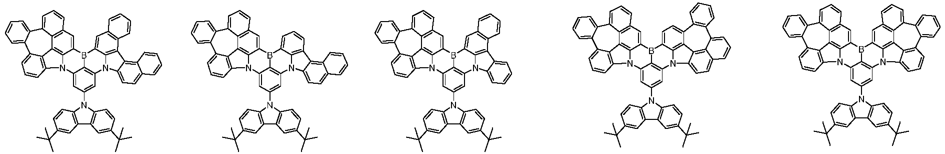

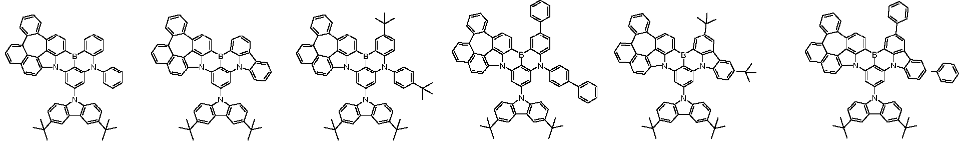

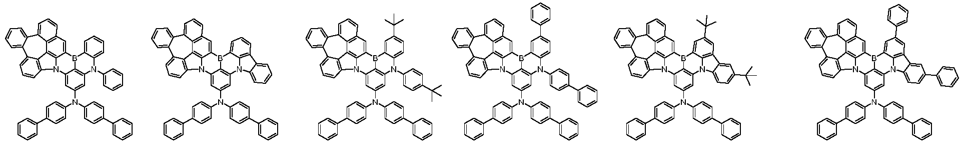

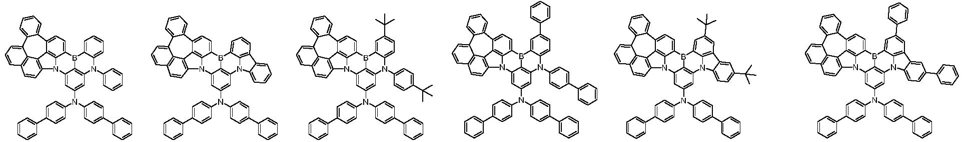

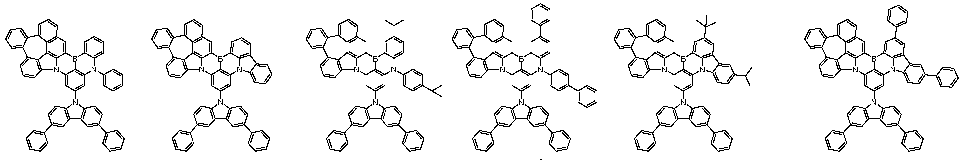

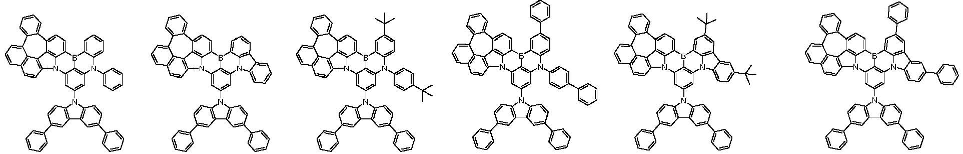

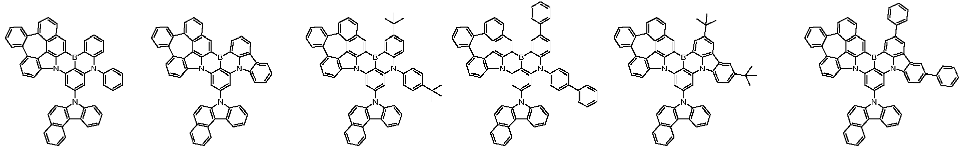

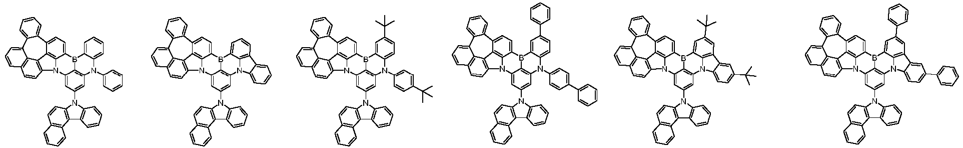

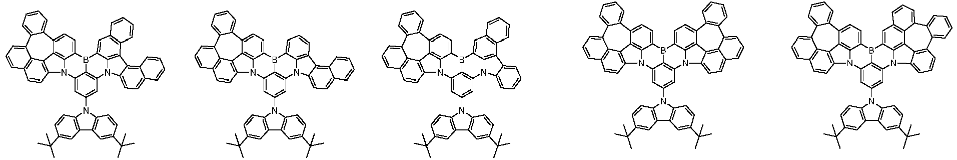

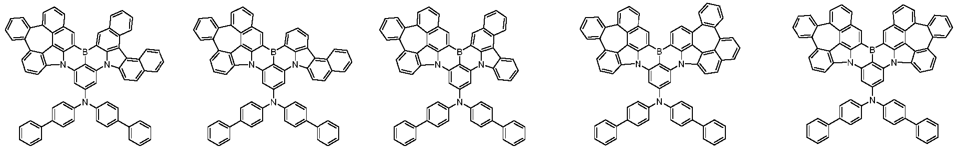

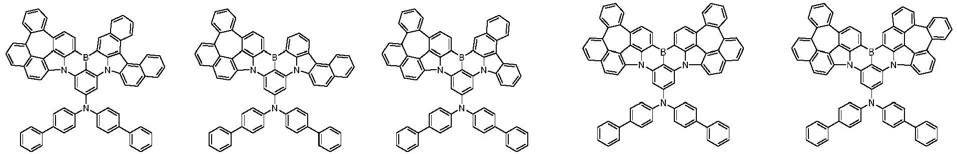

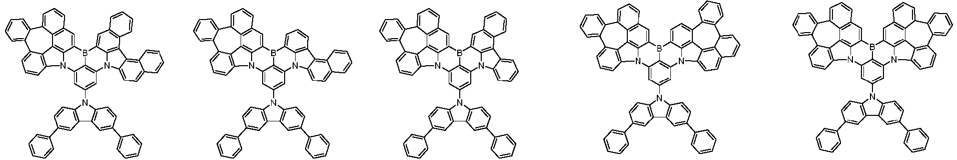

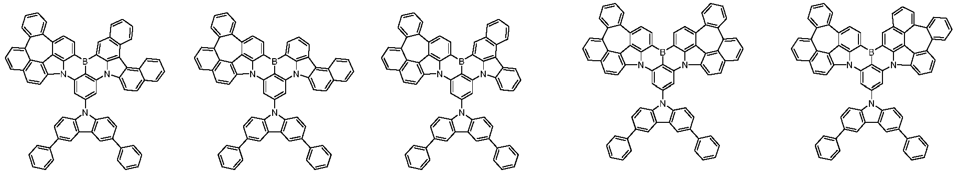

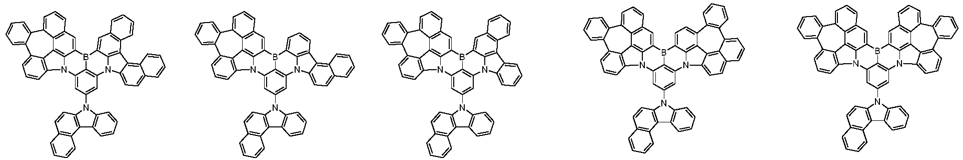

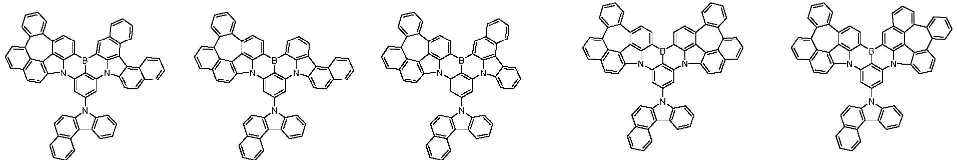

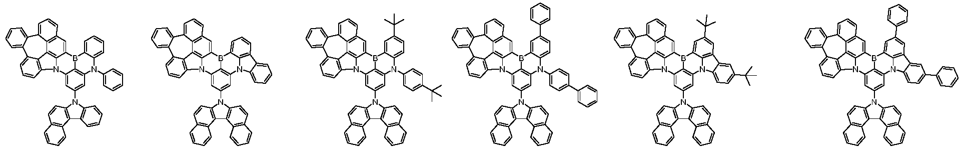

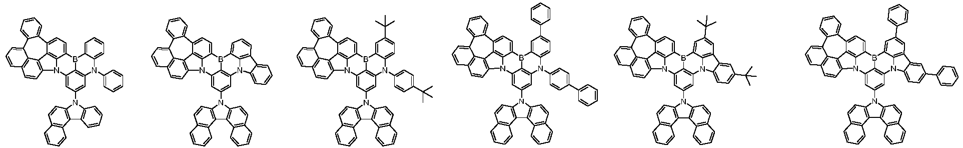

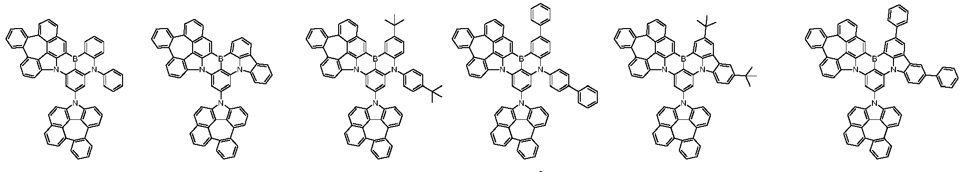

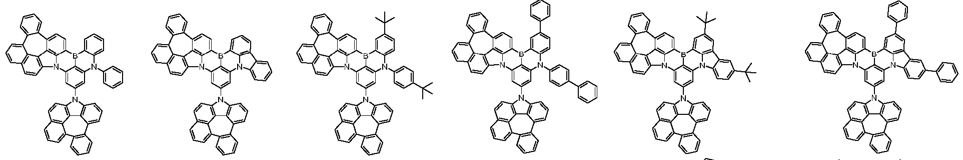

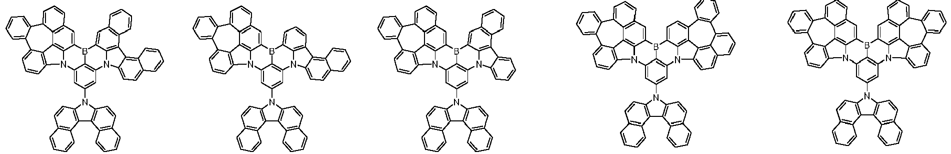

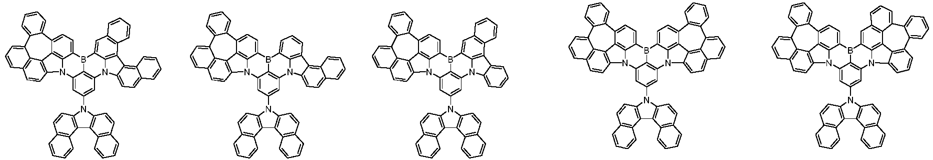

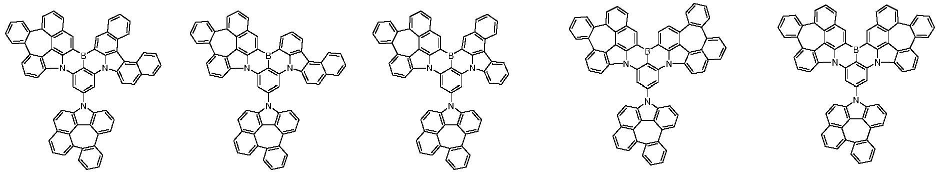

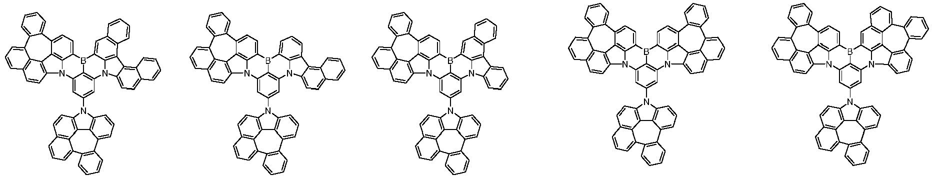

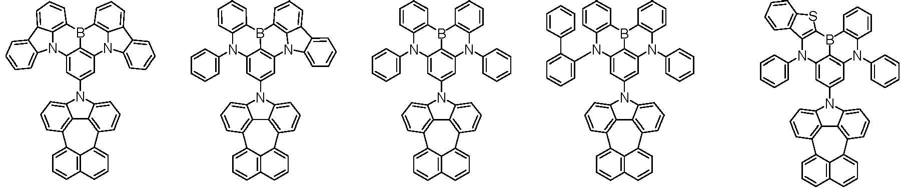

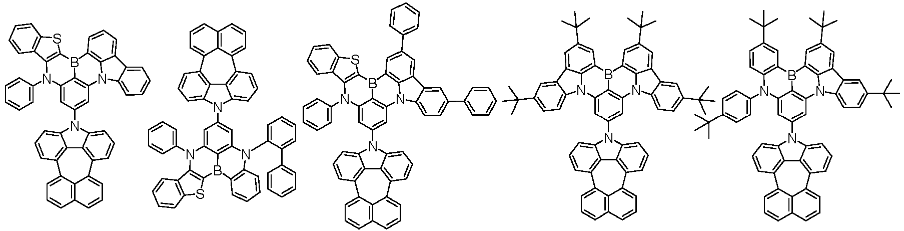

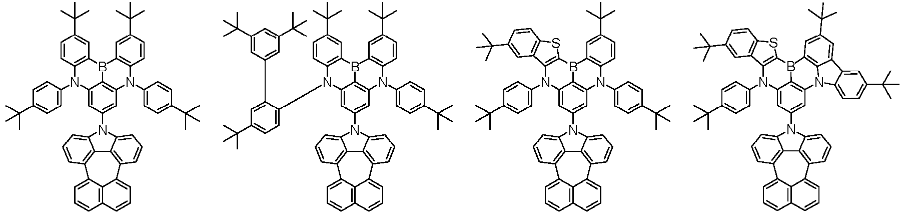

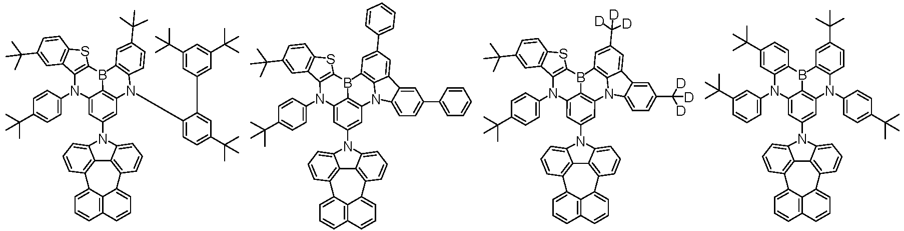

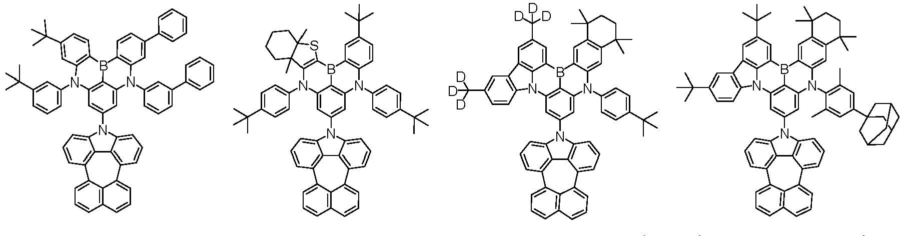

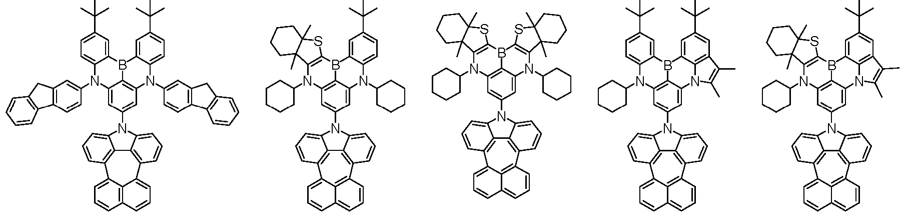

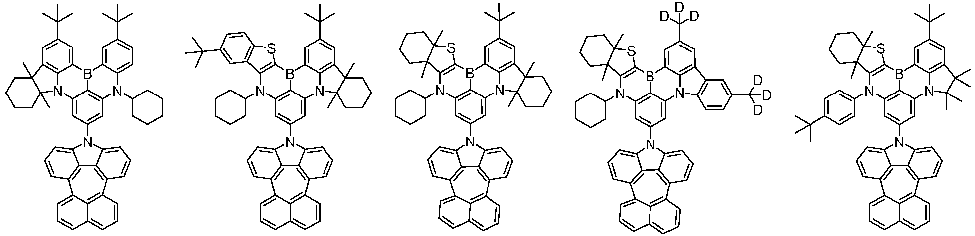

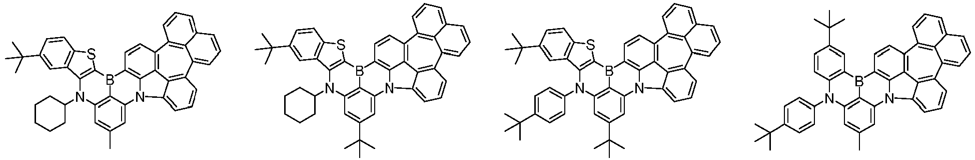

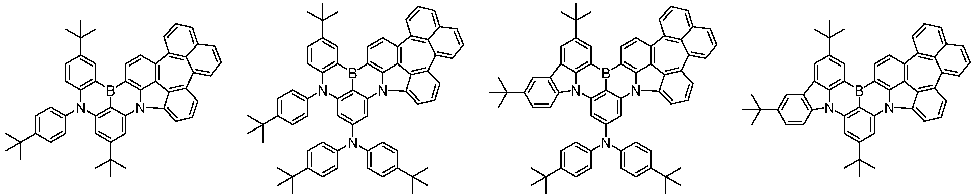

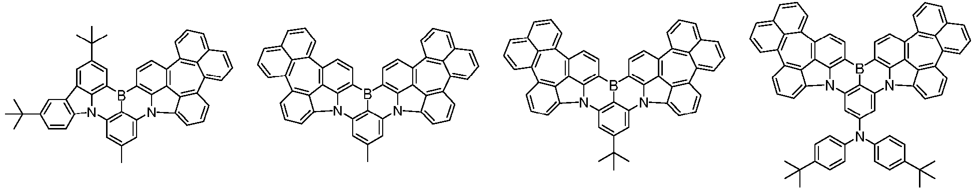

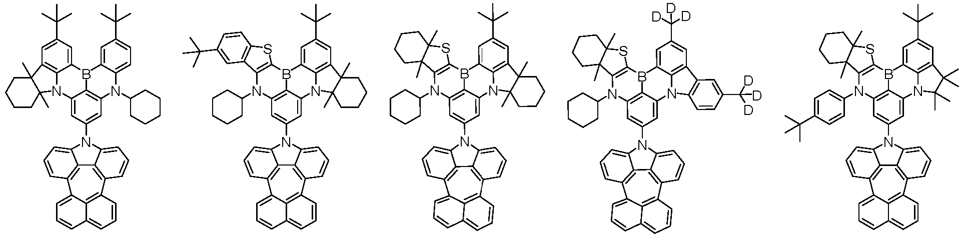

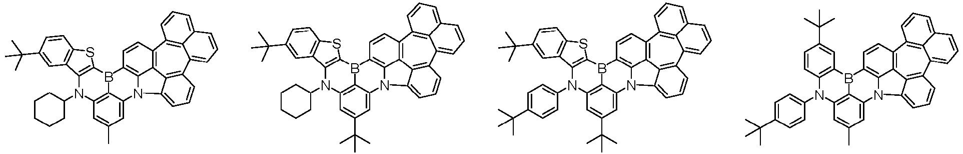

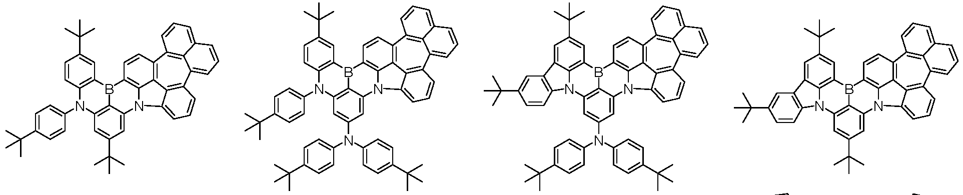

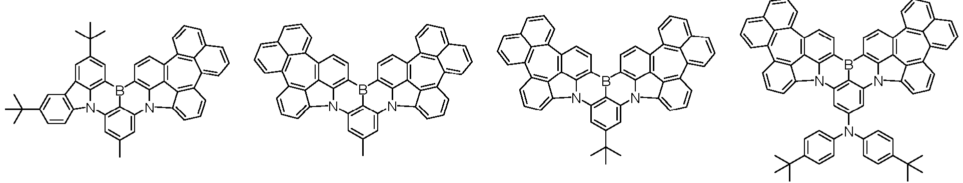

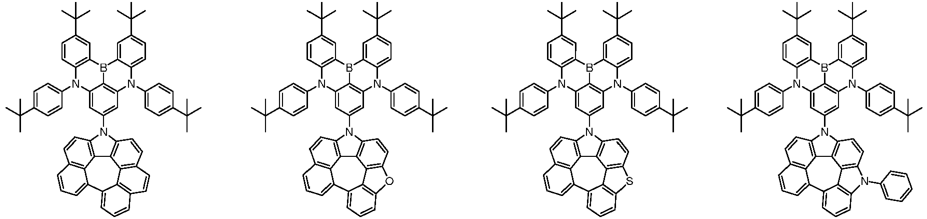

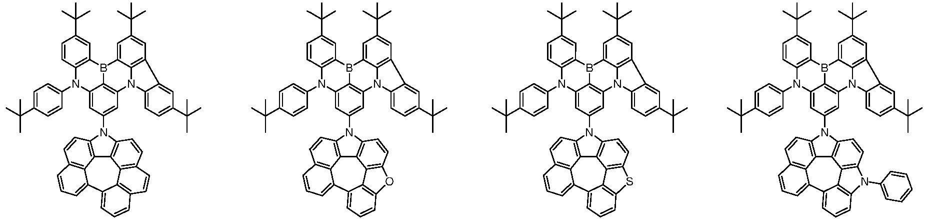

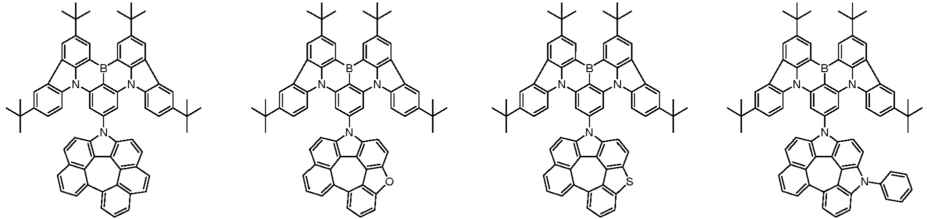

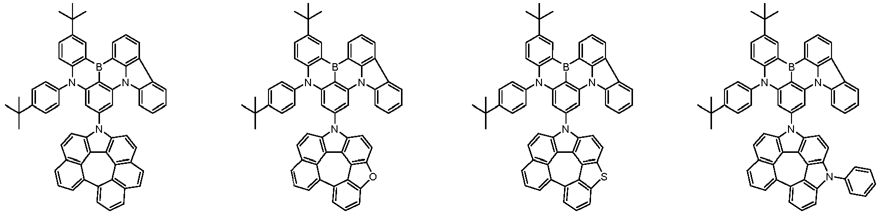

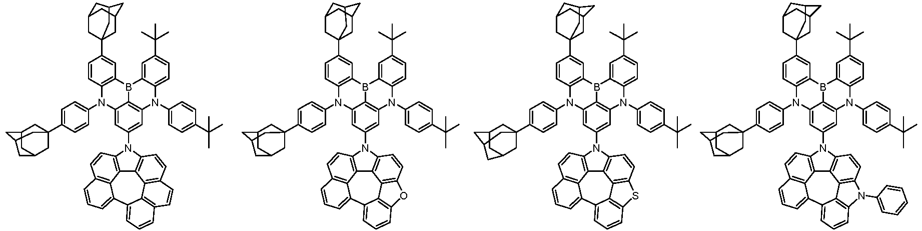

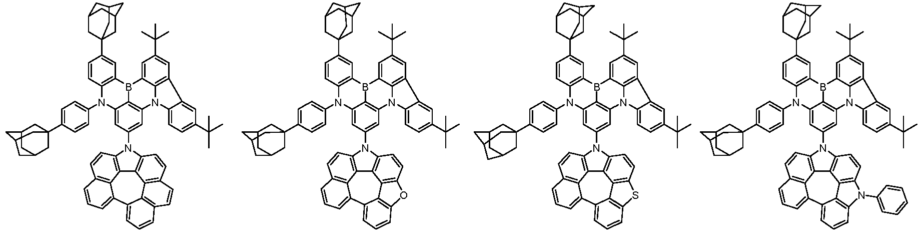

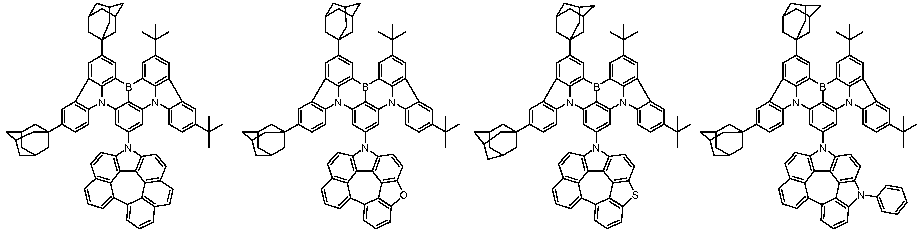

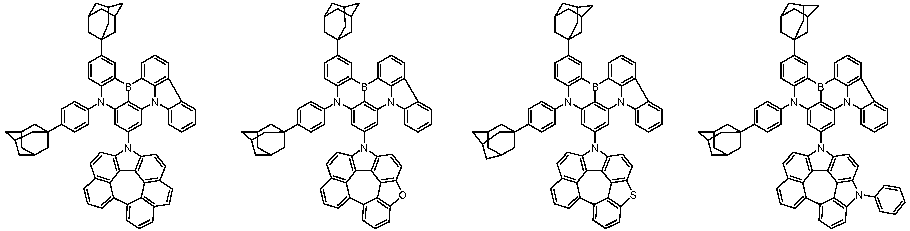

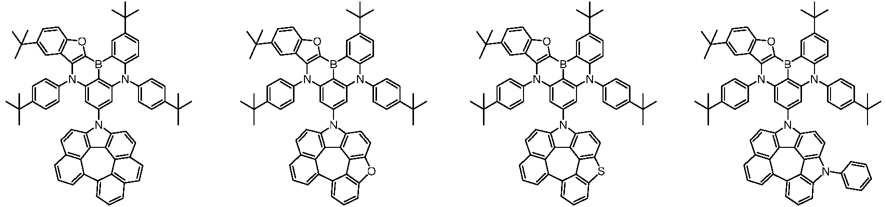

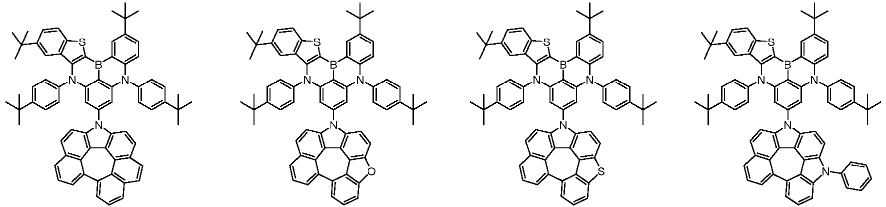

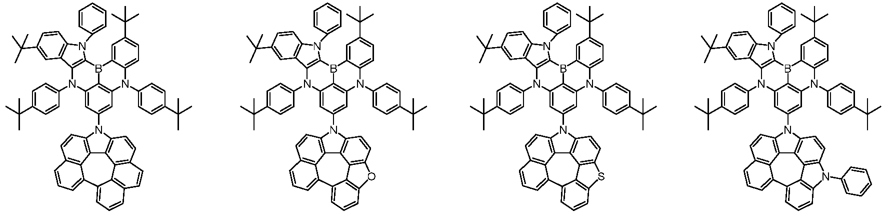

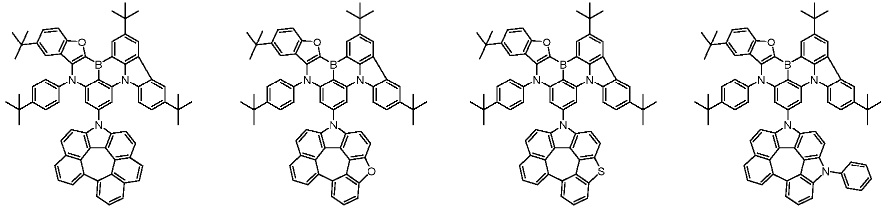

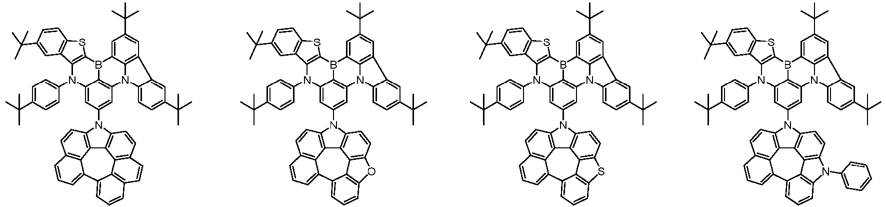

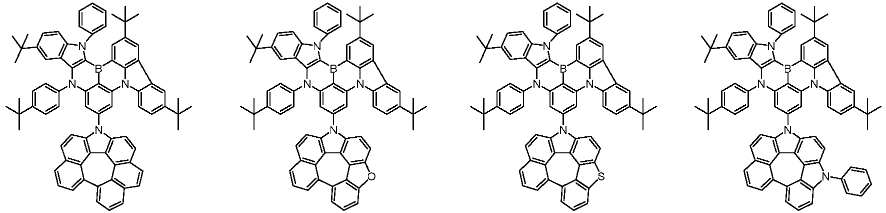

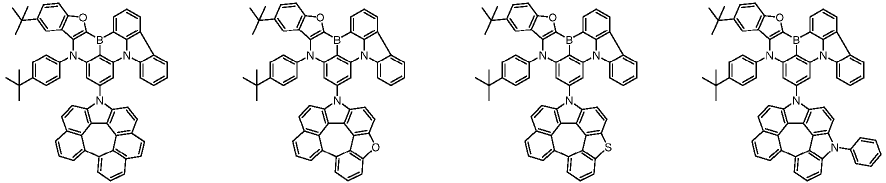

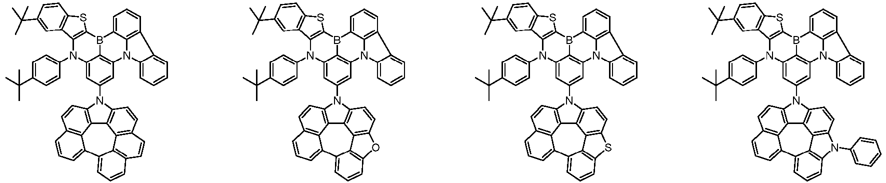

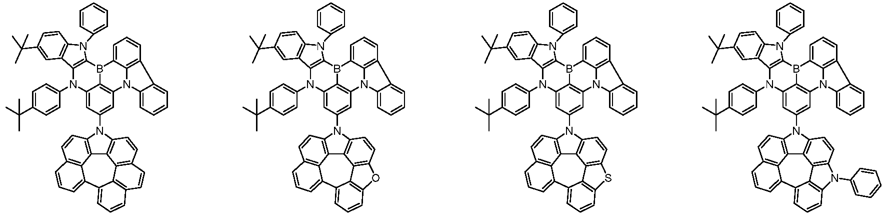

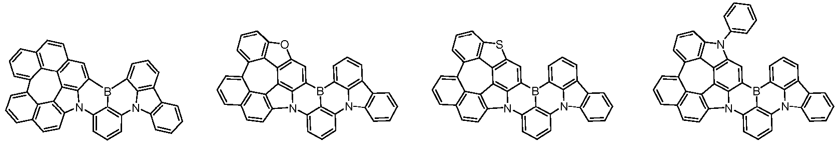

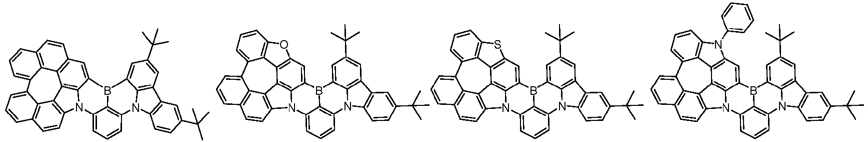

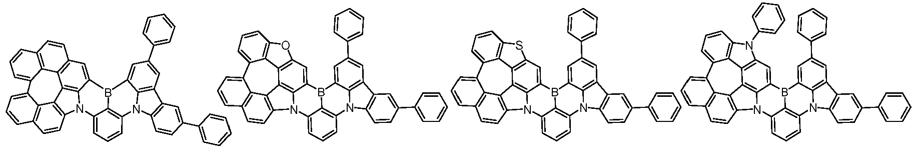

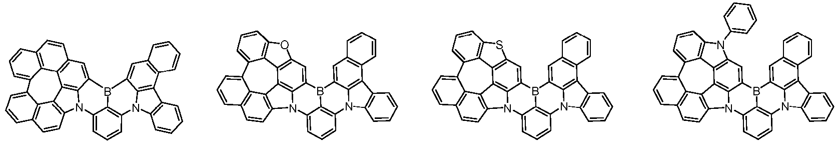

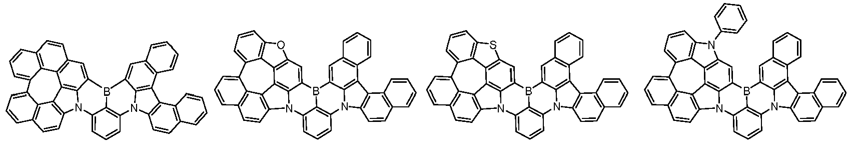

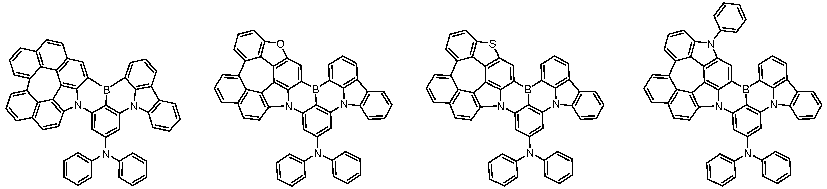

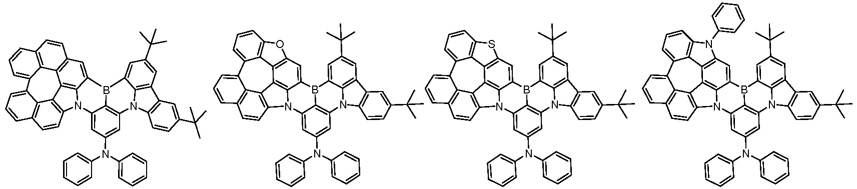

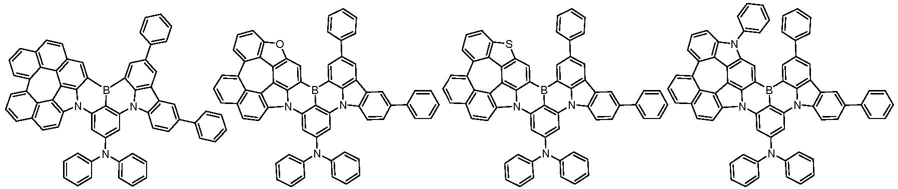

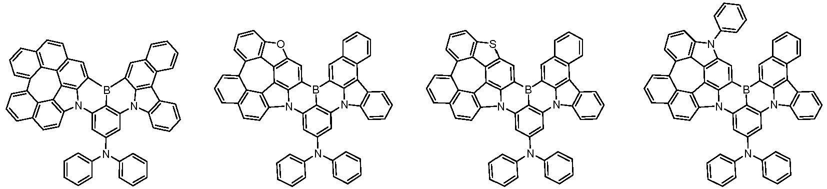

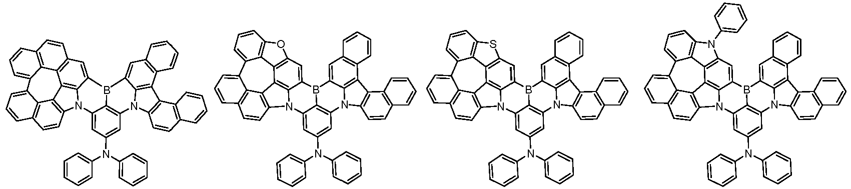

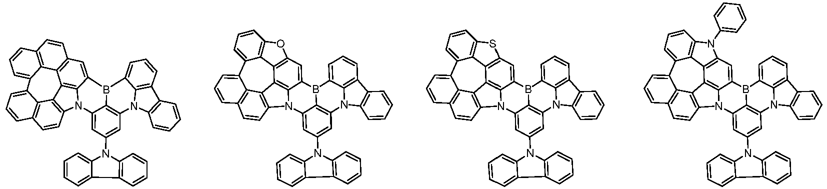

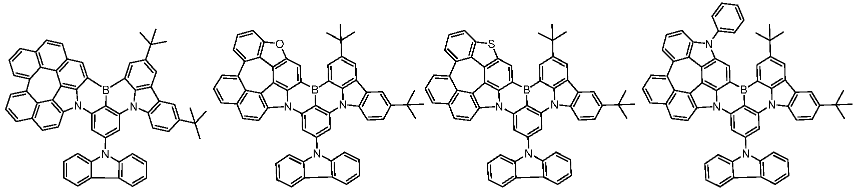

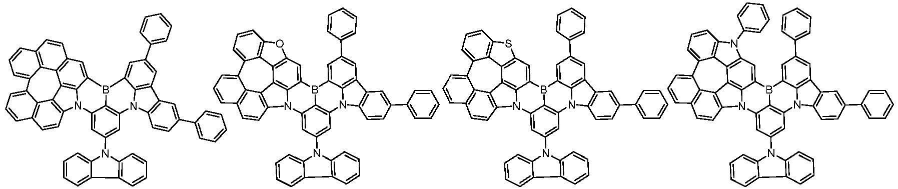

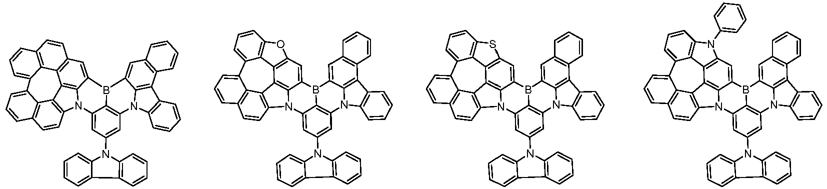

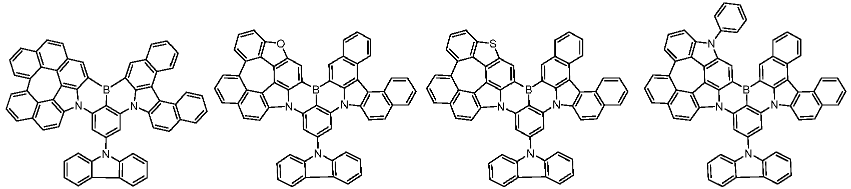

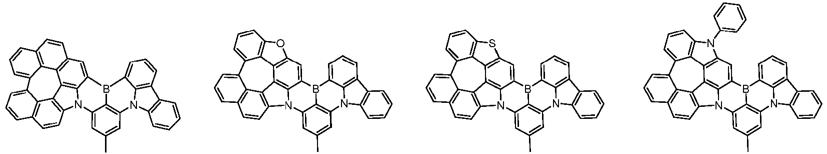

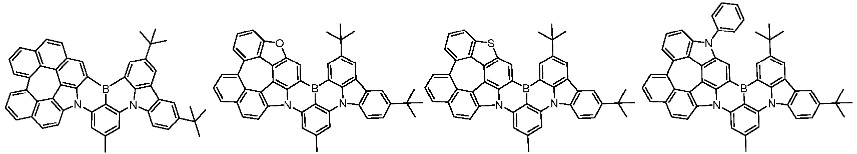

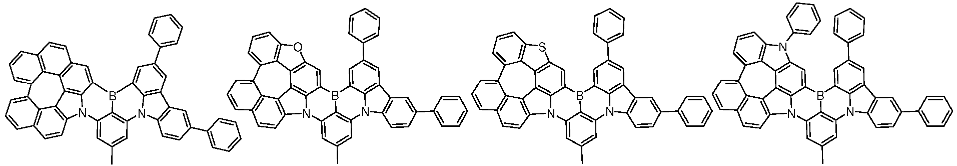

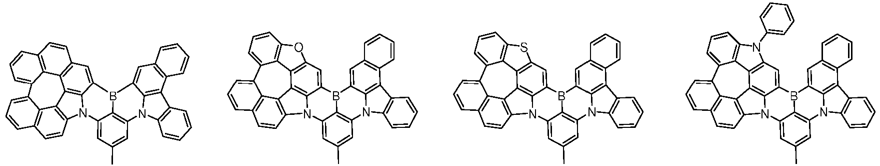

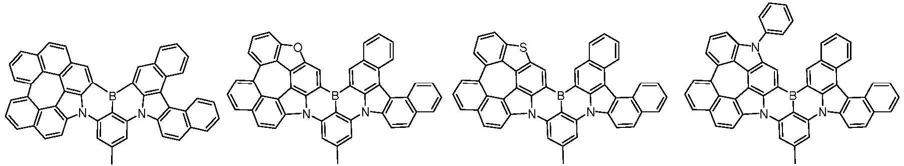

The foregoing organic compounds synthesized in embodiments of this application are mainly classified into three types. One type of compounds are obtained by introducing the six compounds specifically provided from formula (III), formula (IV), and formula (V) as modifying groups to benzene rings linked to boron atoms of boron-nitrogen ring structures to modify the boron-nitrogen ring structures, modifying optical properties of existing boron-nitrogen frameworks. Specifically, in this embodiment, the six compounds specifically provided from formula (III), formula (IV), and formula (V) are introduced to benzene rings linked to both boron atoms and nitrogen atoms and are at para-positions of the boron atoms, so that a frontier orbital energy level of the new organic compound can be effectively adjusted. In addition, the six compounds specifically provided from formula (III), formula (IV), and formula (V) (especially 3H-3-azadibenzo[G,IJ]naphth[2,1,8-CDE]azulene or 4H-naphtho[1',8':5,6,7]cyclohepta[1,2,3,4-def]carbazole) have strong electron-donating abilities, so that multiple resonance effect of boron-nitrogen ring structures on the rigid conjugation plane can be enhanced, thereby adjusting a color of emitted light and narrowing an FHWM on a luminescence spectrum of the new organic compound.

Another type of compounds are obtained based on a new framework structure designed by introducing the six compounds specifically provided from formula (III), formula (IV), and formula (V) into frameworks of boron-nitrogen rings, so that the boron atom is at a para-position of a nitrogen atom on the compound such as 3H-3-azadibenzo[G,IJ]naphth[2,1,8-CDE]azulene or 4H-naphtho[1',8':5,6,7]cyclohepta[1,2,3,4-def]carbazole on a same conjugated six-membered ring, generating hyperconjugation effect between the boron atom and the nitrogen atom, which can improve light-emitting efficiency of the new organic compound and reduce an FWHM on a luminescence spectrum. In addition, the compound such as 3H-3-azadibenzo[G,IJ]naphth[2,1,8-CDE]azulene or 4H-naphtho[1',8':5,6,7]cyclohepta[1,2,3,4-def]carbazole can increase a conjugation length of the entire molecule, and an electron cloud on the compound such as 3H-3-azadibenzo[G,IJ]naphth[2,1,8-CDE]azulene or 4H-naphtho[1',8':5,6,7]cyclohepta[1,2,3,4-def]carbazole is also involved in super-resonance effect of the entire molecule, which can effectively reduce an FWHM value on a luminescence spectrum of the new organic compound, and can cause a red shift to a sufficient degree on the spectrum of the new organic compound relative to that of the basic ring structure, to adjust a color of light from a blue light region to a green light region to emit green light with a high color rendering index. Moreover, the compound such as 3H-3-azadibenzo[G,IJ]naphth[2,1,8-CDE]azulene or 4H-naphtho[1',8':5,6,7]cyclohepta[1,2,3,4-def]carbazole includes no free aryl, and N is at a para-position of B on a conjugated six-membered ring, with no free rotation, thereby improving planarity of the molecules of the new organic compound, and further narrowing the FWHM on the luminescence spectrum of the new organic compound.

The third type of compounds are obtained by introducing the six compounds specifically provided from formula (III), formula (IV), and formula (V) into frameworks of boron-nitrogen rings, and modifying the boron-nitrogen ring structures using the six compounds such as 3H-3-azadibenzo[G,IJ]naphth[2,1,8-CDE]azulene or 4H-naphtho[1',8':5,6,7]cyclohepta[1,2,3,4-def]carbazole as modifying groups, which can combine the advantages of the foregoing two types of compounds, further improving light-emitting efficiency of the organic compound, narrowing an FWHM on a luminescence spectrum, and the like.

Based on a same inventive concept, the foregoing method for preparing the organic compound by introducing the structure shown in formula (II) into a main chain or side group of formula (I) specifically includes the following steps:
Step S101: Substitute X₂ or X₃ in a compound 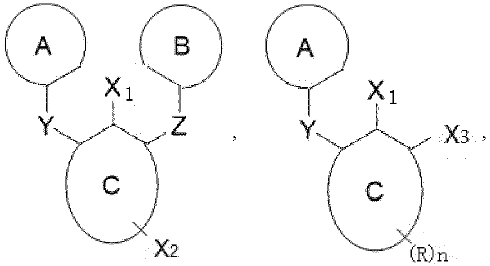 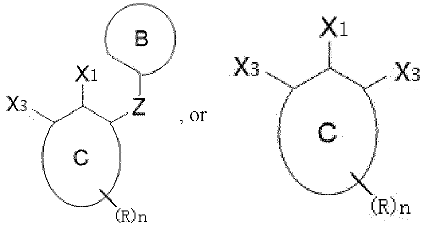 with a compound 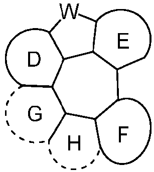 to obtain an intermediate 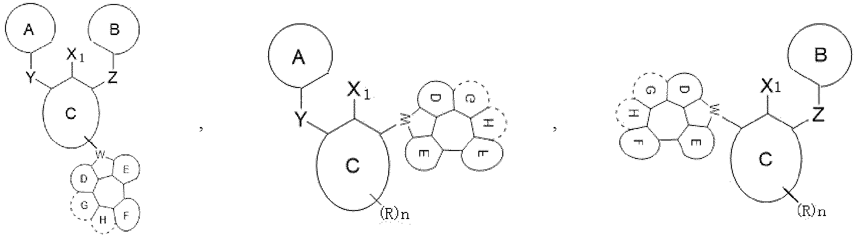 , or 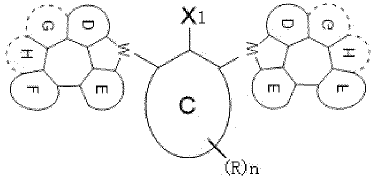 , where in the foregoing structural formulas, X₁, X₂, and X₃ are each independently selected from a halogen atom, specifically, X₁ may be a chlorine atom, X₂ may be fluorine or bromine, and X₃ may be fluorine;
a ring A, a ring B, a ring C, a ring D, a ring E, a ring F, a ring G, and a ring H are each independently selected from substituted or unsubstituted aryl, substituted or unsubstituted heteroaryl, or a substituted or unsubstituted fused-ring structure;
W is selected from C(R₁, R₂), N-R₃, P, P=O, or Al;
Y and Z are each independently selected from C=O, N-R₄, O, S, Se, P, P=O, or P=S, and when Y and Z are both present, at least one of Y and Z is N-R₄;
R and R₁ to R₄ are each independently selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms; and
n is an integer greater than or equal to 0.
Step S102: React the intermediate 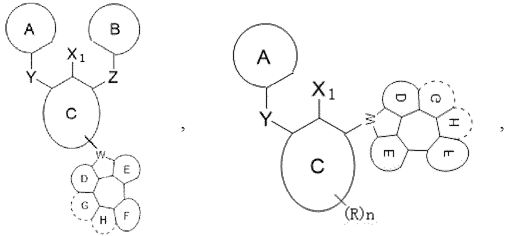 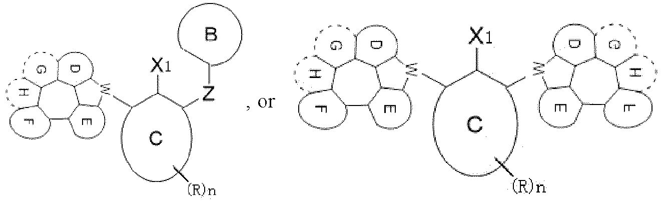 with an X-containing compound to obtain the organic compound 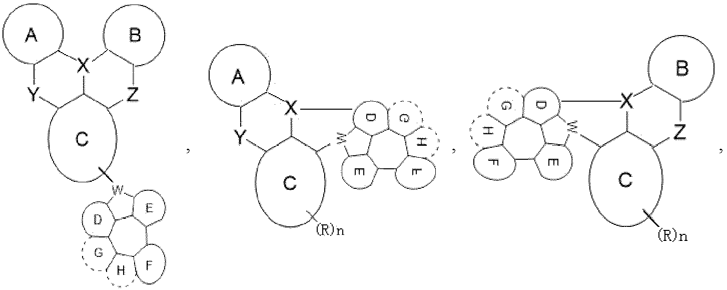 or 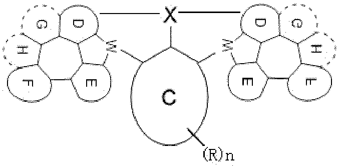 , where X is selected from B, N, P, P=O, or Al.

Based on a same inventive concept, the foregoing method for preparing the organic compound by introducing the structure shown in formula (II) into both a main chain and side group of formula (I) specifically includes the following steps:
Step S201: Substitute X₂ and X₃ in a compound 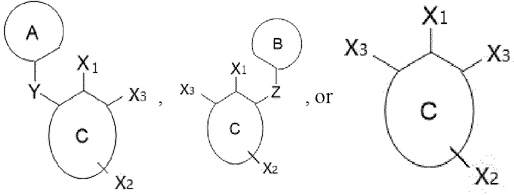 with a compound 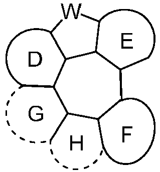 to obtain an intermediate 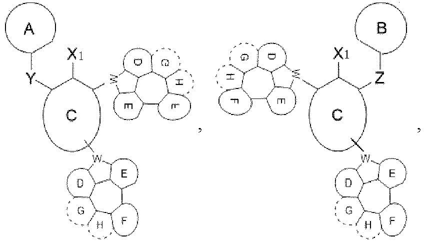 or 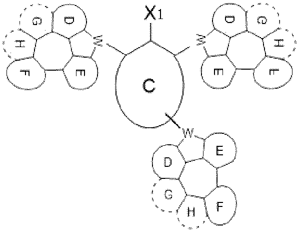 , where in the foregoing structural formulas, X₁, X₂, and X₃ are each independently selected from a halogen atom, specifically, X₁ may be a chlorine atom, X₂ may be fluorine or bromine, and X₃ may be fluorine;
a ring A, a ring B, a ring C, a ring D, a ring E, a ring F, a ring G, and a ring H are each independently selected from substituted or unsubstituted aryl, substituted or unsubstituted heteroaryl, or a substituted or unsubstituted fused-ring structure;
W is selected from C(R₁, R₂), N-R₃, P, P=O, or Al;
Y and Z are each independently selected from C=O, N-R₄, O, S, Se, P, P=O, or P=S, and when Y and Z are both present, at least one of Y and Z is N-R₄; and
R and R₁ to R₄ are each independently selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms.
Step S202: React the intermediate 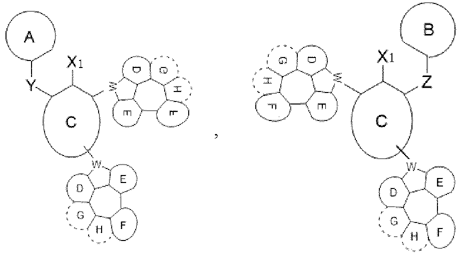 , or 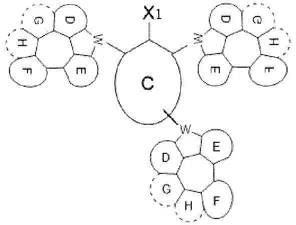 with an X-containing compound to obtain the organic compound 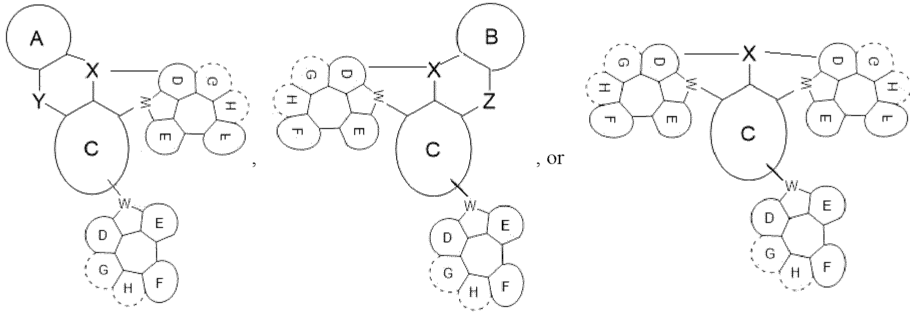 , where X is selected from B, N, P, P=O, or Al.

An embodiment of this application further provides a polymer. The polymer includes at least one first repeating unit, and the first repeating unit is derived from at least one new organic compound described above. A molecular framework of the polymer may be formed by at least one new organic compound.

In some embodiments, the polymer may be a multimer with a plurality of first repeating units. Usually, a multimer includes two to eight first repeating units. A multimer is in a form of a compound with a plurality of first repeating units. For example, the multimer may be obtained by bonding a plurality of first repeating units using a single bond, C1-C3 alkylene (for example, methylene), phenylene, naphthylene, or another structure (into a linked multimer), or may be obtained by bonding in a manner of the plurality of first repeating units sharing any ring (the ring A, the ring B, the ring C, the ring D, the ring E, the ring F, the ring G, and the ring H) contained in the first repeating units (into a ring-shared multimer), or may be obtained by bonding in a manner of condensing any ring (the ring A, the ring B, the ring C, the ring D, the ring E, the ring F, the ring G, and the ring H) contained in the first repeating units (into a ring-condensed multimer). Further, the multimer is a ring-shared multimer and a ring-condensed multimer, and even further, is a ring-shared multimer. The ring-shared multimer can improve planarity of molecules of the multimer, which is conducive to further narrowing an FWHM on a luminescence spectrum of the new organic compound.

In some other embodiments, the polymer may be a high polymer with a high molecular weight.

In some other embodiments, the polymer further includes at least one second repeating unit different from the first repeating unit. The foregoing new organic compound in this application may be copolymerized with another organic monomer to form a copolymer.

In some embodiments, the second repeating unit is selected from at least one of the following repeating units: 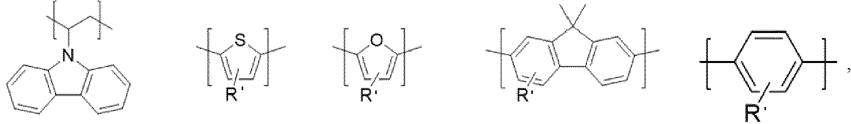 R' in the foregoing repeating units is selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms.

An embodiment of this application further provides another polymer. The polymer includes a polymer molecule main chain and a branched chain linked to the polymer molecule main chain, and the branched chain is derived from the foregoing new organic compound. Alternatively, another polymer may be used as a framework structure, and the framework of the polymer is modified by using the foregoing new organic compound.

In some embodiments, the polymer as the framework structure may be a polymer functional material, for example, a hole injection material, hole transport material, electron transport material, electron injection material, electron blocking material, hole blocking material, light-emitting body, and host material of a polymer type.

In some embodiments, the polymer includes at least one of the following repeating units: 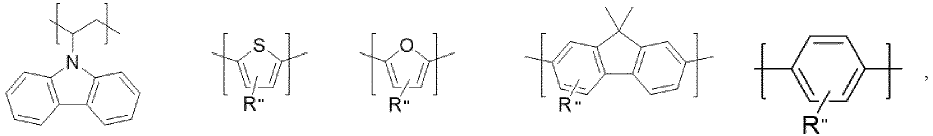 where R" in the foregoing repeating units is selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms.

An embodiment of this application further provides a composition, including an organic solvent and at least one new organic compound described above or at least one polymer described above. The foregoing new organic compound and the foregoing polymer can be dissolved or suspended in the organic solvent, which facilitates film formation. The composition may be a solution or may be a suspension.

In some embodiments, the organic solvent is selected from an aromatic or heteroaromatic compound, an ester, an aromatic ketone or aromatic ether, an aliphatic ketone or aliphatic ether, an alicyclic or olefinic compound, a borate or phosphate compound, or a mixture of any two or more of the foregoing solvents.

In some embodiments, the organic solvent is selected from a solvent based on an aromatic or heteroaromatic compound. Examples of the solvent based on an aromatic or heteroaromatic compound suitable for the foregoing new organic compound or the foregoing polymer in this application include but are not limited to: p-diisopropylbenzene, pentylbenzene, tetrahydronaphthalene, cyclohexylbenzene, chloronaphthalene, 1,4-dimethylnaphthalene, 3-isopropylbiphenyl, p-methylisopropylbenzene, dipentylbenzene, tripentylbenzene, pentyltoluene, o-diethylbenzene, m-diethylbenzene, p-diethylbenzene, 1,2,3,4-tetramethylbenzene, 1,2,3,5-tetramethylbenzene, 1,2,4,5-tetramethylbenzene, butylbenzene, dodecylbenzene, dihexylbenzene, dibutylbenzene, benzylbutylbenzene, dimethylnaphthalene, 1-methylnaphthalene, 1,2,4-trichlorobenzene, 4,4-difluorodiphenylmethane, 1,2-dimethoxy-4-(1-propenyl)benzene, diphenylmethane, 2-phenylpyridine, 3-phenylpyridine, N-methyldiphenylamine, 4-isopropylbiphenyl, α,α-dichlorodiphenylmethane, 4-(3-phenylpropyl)pyridine, benzyl benzoate, 1,1-bis(3,4-dimethylphenyl)ethane, 2-isopropylnaphthalene, quinoline, isoquinoline, methyl 2-furancarboxylate, and ethyl 2-furancarboxylate.

An embodiment of this application further provides a mixture. The mixture includes an organic functional material and at least one new organic compound described above or at least one polymer described above or at least one mixture described above. The organic functional material is selected from at least one of a hole injection material, a hole transport material, an electron transport material, an electron injection material, an electron blocking material, a hole blocking material, a light-emitting body, and a host material. The light-emitting body is selected from a singlet-state light-emitting body (fluorescent light-emitting body), a triplet-state light-emitting body (phosphorescent light-emitting body), and an organic thermally activated delayed fluorescence material (TADF material). For example, various organic functional materials are described in detail in WO2010135519A1, US20090134784A1, and WO2011110277A1, and these three patent documents are hereby incorporated herein by reference in their entireties.

In some embodiments, the organic functional material is a host material, specifically a blue light host material or a green light host material.

In this application, the organic compound, the polymer, the composition, or the mixture may be used as a material for an organic device. The organic device may be an organic electroluminescent device, an organic field-effect transistor, an organic thin-film solar cell, or the like.

The material for the organic device may include at least one functional layer. The functional layer is made of the organic compound, the polymer, the composition, or the mixture, which may be an ultrathin film layer specifically formed by vapor deposition or the like. In some embodiments, the functional layer may exhibit different functions based on different types of organic functional materials, which may be specifically one or a combination of the following: a hole injection layer, a hole transport layer, an electron transport layer, an electron injection layer, an electron blocking layer, a hole blocking layer, and a light-emitting layer.

One of the functional layers may be a light-emitting layer. In particular, in an organic electroluminescent device, the organic compound or the polymer may be used as a dopant material (that is, a guest material) of the light-emitting layer. The organic device using the organic compound has high light-emitting efficiency, a narrow FWHM on a luminescence spectrum, a long luminescence lifetime, and the like.

An embodiment of this application further provides an organic device. The organic device includes at least one functional layer described above. The organic device further includes two electrodes, respectively a cathode and an anode. The at least one functional layer is stacked between the cathode and the anode. The organic device may be but is not limited to an organic light-emitting diode (OLED), an organic photovoltaic (OPV) cell, an organic light-emitting electrochemical cell (OLEEC), an organic field-effect transistor (OFET), an organic light-emitting field-effect transistor, an organic laser, an organic spintronic device, an organic sensor, and an organic plasmon emitting diode (Organic Plasmon Emitting Diode), preferably an organic electroluminescent device, such as an OLED, an OLEEC, or an organic light-emitting field-effect transistor.

In this application, the organic device is an organic light-emitting device, specifically an electroluminescent device, including a light-emitting layer. The light-emitting layer includes the organic compound or the polymer. The foregoing electroluminescent device, especially the OLED, includes at least a substrate, an anode, at least one light-emitting layer, and a cathode. As the foregoing new compound is used in the electroluminescent device, the electroluminescent device has high light-emitting efficiency, high color purity, high device stability, a long device service life, and the like.

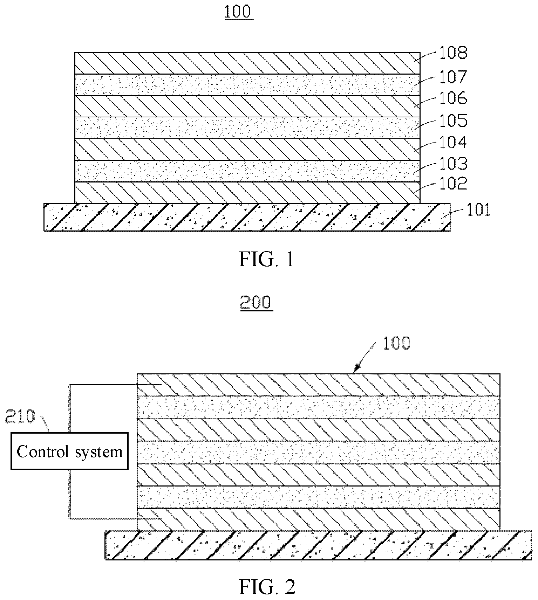

Refer to FIG. 1. An embodiment of this application provides an organic device 100, including: a substrate 101, an anode 102 disposed on the substrate 101, a hole injection layer 103 provided on the anode 102, a hole transport layer 104 provided on the hole injection layer 103, a light-emitting layer 105 provided on the hole transport layer 104, an electron transport layer 106 provided on the light-emitting layer 105, an electron injection layer 107 provided on the electron transport layer 106, and a cathode 108 disposed on the electron injection layer 107.

It may be understood that the organic device 100 may alternatively be formed in an opposite manufacturing sequence into the following composition. The organic device 100 includes: a substrate 101, a cathode 108 disposed on the substrate 101, an electron injection layer 107 provided on the cathode 108, an electron transport layer 106 provided on the electron injection layer 107, a light-emitting layer 105 provided on the electron transport layer 106, a hole transport layer 104 provided on the light-emitting layer 105, a hole injection layer 103 provided on the hole transport layer 104, and an anode 102 disposed on the hole injection layer 103.

It may be understood that not all of the layers are required in the organic device 100, a minimum composition unit is configured as the anode 102, the light-emitting layer 105, and the cathode 108, and the functional layers including the hole injection layer 103, the hole transport layer 104, the electron transport layer 106, and the electron injection layer 107 are provided according to actual requirements. In addition, each of the layers may include a single layer or may include a plurality of layers.

A combination form of layers forming an electroluminescent device may include a combination form of "substrate/anode/hole injection layer/hole transport layer/light-emitting layer/electron transport layer/electron injection layer/cathode", or may be a combination form of "substrate/anode/hole transport layer/light-emitting layer/electron transport layer/electron injection layer/cathode", "substrate/anode/hole injection layer/light-emitting layer/electron transport layer/electron injection layer/cathode", "substrate/anode/hole injection layer/hole transport layer/light-emitting layer/electron injection layer/cathode", "substrate/anode/hole injection layer/hole transport layer/light-emitting layer/electron transport layer/cathode", "substrate/anode/light-emitting layer/electron transport layer/electron injection layer/cathode", "substrate/anode/hole transport layer/light-emitting layer/electron injection layer/cathode", "substrate/anode/hole transport layer/light-emitting layer/electron transport layer/cathode", "substrate/anode/hole injection layer/light-emitting layer/electron injection layer/cathode", "substrate/anode/hole injection layer/light-emitting layer/electron transport layer/cathode", "substrate/anode/light-emitting layer/electron transport layer/cathode", or "substrate/anode/light-emitting layer/electron injection layer/cathode".

The substrate 101 is a support for the organic device 100, and may be opaque or transparent. A transparent substrate may be used for manufacturing a transparent light-emitting device. The substrate 101 may be rigid or elastic. The substrate 101 may be plastic, a metal, a semiconductor wafer, or glass. The substrate 101 optimally provides a smooth surface. A substrate with no surface disadvantage is particularly desirable. In some embodiments, the substrate 101 is flexible, and may be selected from a polymer film or plastic, with a glass transition temperature Tg of 150°C or more, preferably more than 200°C, more preferably more than 250°C, and optimally more than 300°C.

In some embodiments, the substrate 101 is formed into a plate, film, or sheet shape as required, such as a glass plate, a metal plate, metal foil, a plastic film, or a plastic sheet, preferably a glass plate, and a plate made of transparent synthetic resin such as polyester, polymethacrylate, polycarbonate, or polysulfone. Soda-lime glass, alkali-free glass, or the like may be used for a glass substrate. In addition, a thickness sufficient to maintain mechanical strength is required, and therefore, for example, at least 0.2 mm is required. An upper limit of the thickness is, for example, 2 mm, preferably 1 mm. For a material of glass, as fewer released ions from the glass are preferred, the glass is preferably alkali-free glass, and soda-lime glass may also be used because the soda-lime glass to which an isolation coating, for example, SiO₂, is applied is commercially available. In addition, to improve a gas barrier property, a fine gas barrier film, for example, a silicon oxide film, may also be provided on at least one side of the substrate 101. Especially, when a plate, film, or sheet made of synthetic resin with a low gas barrier property is used as the substrate 101, a gas barrier film is preferably provided.

The anode 102 is configured to inject holes into the light-emitting layer 105. It may be understood that, when the hole injection layer 103 and/or the hole transport layer 104 are/is provided between the anode 102 and the light-emitting layer 105, holes are injected into the light-emitting layer 105 through these layers.

In some embodiments, a material for forming the anode 102 may include an inorganic compound and an organic compound. The inorganic compound, for example, may include a metal (aluminum, gold, silver, nickel, palladium, chromium, or the like), a metal oxide (an indium oxide, a tin oxide, an indium-tin oxide (Indium Tin Oxide, ITO), an indium-zinc oxide (Indium Zinc Oxide, IZO), or the like), a halogenated metal (copper iodide or the like), copper sulfide, carbon black, ITO glass, NESA (NESA) glass, or the like. The organic compound, for example, may include polythiophene such as poly(3-methylthiophene), polypyrrole, polyaniline, and other conductive polymers. In addition, a material for an anode of an organic electroluminescent device may be appropriately selected and used. A resistance of a transparent electrode is not limited as long as sufficient current for light emitting by the light-emitting device can be supplied, but in terms of power consumption of the light-emitting device, a low resistance is desirable. A thickness of an ITO may be selected based on a resistance value, but is usually 50 nm to 300 nm in most cases.

The hole injection layer 103 is used to efficiently inject holes that migrate from the anode 102 into the light-emitting layer 105 or the hole transport layer 104. The hole transport layer 104 is used to efficiently transport, to the light-emitting layer 105, holes injected from the anode 102 or holes injected from the anode 102 through the hole injection layer 103. The hole injection layer 103 and the hole transport layer 104 are each formed by stacking and mixing one or more of hole injection/transport materials, or are each formed from a mixture of a hole injection/transport material and a polymer binder.

The light-emitting layer 105 is a layer that emits light by recombining, between the electrodes to which an electric field is applied, holes injected from the anode 102 with electrons injected from the cathode 108. A material for forming the light-emitting layer 105 is a compound (light-emitting compound) that emits light through excitation by recombining holes with electrons, preferably a compound that can be formed into a stable film shape and that can show strong light-emitting (fluorescence) efficiency in a solid state. In the present invention, the organic compound shown in formula (I) may be used as a material for the light-emitting layer.

In some embodiments, the light-emitting layer may be a single layer, or may include a plurality of layers that are respectively formed from materials (a host material and a dopant material) for the light-emitting layer. There may be one or a combination of materials for each of the host material and the dopant material. The dopant material may be contained in the entire host material, or may be contained in a part of the host material. For a doping method, the dopant material may be co-evaporated with the host material, or may be mixed with the host material in advance and then evaporated together, or may be mixed with an organic solvent and the host material in advance, and then subjected to wet film formation to form a film.

In some embodiments, an amount of the used host material varies based on types of the host material, as long as the amount matches a feature of the host material. The host material may be the new organic compound or the polymer in this application, or may be the following substances known as light-emitting bodies: a fused-ring derivative such as anthracene or pyrene, a bisstyryl derivative such as a bisstyryl anthracene derivative or a bisstyryl benzene derivative, a tetraphenylbutadiene derivative, a cyclopentadiene derivative, a fluorene derivative, a benzofluorene derivative, and the like.

In some embodiments, an amount of the used dopant material varies based on types of the dopant material, as long as the amount matches a feature of the dopant material. The dopant material may be the new organic compound or the polymer in this application, or may be an existing common dopant material selected from various materials based on a desired luminescent color, for example, a thermally activated delayed fluorescence dopant material.

In some embodiments, the light-emitting layer 105 is made of the organic compound, the polymer, the composition, or the mixture in this application.

The electron injection layer 107 is used to efficiently inject electrons that migrate from the cathode 108 into the light-emitting layer 105 or the electron transport layer 106. The electron transport layer 106 is used to efficiently transport, to the light-emitting layer 105, electrons injected from the cathode 108 or electrons injected from the cathode 108 through the electron injection layer 107. The electron transport layer 106 and the electron injection layer 107 are each formed by stacking and mixing one or more of electron transport/injection materials, or are each formed from a mixture of an electron transport/injection material and a polymer binder.

In some embodiments, a material (an electron transport material) for forming the electron transport layer 106 or the electron injection layer 107 may be selected from a compound commonly used as an electron transport compound in a photoconductive material or an existing compound used in an electron injection layer and an electron transport layer of an organic electroluminescent device.

Refer to FIG. 2. This application further provides a display apparatus 200 using the foregoing organic device 100. The display apparatus 200 includes a control system 210 and at least one organic device 100 described above. The control system 210 is configured to control the organic device 100. The control system 210 may be electrically connected to the anode 102 and the cathode 108 of the organic device 100, to control the organic device 100 to emit light.

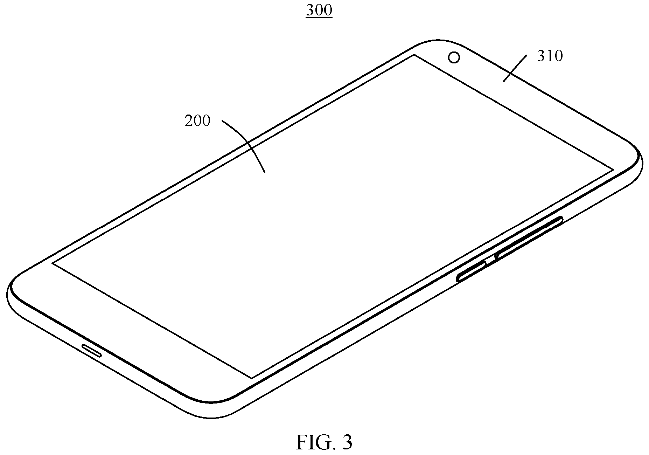

Refer to FIG. 3. This application further provides an electronic device 300 using the foregoing organic device 100. The electronic device 300 includes a housing 310 and the foregoing display apparatus 200 disposed on the housing 310. The electronic device 300 may be various terminal devices equipped with an OLED display screen, including but not limited to a smartphone, a tablet computer, a personal notebook computer, a smart television, an on-board display, and a smartwatch. In this embodiment, the electronic device 300 is a smartphone.

The following further describes embodiments of this application by using specific embodiments.

### Synthesis Example 1

### Synthesis of compound 1:

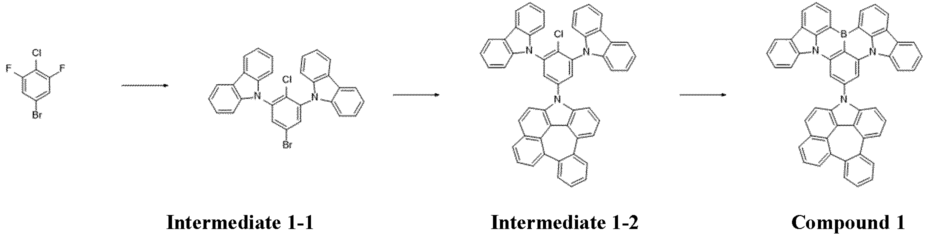

5-bromo-2-chloro-1,3-difluorobenzene (50 g), carbazole (110 g), and anhydrous cesium carbonate (285 g) were dissolved in anhydrous N,N-dimethylformamide (500 mL), heated to 100°C, and stirred for 8 h. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 105 g of intermediate 1-1 with a yield of 92%.

The intermediate 1-1 (100 g), 3H-3-azadibenzo[G,IJ]naphth[2,1,8-CDE]azulene (84 g), activated copper powder (6.2 g), cuprous iodide (18.3 g), anhydrous potassium carbonate (53 g), and 2,2,6,6-tetramethyl-3,5-heptanedione (7 g) were mixed in 1,3-dimethyl-3,4,5,6-tetrahydro-2(1H)-pyrimidinone (200 mL), heated to 150°C, and stirred for 24 h. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 119 g of intermediate 1-2 with a yield of 85%.

The intermediate 1-2 (10 g) was dissolved in tert-butylbenzene (300 mL), and tert-butyllithium (16 mL, 1.3 M n-hexane solution) was added dropwise with an ice bath. After the dropwise addition, the reaction solution was heated to 100°C, and stirred for 2 h. Then, the reaction solution was cooled down in an ice bath, and boron tribromide (5.2 mL) was added dropwise. After the dropwise addition, the reaction solution was heated to 180°C, and stirred for 8 h. Then, N,N-diisopropylethylamine (24 mL) was added dropwise with an ice bath. After the dropwise addition, the reaction solution was heated to 180°C, and stirred overnight. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 3.3 g of compound 1 with a yield of 34%. Product characterization: MS (ASAP) = 705.5.

### Synthesis Example 2

### Synthesis of compound 2:

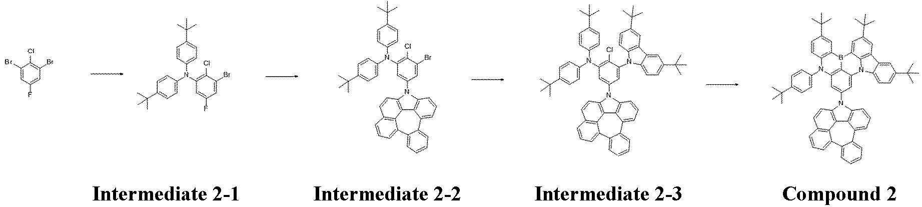

1,3-dibromo-2-chloro-5-fluorobenzene (100 g), 4,4-di-tert-butyldiphenylamine (100 g), tris(dibenzylideneacetone)dipalladium (16 g), 4,5-bis(diphenylphosphino)-9,9-dimethylxanthene (20 g), and anhydrous sodium tert-butoxide (67 g) were dissolved in toluene (500 mL), heated to 90°C, and stirred for 2 h. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 126 g of intermediate 2-1 with a yield of 77%.

The intermediate 2-1 (100 g), 3H-3-azadibenzo[G,IJ]naphth[2,1,8-CDE]azulene (90 g), and anhydrous cesium carbonate (133 g) were dissolved in anhydrous N,N-dimethylformamide (500 mL), heated to 100°C, and stirred for 8 h. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 129 g of intermediate 2-2 with a yield of 83%.

The intermediate 2-2 (100 g), 3,6-di-tert-butylcarbazole (44 g), activated copper powder (4.2 g), cuprous iodide (12.4 g), anhydrous potassium carbonate (36 g), and 2,2,6,6-tetramethyl-3,5-heptanedione (5 g) were mixed in 1,3-dimethyl-3,4,5,6-tetrahydro-2(1H)-pyrimidinone (200 mL), heated to 150°C, and stirred for 24 h. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 98 g of intermediate 2-3 with a yield of 78%.

The intermediate 2-3 (10 g) was dissolved in tert-butylbenzene (300 mL), and tert-butyllithium (16 mL, 1.3 M n-hexane solution) was added dropwise with an ice bath. After the dropwise addition, the reaction solution was heated to 100°C, and stirred for 2 h. Then, the reaction solution was cooled down in an ice bath, and boron tribromide (5.2 mL) was added dropwise. After the dropwise addition, the reaction solution was heated to 180°C, and stirred for 8 h. Then, N,N-diisopropylethylamine (24 mL) was added dropwise with an ice bath. After the dropwise addition, the reaction solution was heated to 180°C, and stirred overnight. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 3.2 g of compound 2 with a yield of 33%. Product characterization: MS (ASAP) = 932.7.

### Synthesis Example 3

### Synthesis of compound 3:

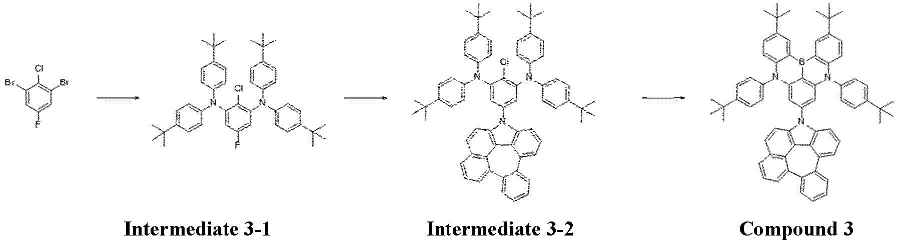

1,3-dibromo-2-chloro-5-fluorobenzene (50 g), 4,4-di-tert-butyldiphenylamine (100 g), tris(dibenzylideneacetone)dipalladium (16 g), tri-tert-butylphosphine tetrafluoroborate (20 g), and anhydrous sodium tert-butoxide (67 g) were dissolved in toluene (500 mL), heated to 90°C, and stirred for 2 h. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 110 g of intermediate 3-1 with a yield of 92%.

The intermediate 3-1 (100 g), 3H-3-azadibenzo[G,IJ]naphth[2,1,8-CDE]azulene (63 g), and anhydrous cesium carbonate (141 g) were dissolved in anhydrous N,N-dimethylformamide (500 mL), heated to 100°C, and stirred for 8 h. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 127 g of intermediate 3-2 with a yield of 91%.

The intermediate 3-2 (10 g) was dissolved in tert-butylbenzene (300 mL), and tert-butyllithium (16 mL, 1.3 M n-hexane solution) was added dropwise with an ice bath. After the dropwise addition, the reaction solution was heated to 100°C, and stirred for 2 h. Then, the reaction solution was cooled down in an ice bath, and boron tribromide (5.2 mL) was added dropwise. After the dropwise addition, the reaction solution was heated to 180°C, and stirred for 8 h. Then, N,N-diisopropylethylamine (24 mL) was added dropwise with an ice bath. After the dropwise addition, the reaction solution was heated to 180°C, and stirred overnight. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 2.7 g of compound 3 with a yield of 28%. Product characterization: MS (ASAP) = 934.1.

### Synthesis Example 4

### Synthesis of compound 4:

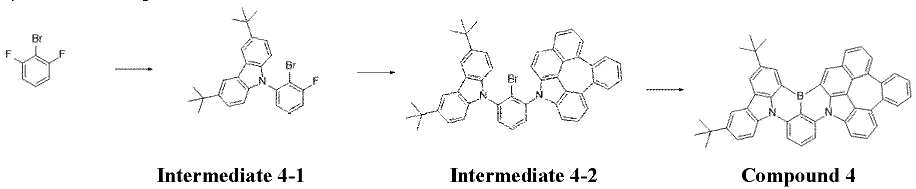

2,6-difluorobromobenzene (100 g), 3,6-di-tert-butylcarbazole (72 g), and anhydrous cesium carbonate (170 g) were dissolved in anhydrous N,N-dimethylformamide (500 mL), heated to 100°C, and stirred for 8 h. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 101 g of intermediate 4-1 with a yield of 87%.

The intermediate 4-1 (100 g), 3H-3-azadibenzo[G,IJ]naphth[2,1,8-CDE]azulene (100 g), and anhydrous cesium carbonate (150 g) were dissolved in anhydrous N,N-dimethylformamide (500 mL), heated to 100°C, and stirred for 8 h. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 147 g of intermediate 4-2 with a yield of 94%.

The intermediate 4-2 (10 g) was dissolved in o-dichlorobenzene (300 mL), and n-butyllithium (6.1 mL, 2.5 M n-hexane solution) was added dropwise with an ice bath. After the dropwise addition, the reaction solution was heated to 100°C, and stirred for 2 h. Then, the reaction solution was cooled down in an ice bath, and boron tribromide (3.8 mL) was added dropwise. After the dropwise addition, the reaction solution was heated to 180°C, and stirred for 8 h. Then, N,N-diisopropylethylamine (18 mL) was added dropwise with an ice bath. After the dropwise addition, the reaction solution was heated to 180°C, and stirred overnight. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 2.5 g of compound 4 with a yield of 28%. Product characterization: MS (ASAP) = 652.6.

### Synthesis Example 5

### Synthesis of compound 5:

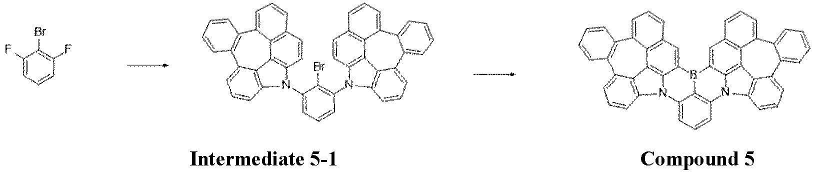

2,6-difluorobromobenzene (50 g), 3H-3-azadibenzo[G,IJ]naphth[2,1,8-CDE]azulene (200 g), and anhydrous cesium carbonate (150 g) were dissolved in anhydrous N,N-dimethylformamide (500 mL), heated to 100°C, and stirred for 8 h. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 202 g of intermediate 5-1 with a yield of 91%.

The intermediate 5-1 (10 g) was dissolved in o-dichlorobenzene (300 mL), and n-butyllithium (6.1 mL, 2.5 M n-hexane solution) was added dropwise with an ice bath. After the dropwise addition, the reaction solution was heated to 100°C, and stirred for 2 h. Then, the reaction solution was cooled down in an ice bath, and boron tribromide (3.8 mL) was added dropwise. After the dropwise addition, the reaction solution was heated to 180°C, and stirred for 8 h. Then, N,N-diisopropylethylamine (18 mL) was added dropwise with an ice bath. After the dropwise addition, the reaction solution was heated to 180°C, and stirred overnight. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 2.5 g of compound 5 with a yield of 28%. Product characterization: MS (ASAP) = 664.2.

### Synthesis Example 6

### Synthesis of compound 6:

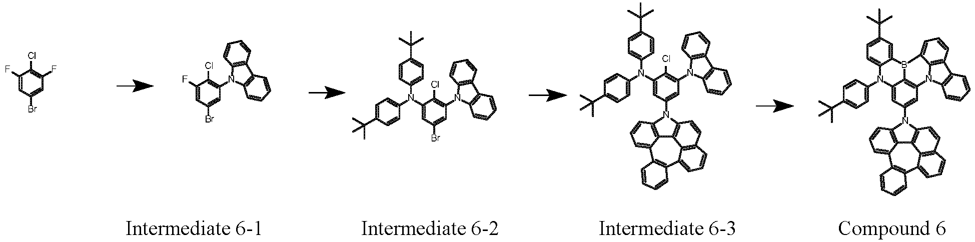

5-bromo-2-chloro-1,3-difluorobenzene (100 g), carbazole (55 g), and anhydrous cesium carbonate (145 g) were dissolved in anhydrous N,N-dimethylformamide (500 mL), heated to 100°C, and stirred for 8 h. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 118 g of intermediate 6-1 with a yield of 96%.

The intermediate 6-1 (50 g), 4,4-di-tert-butyldiphenylamine (40 g), and anhydrous cesium carbonate (100 g) were dissolved in anhydrous N,N-dimethylformamide (500 mL), heated to 100°C, and stirred for 8 h. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 82 g of intermediate 6-2 with a yield of 97%.

The intermediate 6-2 (50 g), 3H-3-azadibenzo[G,IJ]naphth[2,1,8-CDE]azulene (25 g), activated copper powder (3 g), cuprous iodide (9 g), anhydrous potassium carbonate (25 g), and 2,2,6,6-tetramethyl-3,5-heptanedione (3 g) were mixed in 1,3-dimethyl-3,4,5,6-tetrahydro-2(1H)-pyrimidinone (200 mL), heated to 150°C, and stirred for 24 h. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 54 g of intermediate 6-3 with a yield of 82%.

The intermediate 6-3 (10 g) was dissolved in tert-butylbenzene (300 mL), and tert-butyllithium (16 mL, 1.3 M n-hexane solution) was added dropwise with an ice bath. After the dropwise addition, the reaction solution was heated to 100°C, and stirred for 2 h. Then, the reaction solution was cooled down in an ice bath, and boron tribromide (5.2 mL) was added dropwise. After the dropwise addition, the reaction solution was heated to 180°C, and stirred for 8 h. Then, N,N-diisopropylethylamine (24 mL) was added dropwise with an ice bath. After the dropwise addition, the reaction solution was heated to 180°C, and stirred overnight. The reaction solution was cooled down to room temperature. Then, a large amount of deionized water and dichloromethane were added for extraction. An organic phase was retained. An organic solvent was removed by distillation under reduced pressure. Then, purification was performed by silica gel column chromatography to obtain 3.2 g of compound 6 with a yield of 34%. Product characterization: MS (ASAP): 819.9.

### Synthesis Example 7

### Synthesis of compound 7:

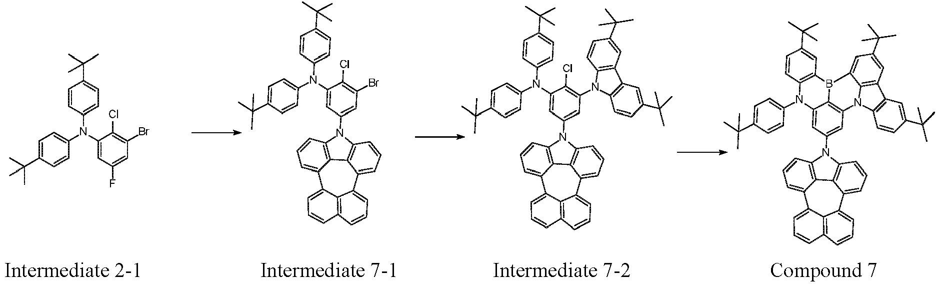

A synthetic route for the compound 7 is essentially the same as that for the compound 2, except that 3H-3-azadibenzo[G,IJ]naphth[2,1,8-CDE]azulene in the step of synthesizing the intermediate 2-2 is replaced by 4H-naphtho[1',8':5,6,7]cyclohepta[1,2,3,4-def]carbazole in an equal amount. A yield of the compound 7 is about 40%. Product characterization: MS (ASAP): 931.5.

### Comparative Synthesis Example 1

An existing boron-nitrogen material has the following structure: 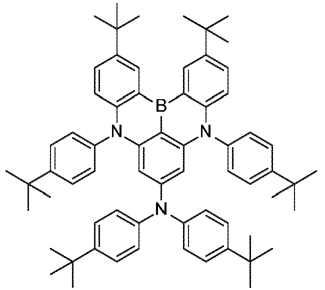 . The existing boron-nitrogen material is specifically synthesized by using an existing synthesis method, and details are not described in this application.

Optical properties of the compounds 1 to 7 in the synthesis examples and Comparative Synthesis Example 1 are shown in Table 1.

**Table 1**

| Sequence number | Luminescence peak (nm) | FWHM (nm) | Fluorescence quantum efficiency (PLQY) (%) |
|---|---|---|---|
| Compound 1 | 469 | 25 | 92 |
| Compound 2 | 463 | 24 | 100 |
| Compound 3 | 454 | 30 | 91 |
| Compound 4 | 510 | 31 | 82 |
| Compound 5 | 523 | 28 | 91 |
| Compound 6 | 461 | 26 | 96 |
| Compound 7 | 465 | 22 | 75 |
| Comparative Synthesis Example 1 | 453 | 26 | 85 |

| | | | |
|---|---|---|---|
| Note: A blue light region is within a wavelength range of 407 nm to 505 nm, and a green light region is within a wavelength range of 505 nm to 525 nm. | | | |

It can be learned from Table 1 that, in the new organic compounds 1, 2, 3, and 6 synthesized in this application, a benzene ring at a para-position of a B atom is modified using 3H-3-azadibenzo[G,IJ]naphth[2,1,8-CDE]azulene as a modifying group, in the compound 7, a benzene ring at a para-position of a B atom is modified using 4H-naphtho[1',8':5,6,7]cyclohepta[1,2,3,4-def]carbazole as a modifying group, and both 3H-3-azadibenzo[G,IJ]naphth[2,1,8-CDE]azulene and 4H-naphtho[1',8':5,6,7]cyclohepta[1,2,3,4-def]carbazole can effectively suppress rotation and vibration of free radicals therein, so that a color of light is adjusted and an FHWM on a luminescence spectrum is narrowed, thereby implementing highly efficient narrow-FHMW blue light emission.

In the compounds 4 and 5, 3H-3-azadibenzo[G,IJ]naphth[2,1,8-CDE]azulene is introduced into the framework of the boron-nitrogen ring structure, making 3H-3-azadibenzo[G,IJ]naphth[2,1,8-CDE]azulene a part of the framework of the ring structure on a rigid conjugation plane, so that a conjugation length of the entire molecule can be increased, improving super-resonance effect of the entire compound, which can effectively narrow an FWHM on a luminescence spectrum of the new organic compound, and can cause a red shift to a sufficient degree on the spectrum of the new organic compound relative to that of the basic boron-nitrogen ring structure, to adjust a color of light from a blue light region to a green light region to achieve a high color rendering index and highly efficient green light emission.

In Comparative Synthesis Example 1, as there is a large torsion angle between a phenyl group linked to a nitrogen atom and a boron-nitrogen rigid conjugation plane, the phenyl group is not directly involved in super-resonance effect of the boron-nitrogen rigid conjugation plane, and cannot be directly involved in a light-emitting process of the boron-nitrogen ring compound. Moreover, such phenyl group usually has large vibrational and rotational degrees of freedom. Consequently, the light-emitting material of the boron-nitrogen ring compound type in Comparative Synthesis Example 1 is deficient in achieving higher light-emitting efficiency and a narrower FWHM.

### Embodiment 1 (Manufacturing of an OLED device 1)

Step S1: Ultrasonically clean an anode (ITO (15 nm)/Ag (150 nm)/ITO (15 nm)) on transparent glass used as a substrate with a stripping solution, pure water, and isopropanol, then dry the anode, and then perform Ar₂ ozone treatment.

Step S2: Move the cleaned substrate into a vacuum vapor deposition device, and control a ratio of PD to HT-1 to be 3:100 under high vacuum (1×10⁻⁶ millibars), to form a 10 nm hole injection layer (HIL).

Step S3: Deposit a 125 nm hole transport layer material HT-1 on the hole injection layer by vacuum evaporation.

Step S4: Deposit a 10 nm electron blocking layer material HT-2 on the hole transport layer by vacuum evaporation.

Step S5: Deposit a light-emitting layer on the electron blocking layer by vacuum evaporation, where a host material is BH, a guest material is the compound 1 in the present invention, a mass ratio is 98:2, and a thickness is 25 nm.

Step S6: Deposit a 2 nm hole blocking layer material ET-1 on the light-emitting layer by vacuum evaporation.

Step S7: Deposit ET-2 and Liq as an electron transport layer on the hole blocking layer by vacuum evaporation, where a mass ratio is 50:50, and a thickness is 35 nm.

Step S8: Deposit 1.5 nm Yb as an electron injection layer on the electron transport layer by vacuum evaporation.

Step S9: Deposit a 17 nm Mg:Ag (1:9) alloy as a cathode on the electron injection layer.

Step S10: Deposit a 55 nm CPL material on the cathode layer.

Structural formulas of the materials used for all the functional layers are shown as follows:

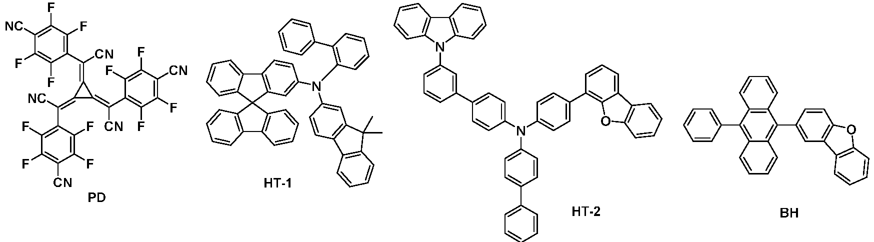

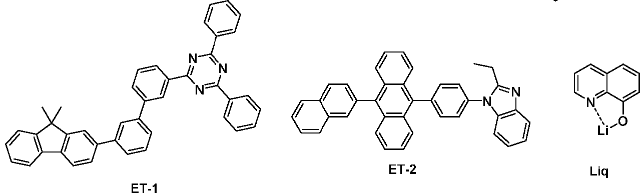

### Embodiment 2 (Manufacturing of an OLED device 2)

A difference between Embodiment 2 and Embodiment 1 lies in that: in step S3, a 120 nm hole transport layer material HT-1 is deposited on the hole injection layer by vacuum evaporation, and in step S5, the guest material is the compound 2. Other steps are the same as those in Embodiment 1. For details, refer to Embodiment 1.

### Embodiment 3 (Manufacturing of an OLED device 3)

A difference between Embodiment 3 and Embodiment 1 lies in that: in step S3, a 122 nm hole transport layer material HT-1 is deposited on the hole injection layer by vacuum evaporation, and in step S5, the guest material is the compound 6. Other steps are the same as those in Embodiment 1. For details, refer to Embodiment 1.

### Comparative Example 1 (Manufacturing of an OLED device 4)

A difference between Comparative Example 1 and Embodiment 1 lies in that: in step S3, a 121 nm hole transport layer material HT-1 is deposited on the hole injection layer by vacuum evaporation, and in step S5, the guest material is the existing boron-nitrogen material in Comparative Synthesis Example 1. Other steps are the same as those in Embodiment 1. For details, refer to Embodiment 1.

Properties of the devices manufactured in Embodiments 1 to 3 and Comparative Example 1 are shown in Table 2.

**Table 2**

| | Voltage (V) | Current efficiency (cd/A) | EQE (%) | Luminescence peak (nm) | FWHM (nm) | LT@1000nit (h) |
|---|---|---|---|---|---|---|
| Embodiment 1 | 3.83 | 9.36 | 12.60 | 470 | 17.2 | 220 |
| Embodiment 2 | 3.37 | 8.71 | 17.90 | 463 | 14.7 | 180 |
| Embodiment 3 | 3.34 | 8.15 | 16.03 | 464 | 15 | 185 |
| Comparative Example 1 | 3.38 | 6.52 | 15.10 | 461 | 18 | 180 |

| | | | | | | |
|---|---|---|---|---|---|---|
| Note: EQE: external quantum efficiency; and | | | | | | |

LT@1000nit: operating duration until a device at 1000 nits of luminance has a 5% maximum luminance loss.

It can be learned from the external quantum efficiency (EQE), the FWHM on the luminescence spectrum, and a service life of the device at 1000 nits of luminance (the operating duration until there is a 5% maximum luminance loss) of the devices in Embodiments 1 to 3 and Comparative Example 1 in Table 2 that, compared with Comparative Example 1, the OLED devices in Embodiments 1 to 3 using the new organic compounds synthesized in this application exhibit higher light-emitting efficiency and narrower FWHMs, and have longer service lives and higher device stability.

It needs to be noted that the foregoing descriptions are merely specific implementations of this application, but are not intended to limit the protection scope of this application. Any variation or replacement readily figured out by a person skilled in the art within the technical scope disclosed in this application shall fall within the protection scope of this application. In the case of no conflict, the implementations of this application and the features in the implementations may be mutually combined. Therefore, the protection scope of this application shall be subject to the protection scope of the claims.

## Claims

1. An organic compound, wherein the organic compound has a structural formula shown in formula (I): 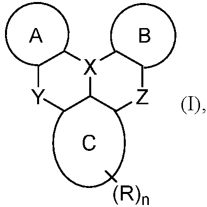 wherein at least one of a ring A, a ring A-Y structure, a ring B, a ring B-Z structure, and R has a structure shown in formula (II): 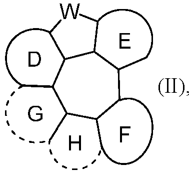 wherein
a ring G and a ring H in the structure shown in formula (II) are absent or at least one of the ring G and the ring H is present;
the ring A, the ring B, a ring C, a ring D, a ring E, a ring F, the ring G, and the ring H are each independently selected from substituted or unsubstituted aryl, substituted or unsubstituted heteroaryl, or a substituted or unsubstituted fused-ring structure;
W is selected from C(R₁, R₂), N-R₃, P, P=O, or Al;
X is selected from B, N, P, P=O, or Al;
Y and Z are each independently selected from C=O, N-R₄, O, S, Se, P, P=O, or P=S, and when Y and Z are both present, at least one of Y and Z is N-R₄;
R and R₁ to R₄ are each independently selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms; and
n is an integer greater than or equal to 0.

2. The organic compound according to claim 1, wherein the ring A, the ring B, the ring C, the ring D, the ring E, the ring F, the ring G, and the ring H are each independently selected from substituted or unsubstituted C6-C30 aryl, substituted or unsubstituted C5-C30 heteroaryl, or a substituted or unsubstituted C10-C30 fused-ring structure.

3. The organic compound according to claim 1 or 2, wherein each time when R₄ is present, R₄ is linked to or fused with the ring A or ring B adjacent thereto to form a ring.

4. The organic compound according to any one of claims 1 to 3, wherein each time when at least two substituents are present in the ring A, the ring B, the ring C, the ring D, the ring E, the ring F, the ring G, and the ring H, at least two adjacent substituents in the substituents are linked or fused together to form a ring.

5. The organic compound according to claim 4, wherein the substituents are each independently selected from one or a combination of the following: aryl, heteroaryl, a fused ring, linear alkyl, branched alkyl, alicyclic hydrocarbyl, alkoxy, allyl, cyano, halogen, hydrogen, and deuterium.

6. The organic compound according to claim 5, wherein the substituents are each independently selected from one or a combination of the following: C6-C30 aryl, C5-C30 heteroaryl, a C10-C30 fused ring, C1-C8 linear alkyl, C1-C8 branched alkyl, C3-C10 alicyclic hydrocarbyl, C1-C8 alkoxy, allyl, cyano, halogen, hydrogen, and deuterium.

7. The organic compound according to any one of claims 1 to 6, wherein formula (II) has a structure shown in formula (III), formula (IV), or formula (V):

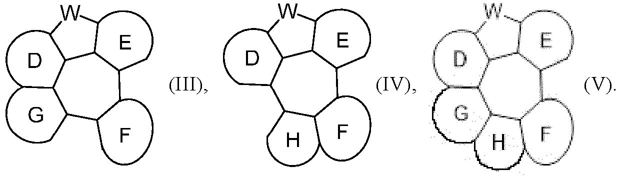

8. The organic compound according to claim 7, wherein the organic compound has a structure shown in formula (VI), formula (VII), formula (VIII), formula (IX), formula (X), formula (XI), formula (XII), formula (XIII), or formula (XIV): 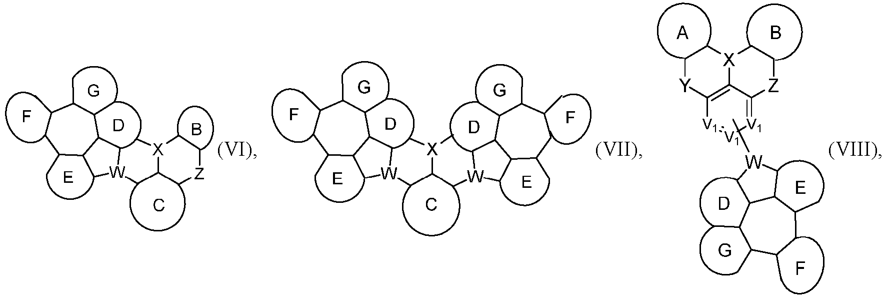 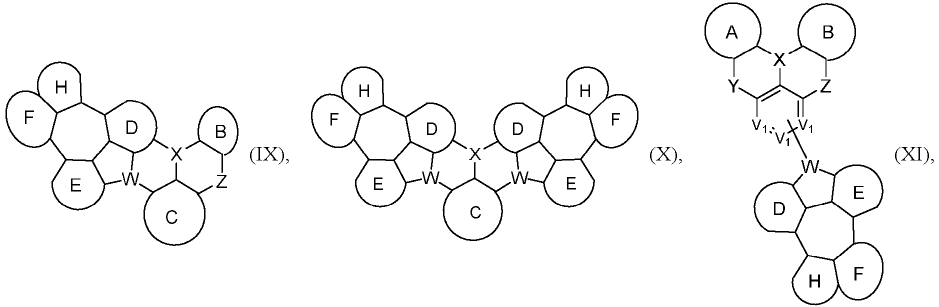 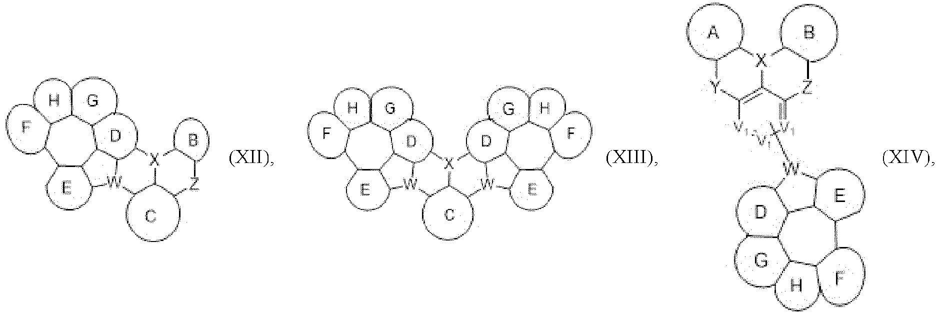 wherein
V₁ is selected from C-R₅ or N-R₆; and
R₅ and R₆ are each independently selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms.

9. The organic compound according to claim 8, wherein the ring A, the ring B, the ring C, the ring D, the ring E, the ring F, the ring G, and the ring H are each independently selected from one or a combination of the following structures: 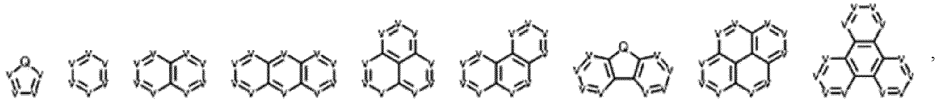 wherein
V is selected from C-R₇ or N-R₈;
Q is selected from B-R₉, C(=O), N-R₁₀, O, S, P, P=O, or P=S; and
R₇ to R₁₀ are each independently selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms.

10. The organic compound according to claim 9, wherein a compound shown in formula (III) is 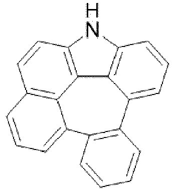 ; a compound shown in formula (IV) is 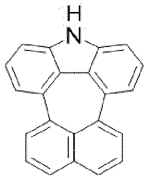 ; and a compound shown in formula (V)

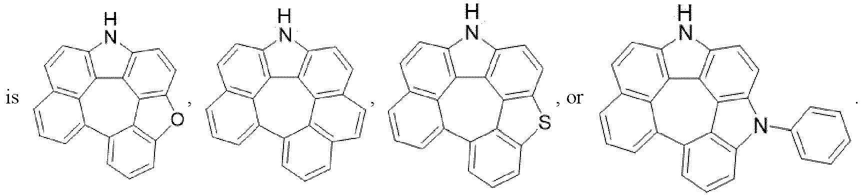

11. The organic compound according to claim 10, wherein the organic compound is any one of the following compounds:

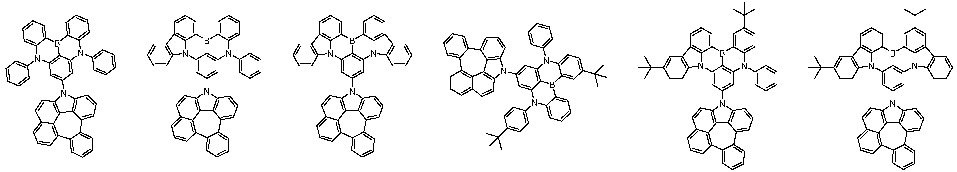

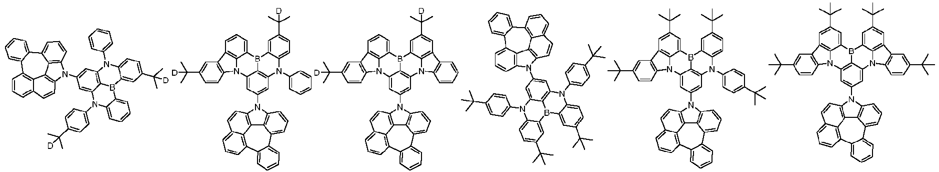

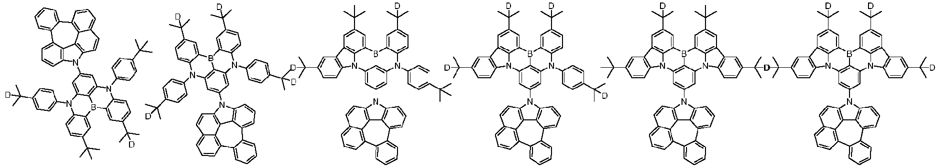

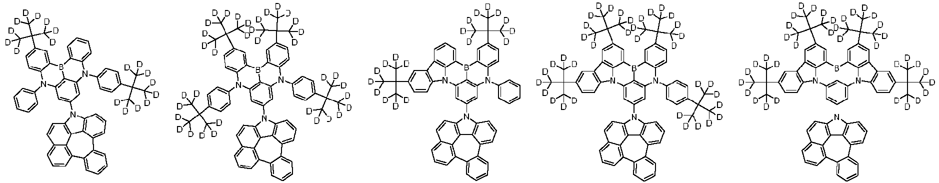

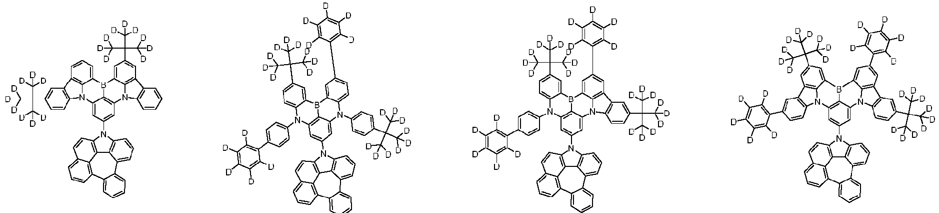

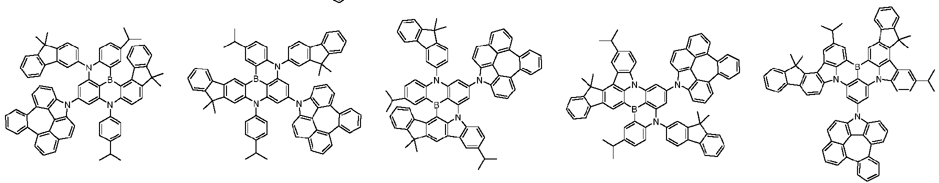

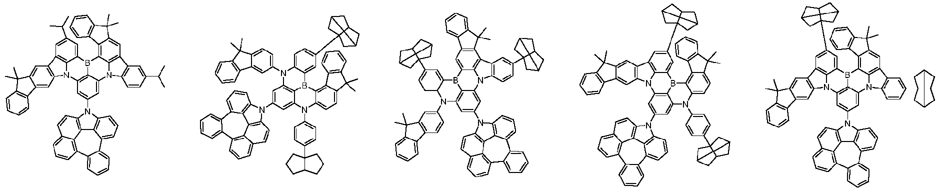

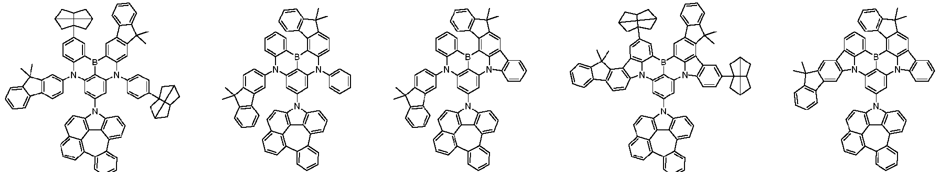

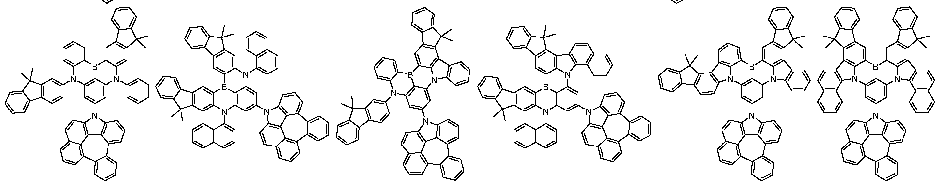

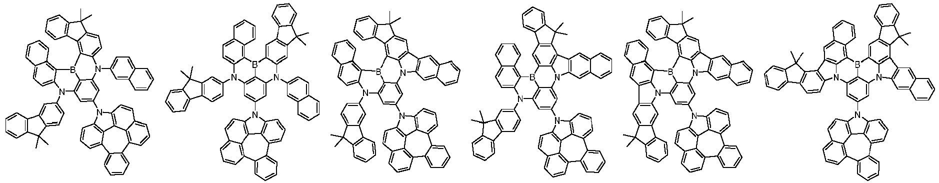

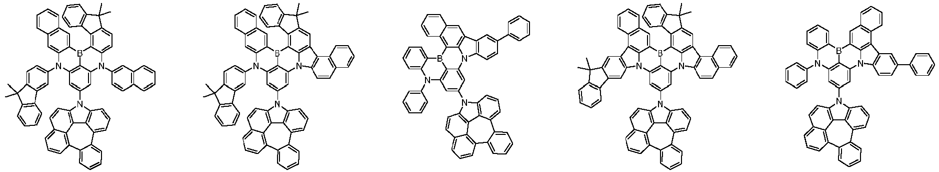

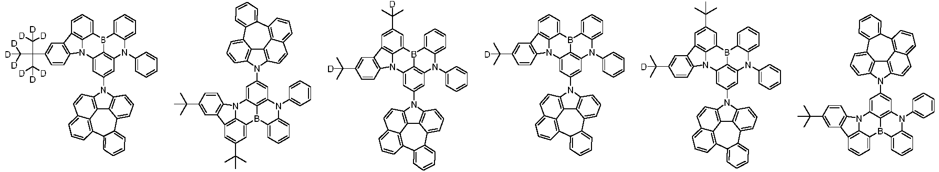

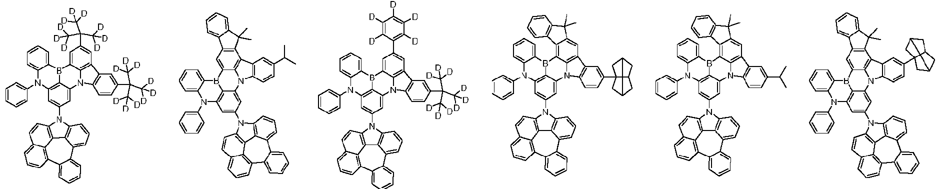

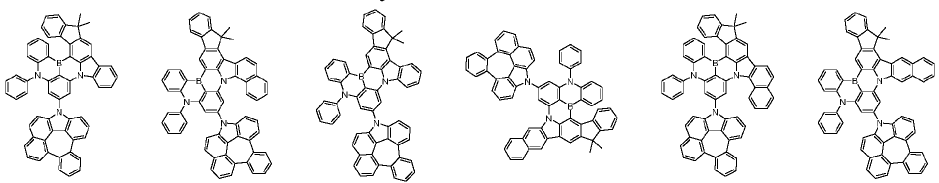

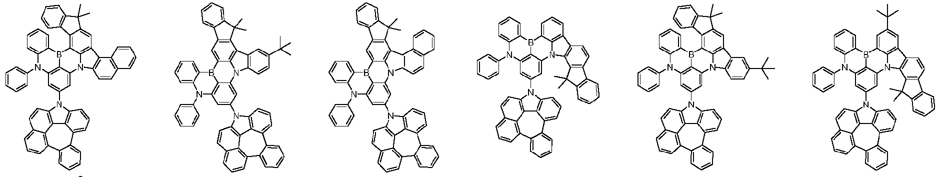

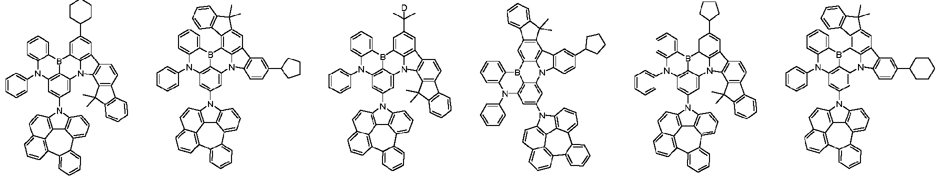

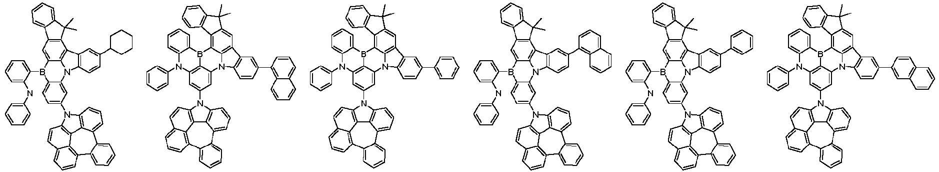

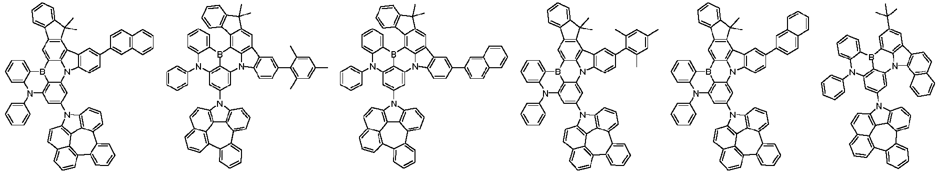

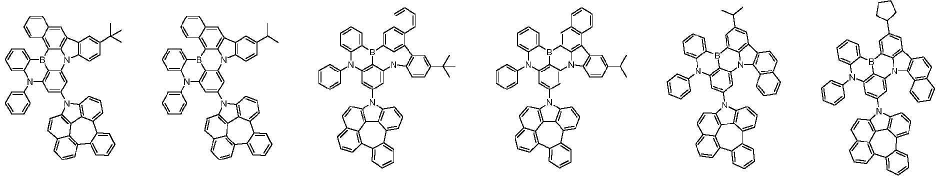

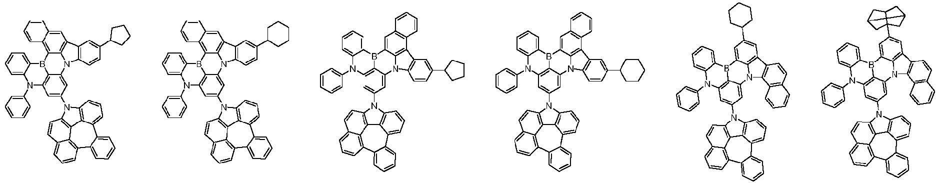

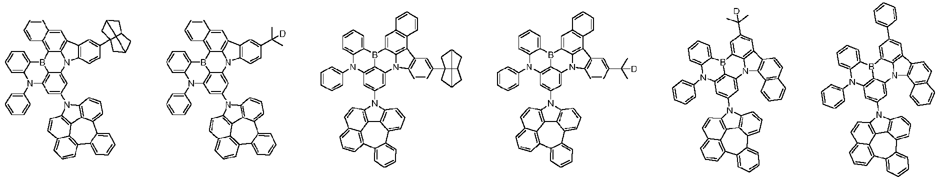

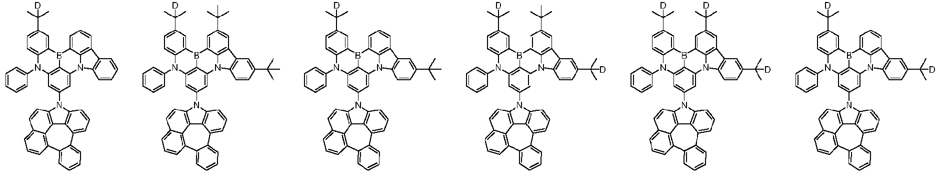

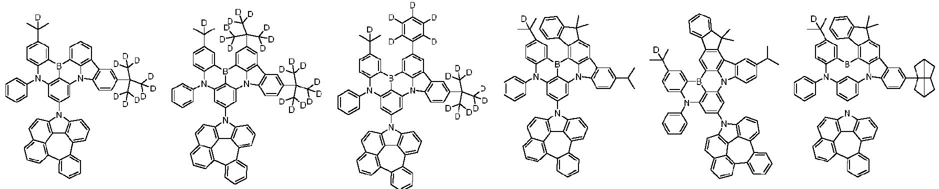

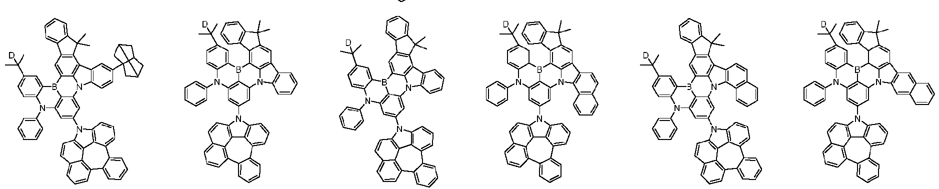

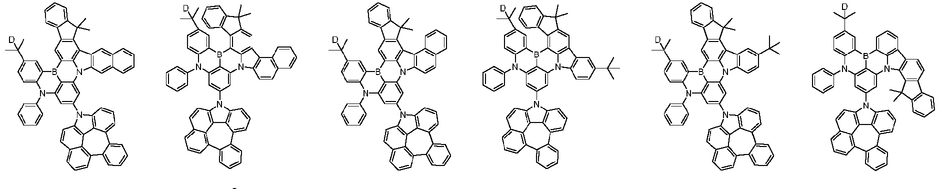

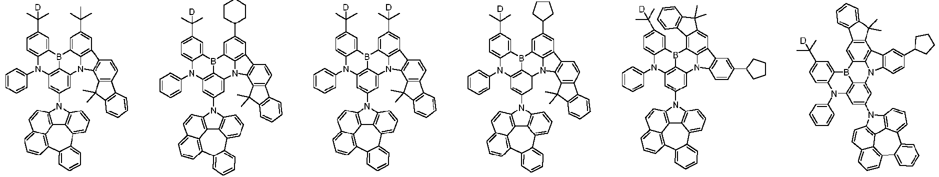

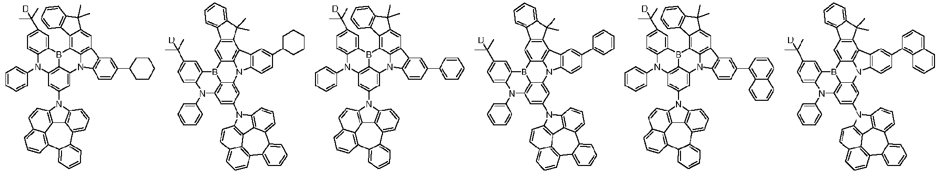

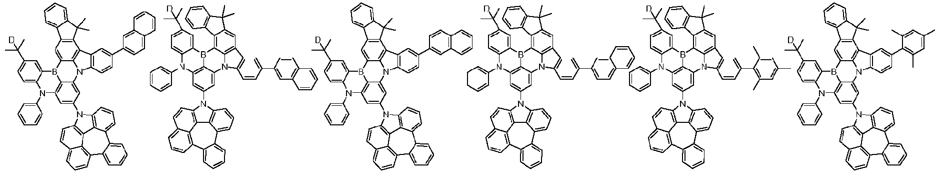

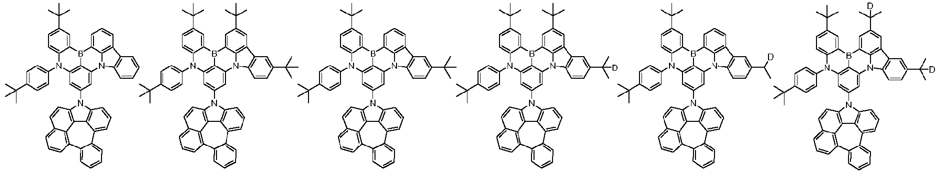

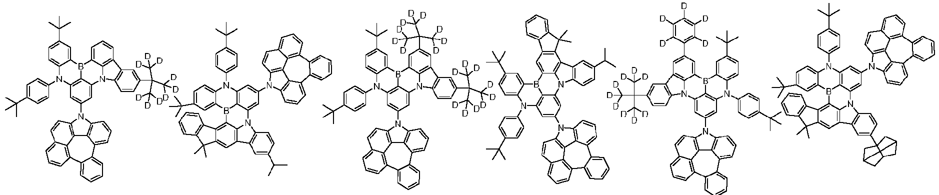

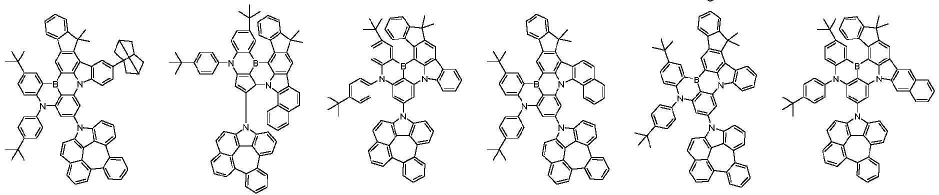

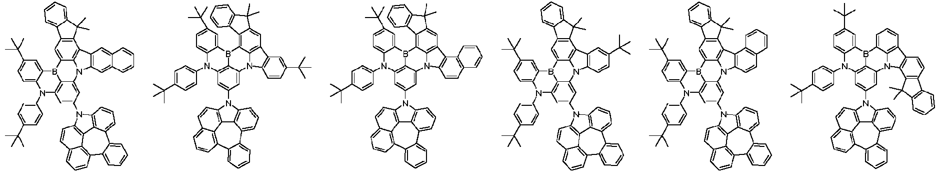

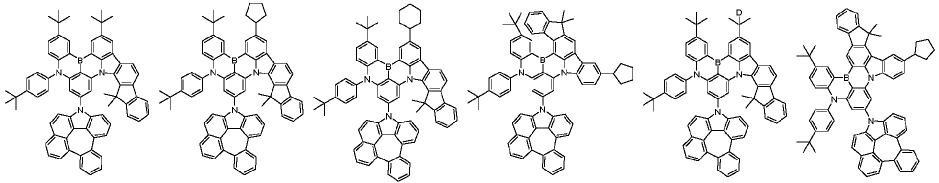

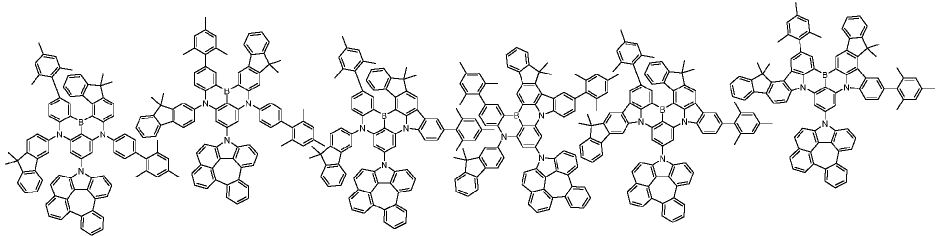

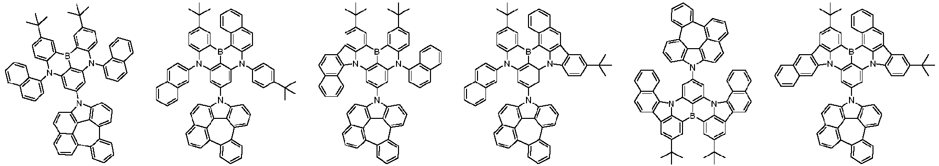

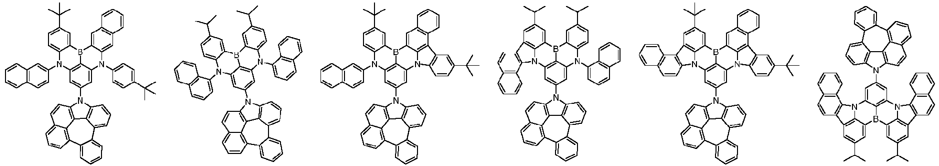

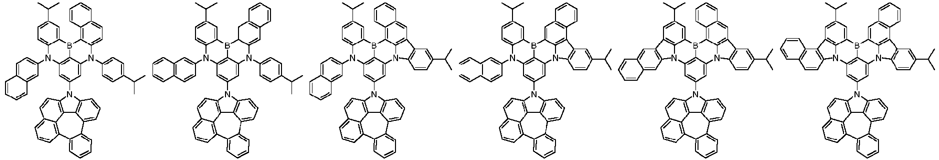

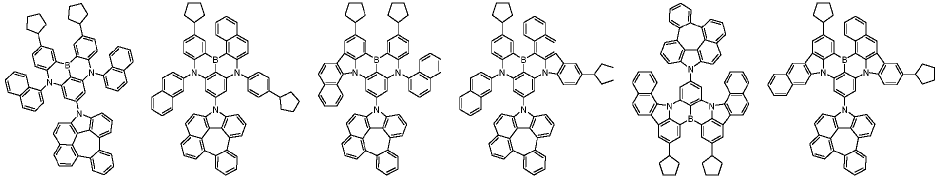

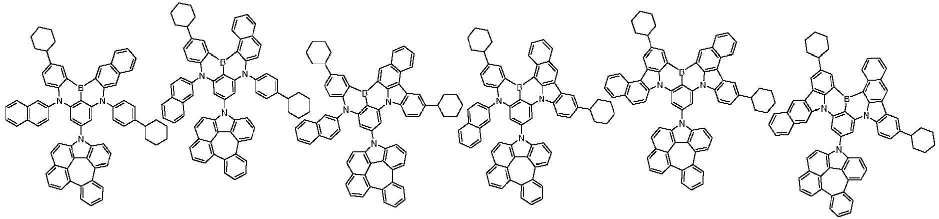

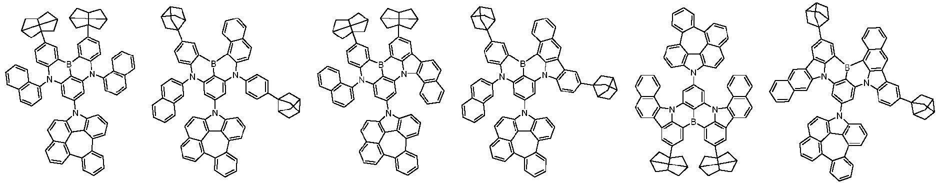

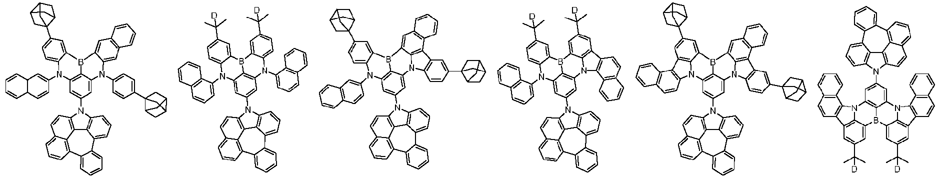

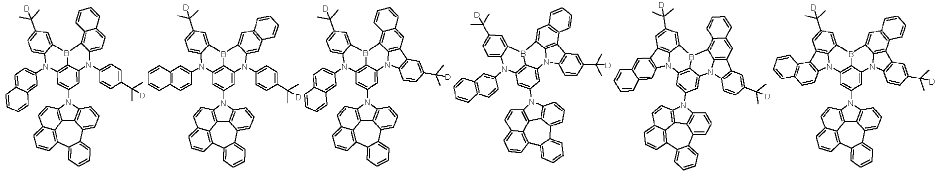

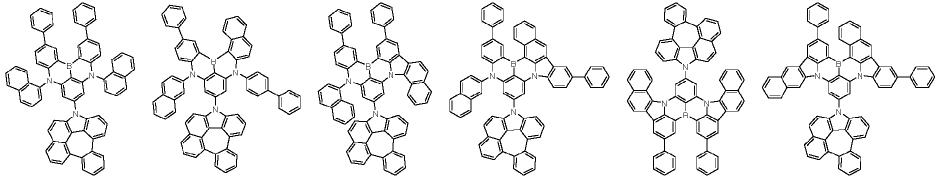

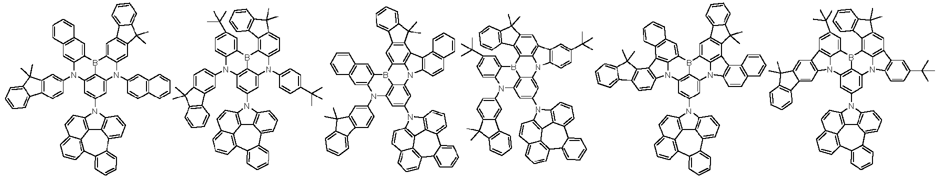

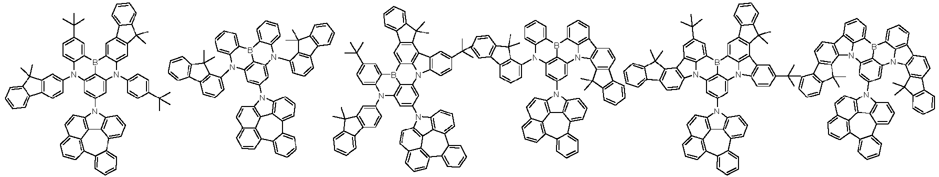

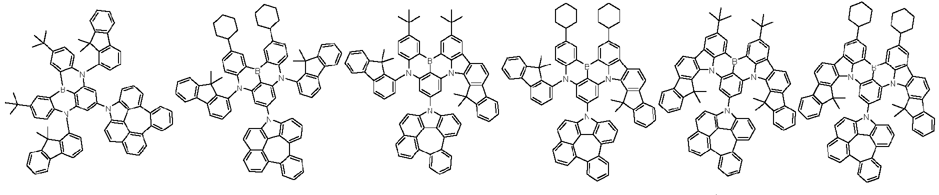

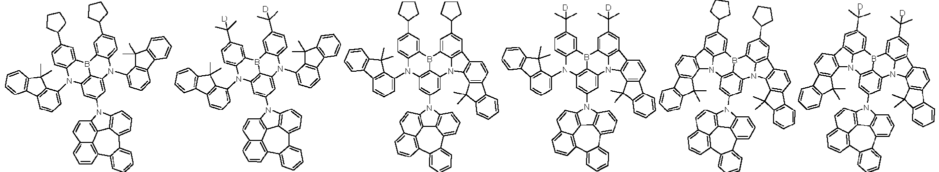

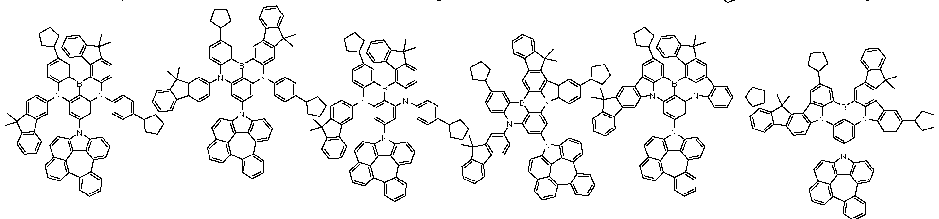

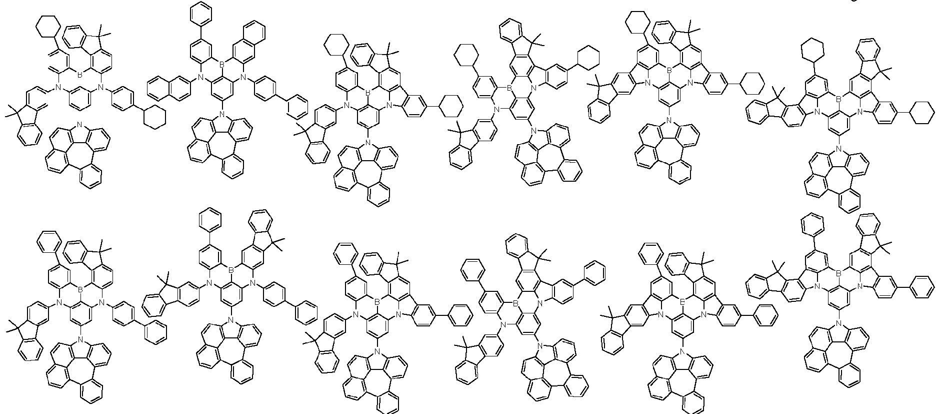

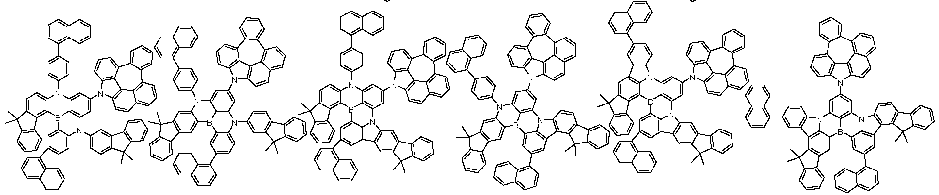

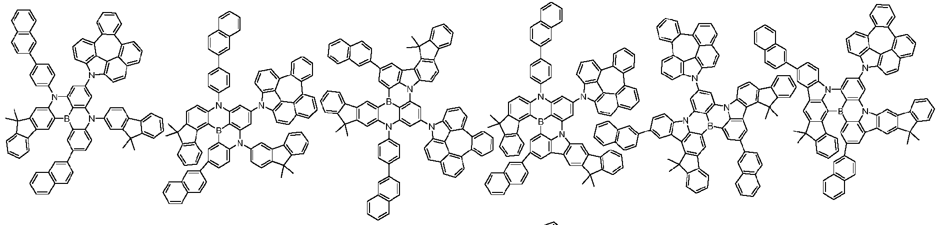

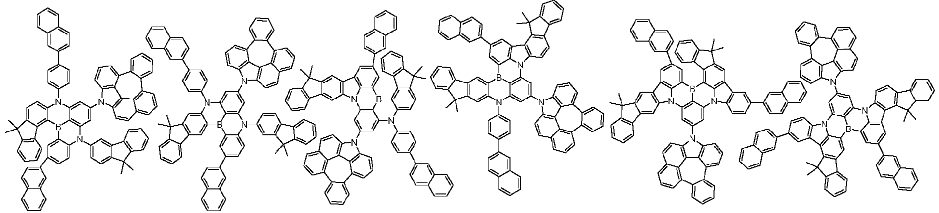

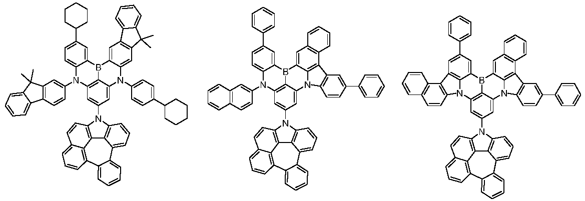

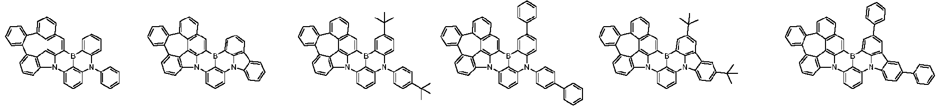

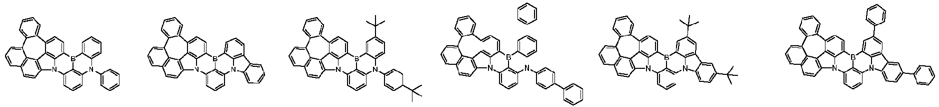

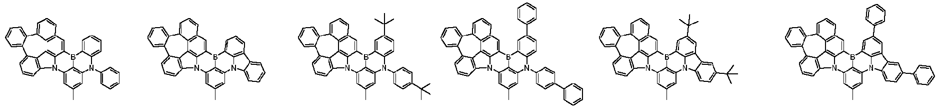

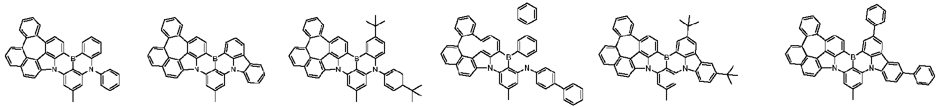

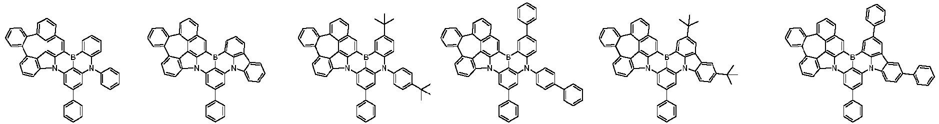

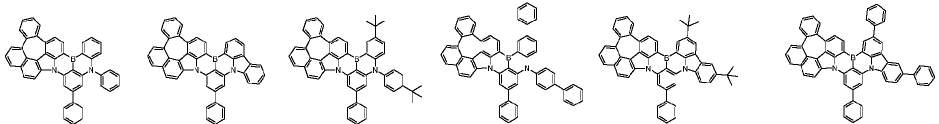

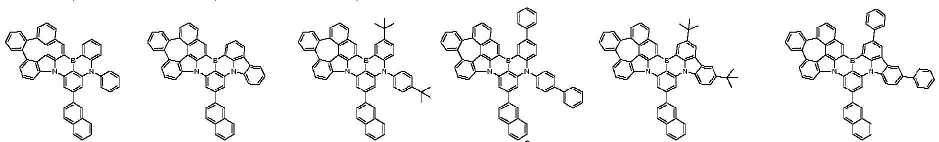

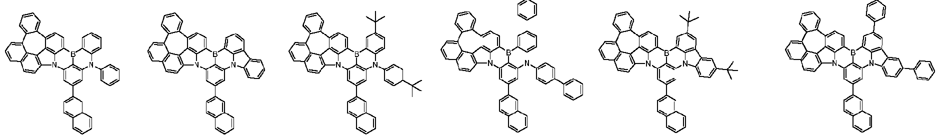

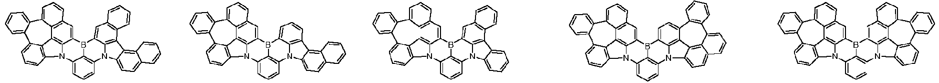

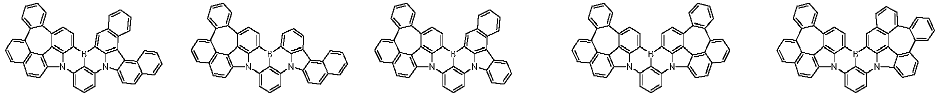

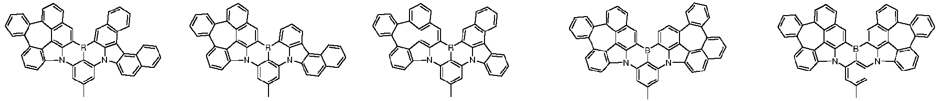

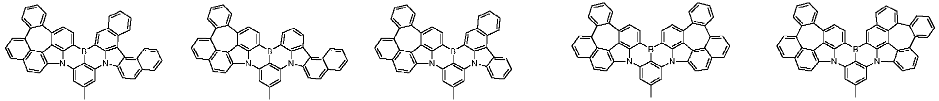

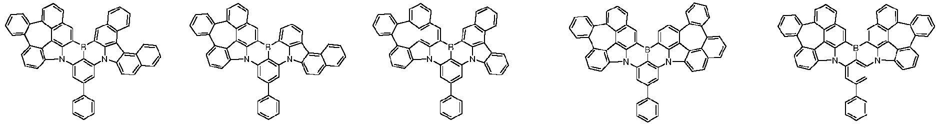

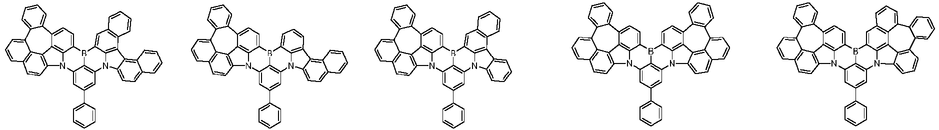

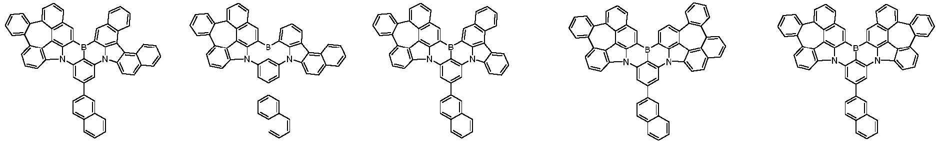

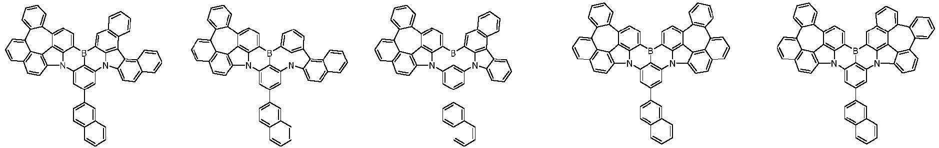

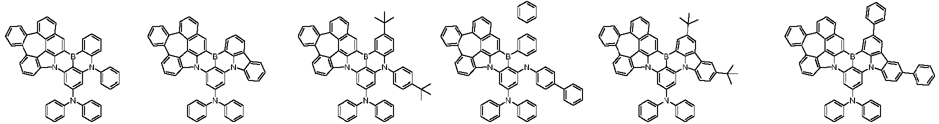

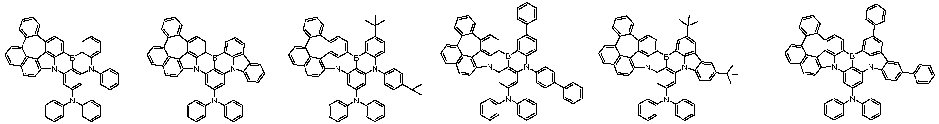

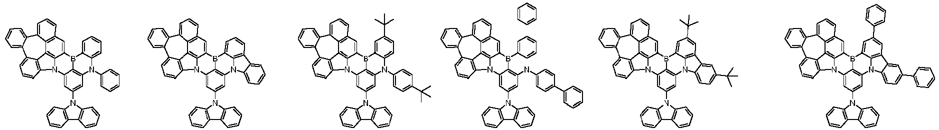

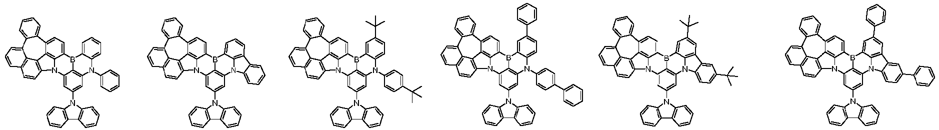

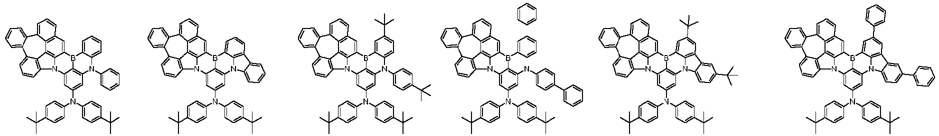

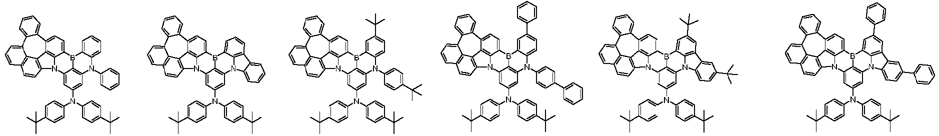

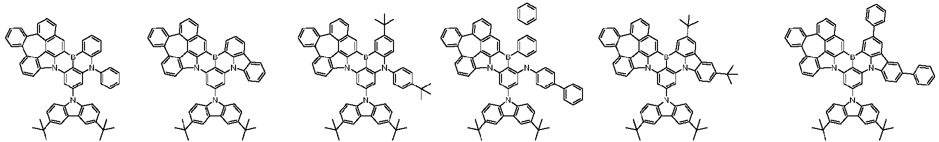

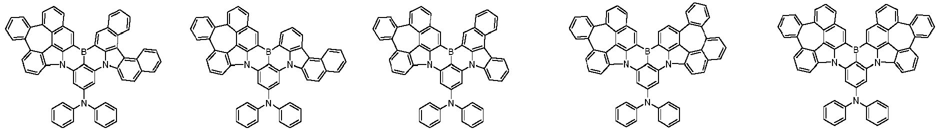

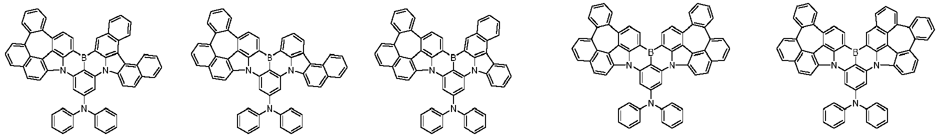

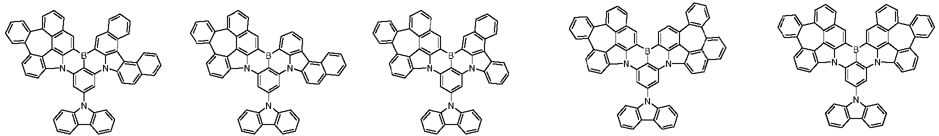

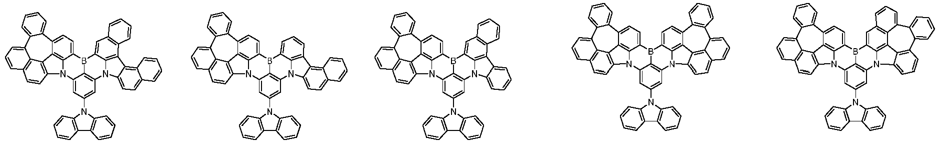

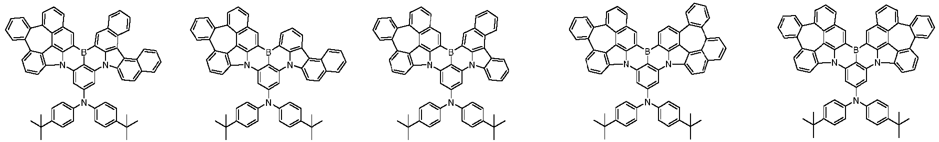

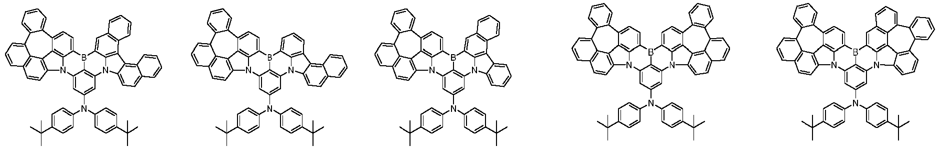

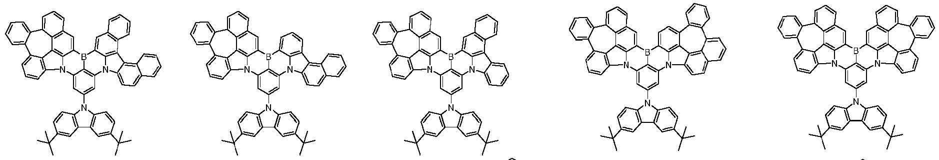

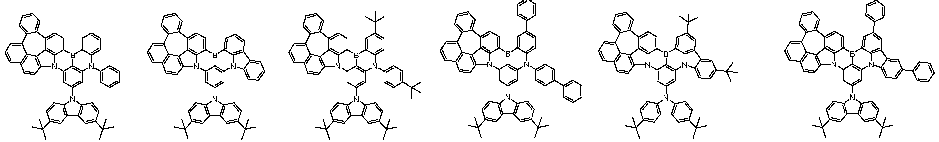

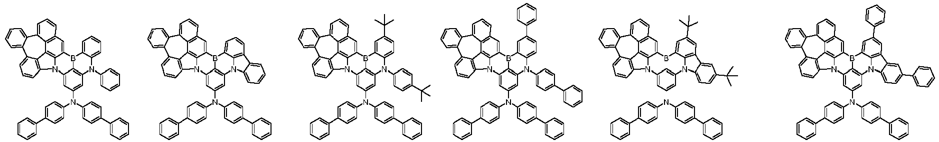

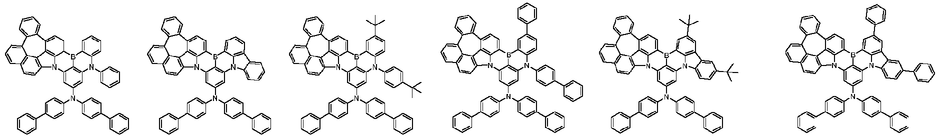

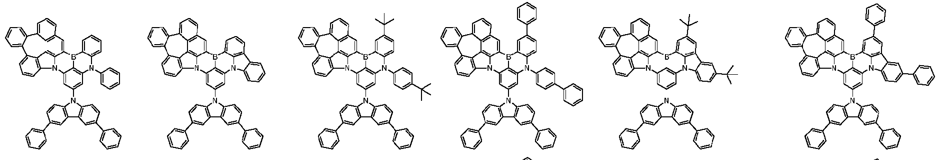

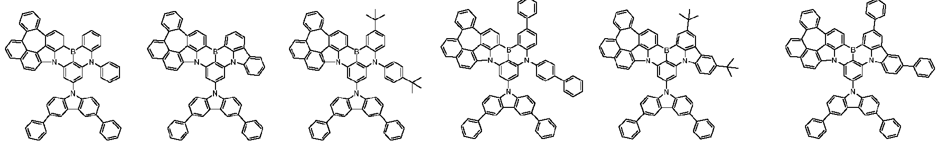

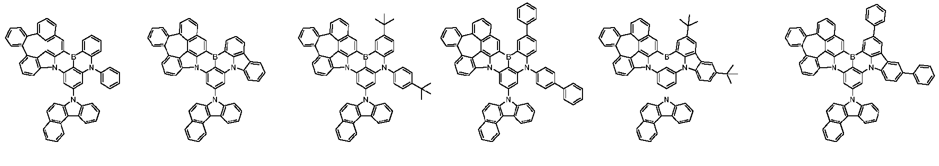

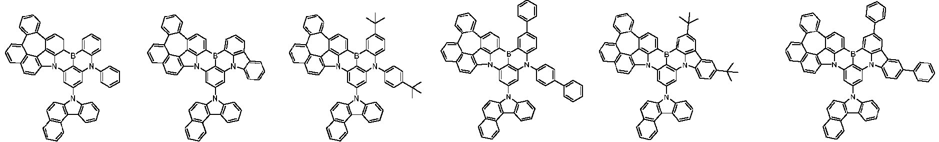

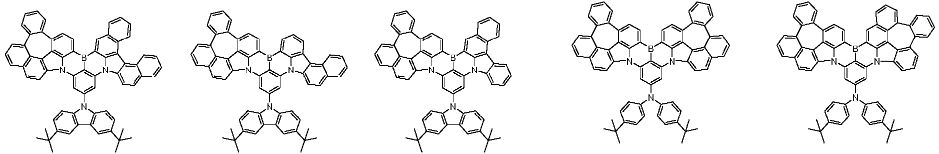

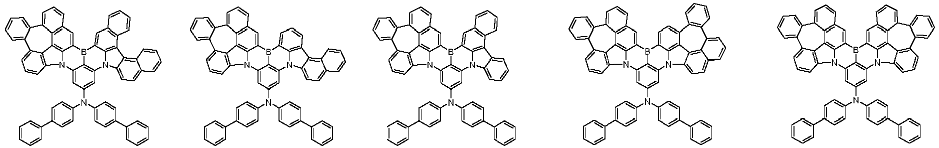

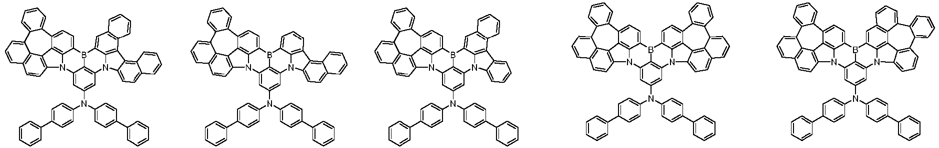

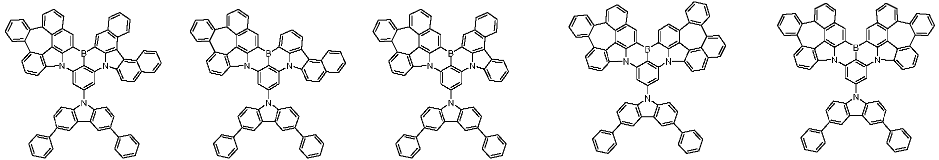

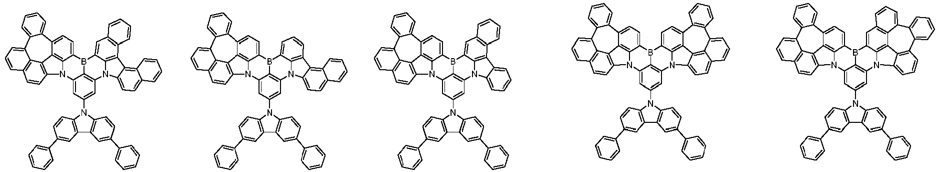

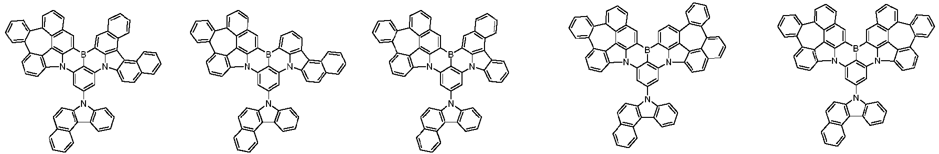

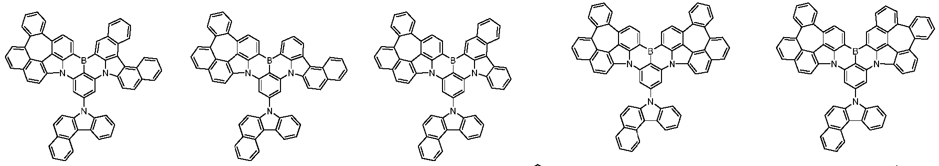

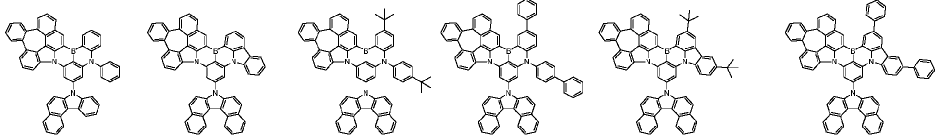

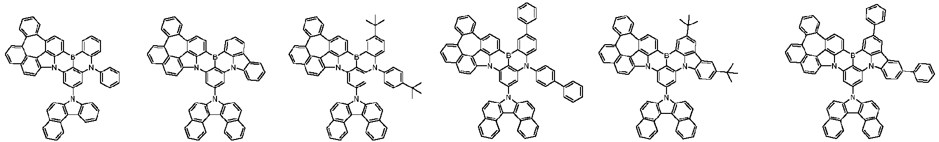

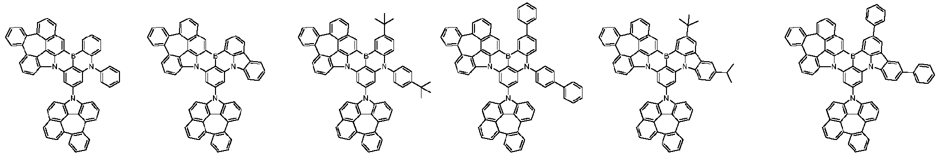

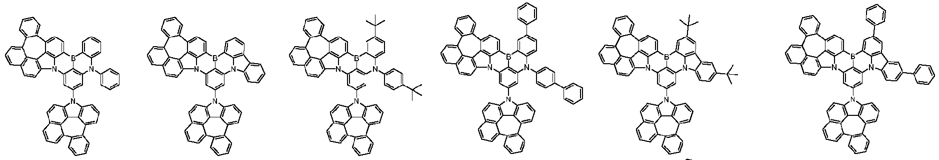

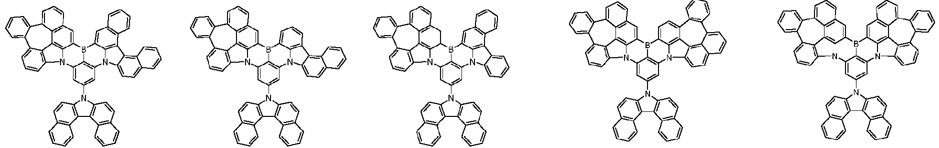

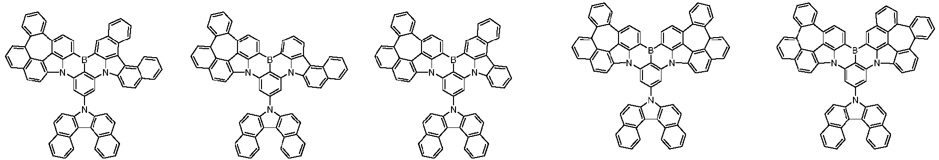

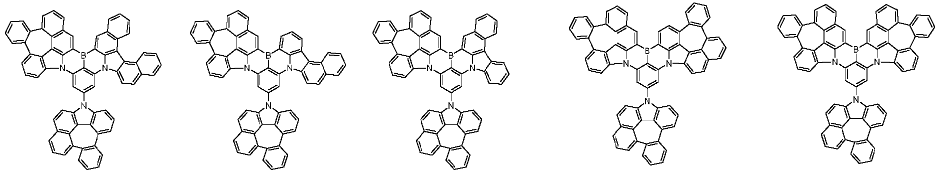

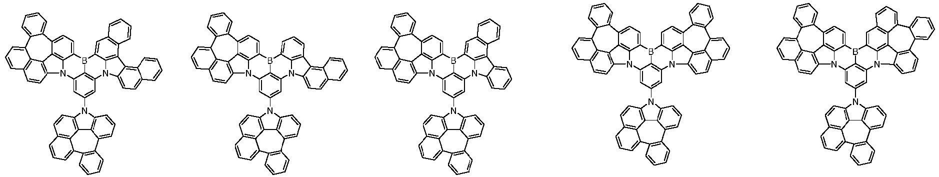

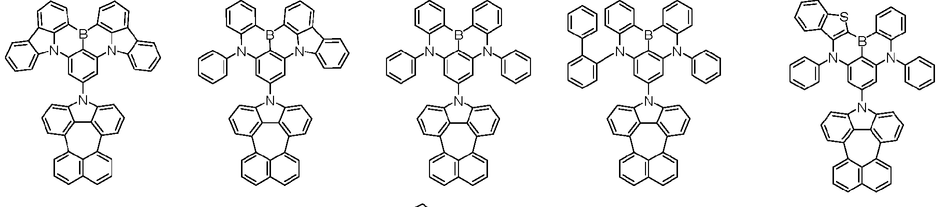

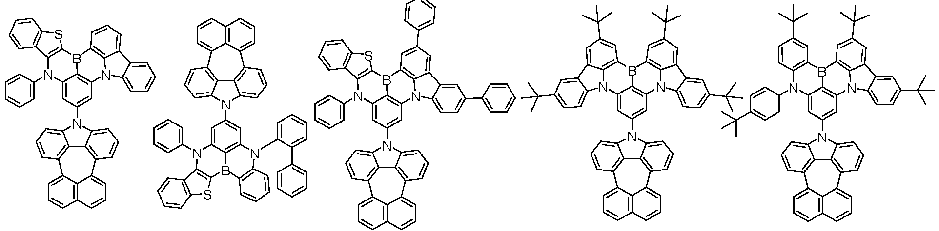

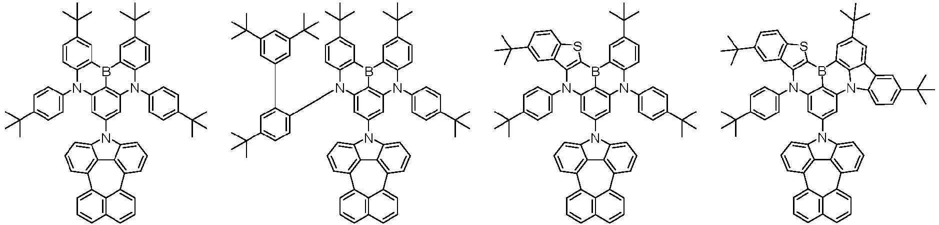

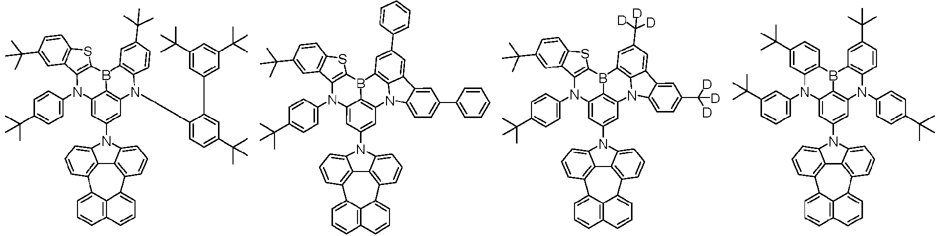

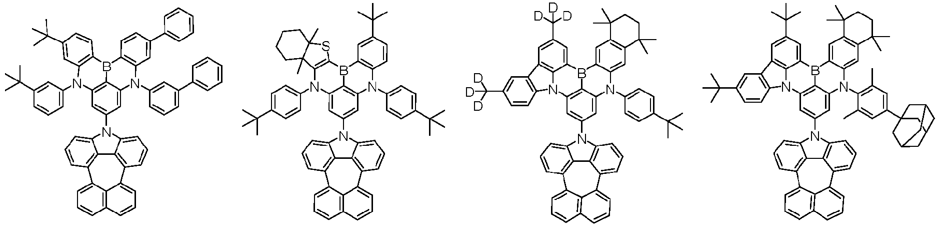

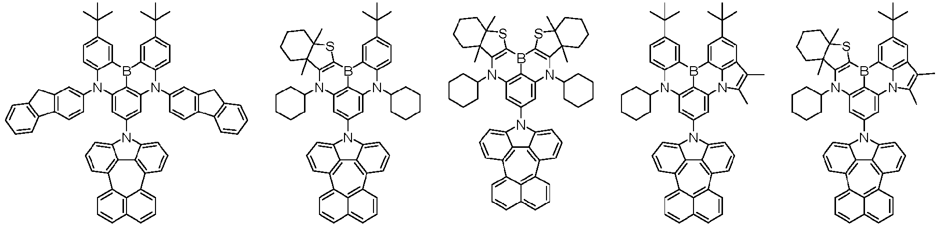

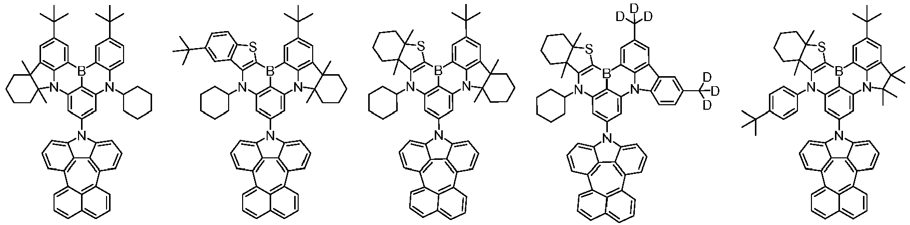

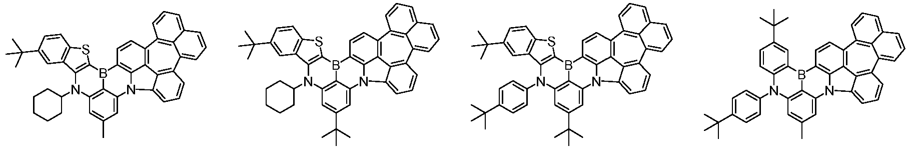

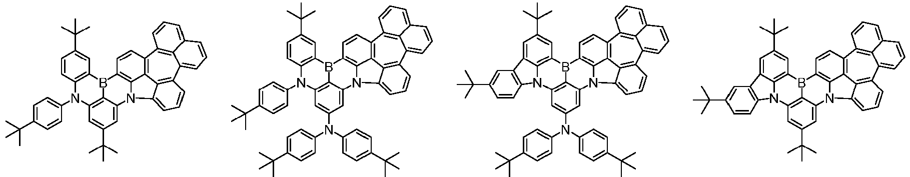

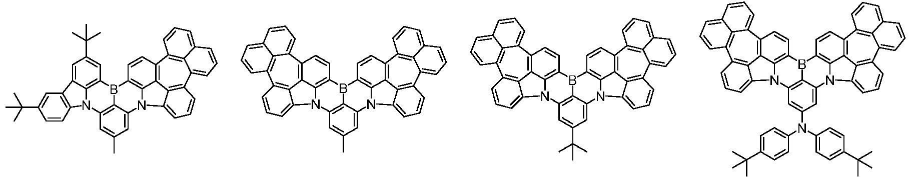

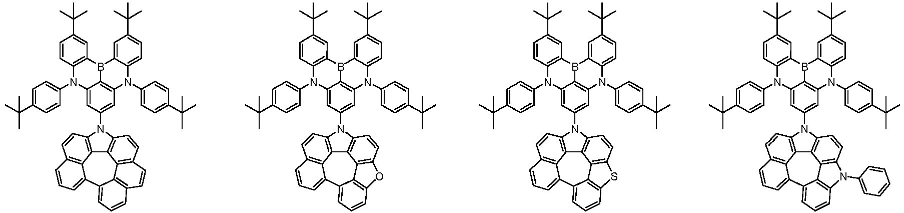

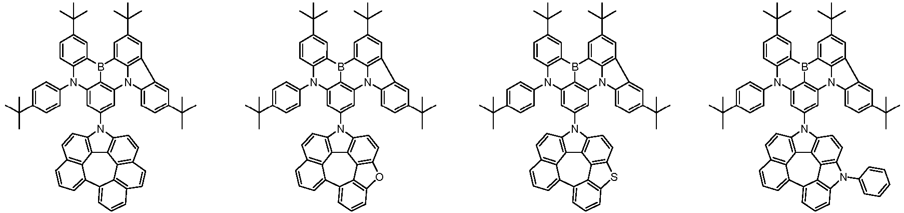

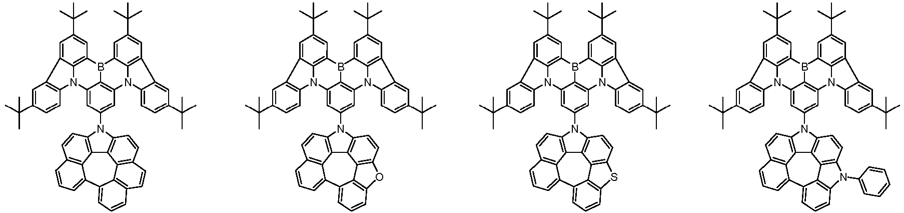

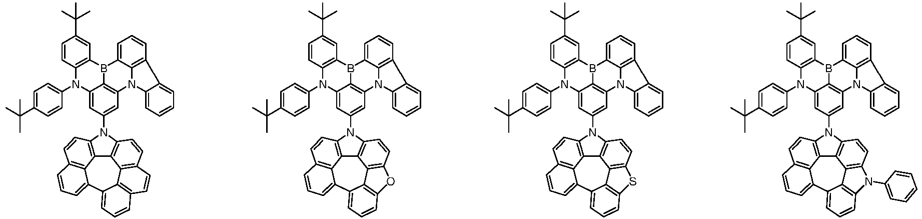

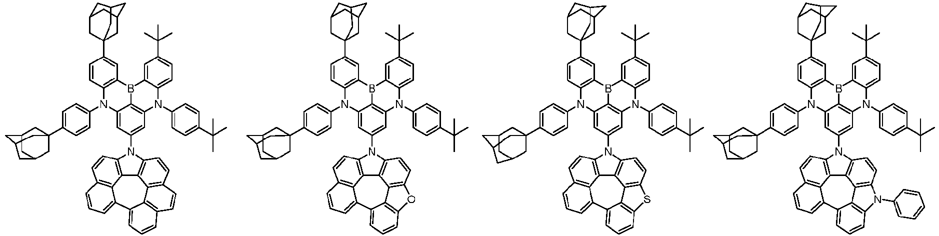

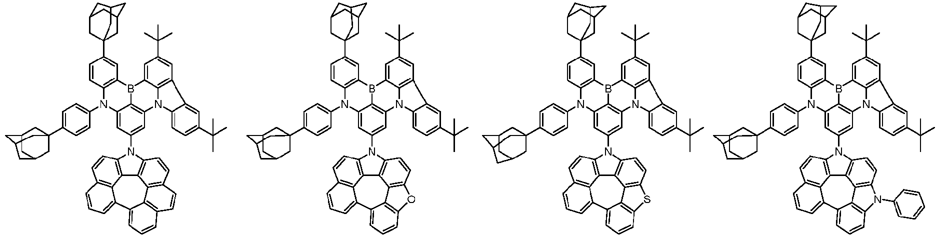

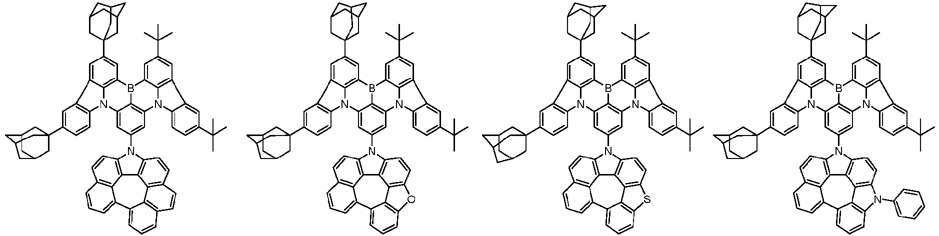

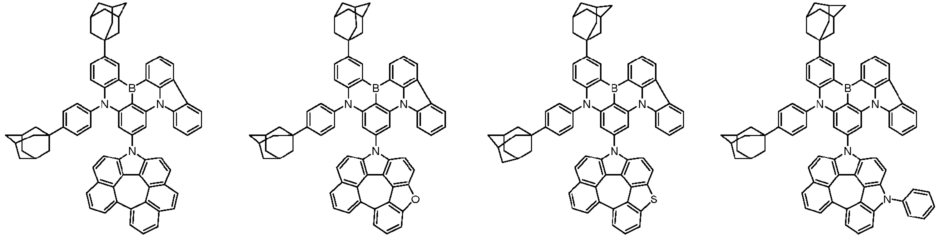

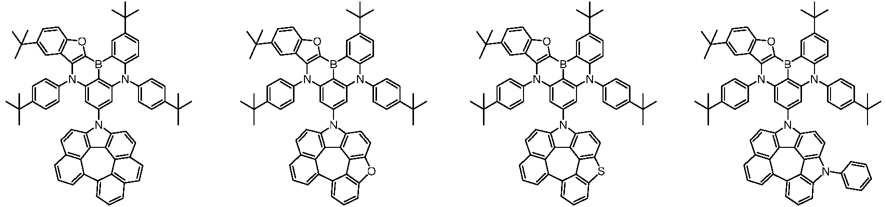

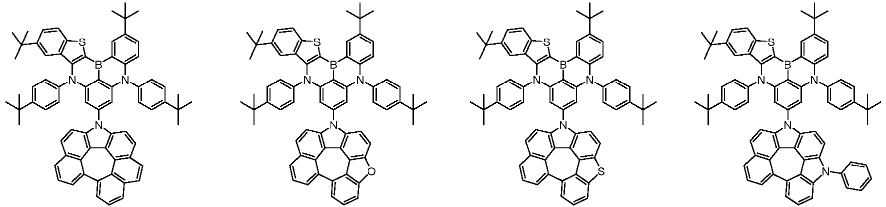

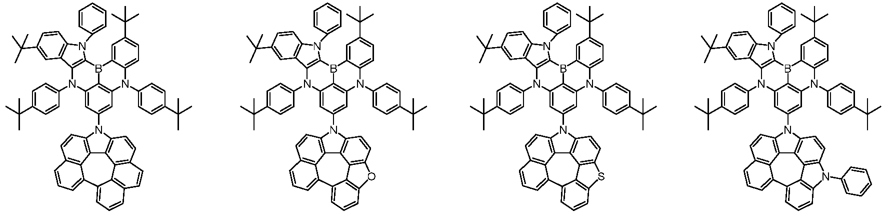

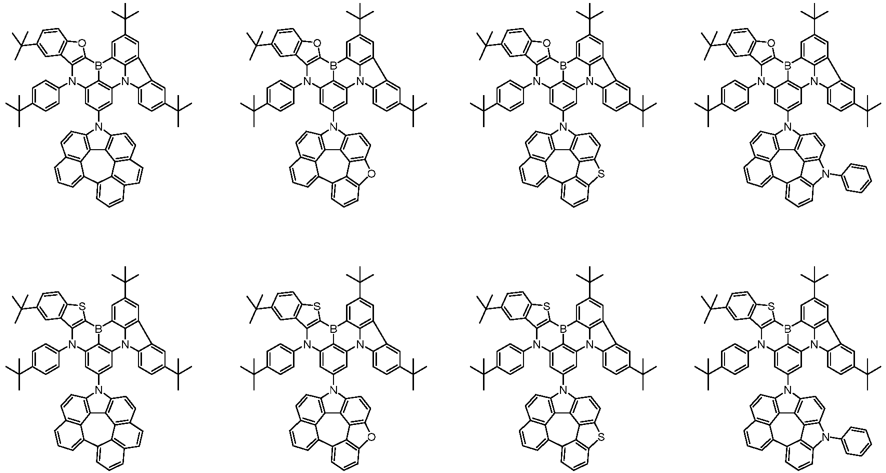

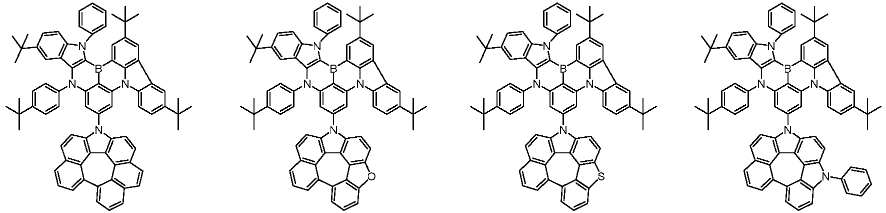

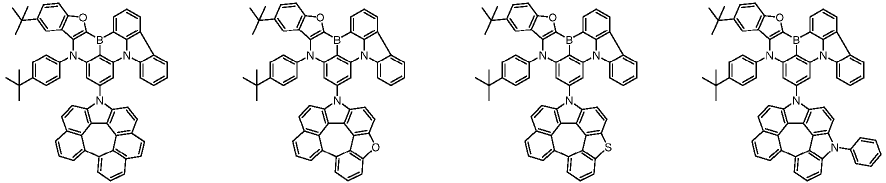

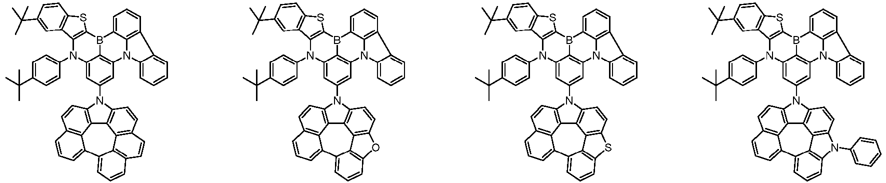

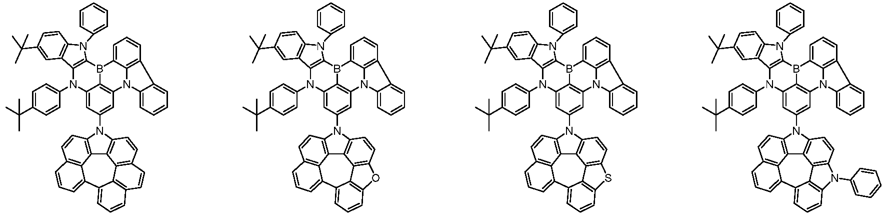

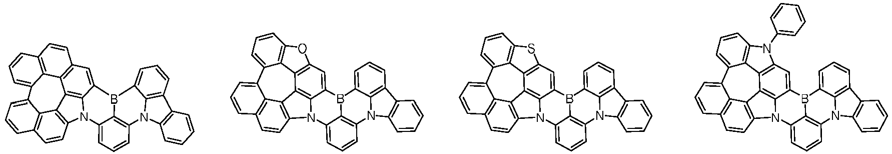

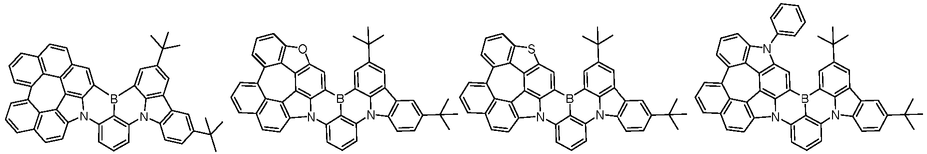

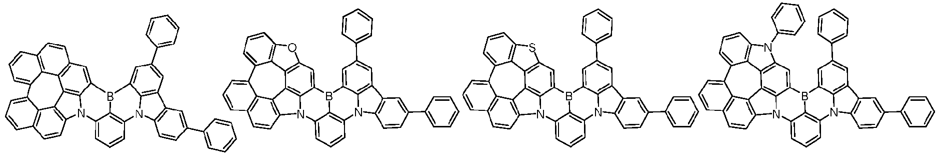

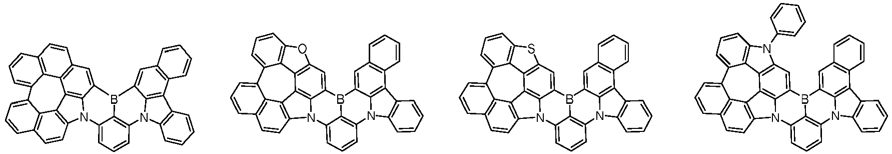

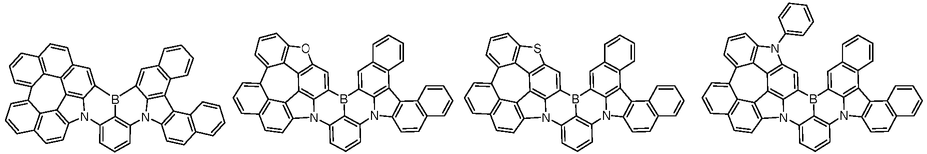

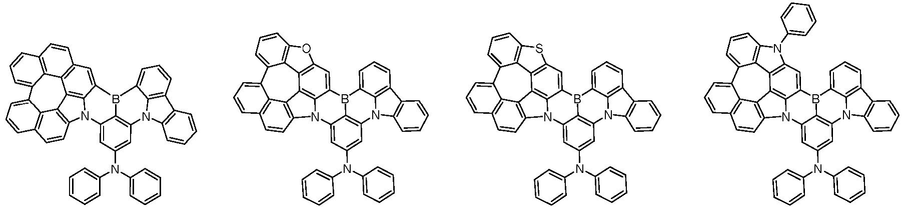

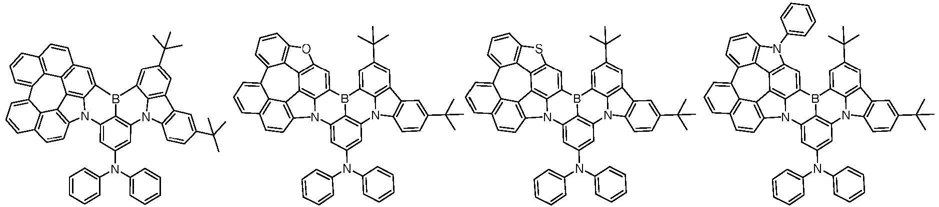

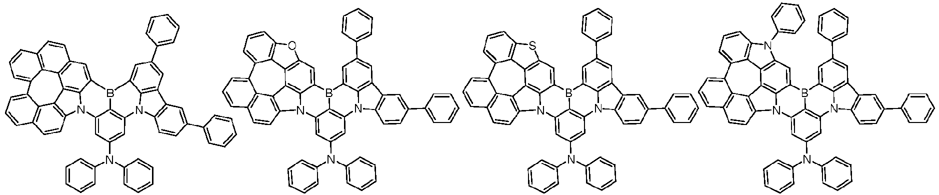

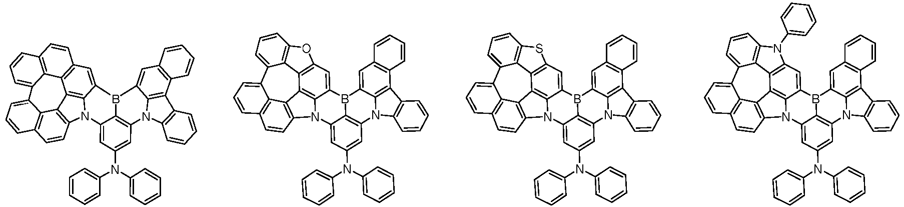

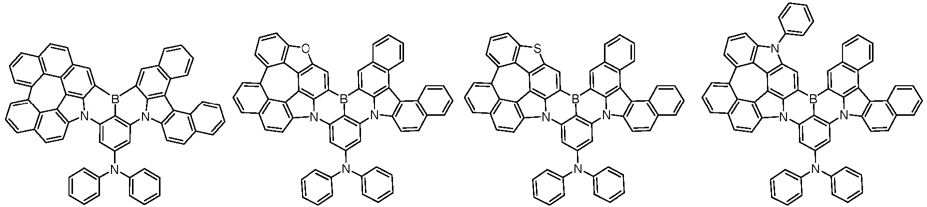

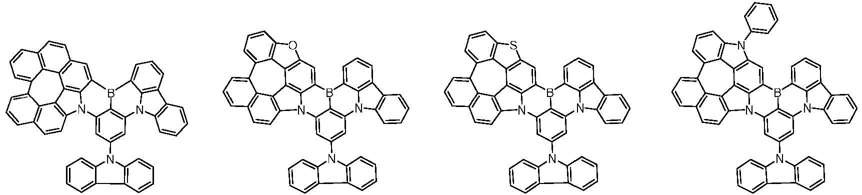

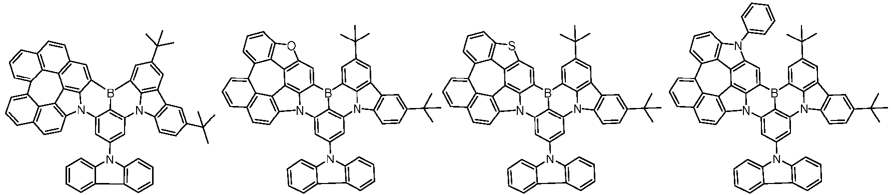

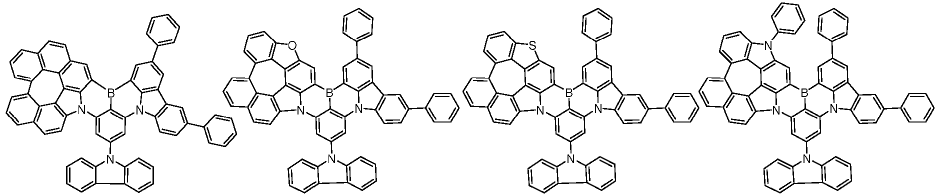

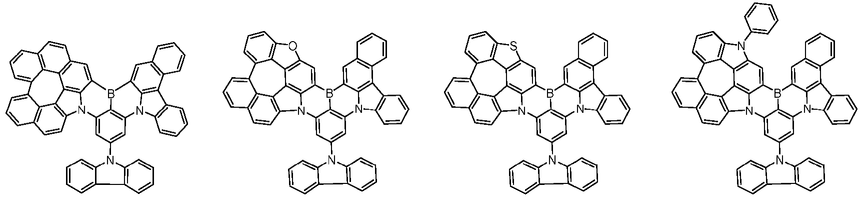

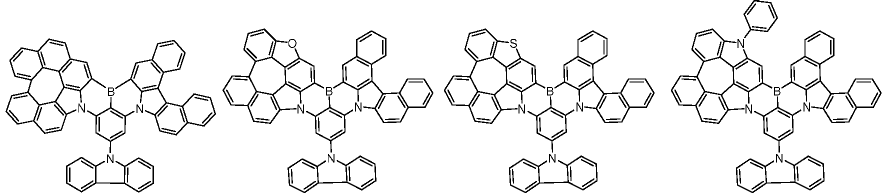

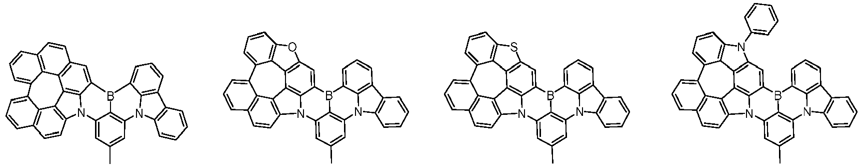

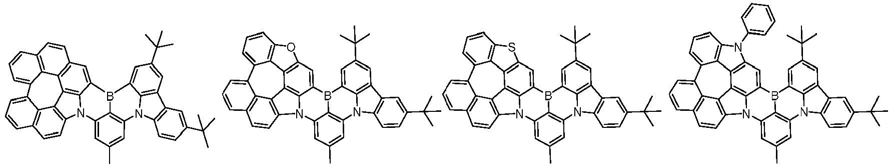

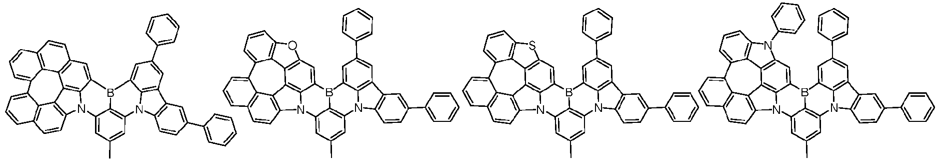

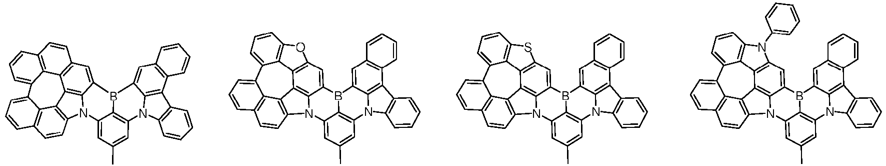

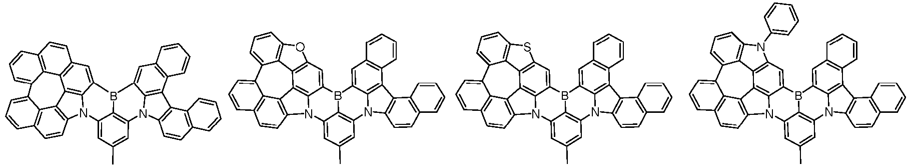

12. A method for preparing an organic compound, comprising the following steps: substituting X₂ or X₃ in a compound 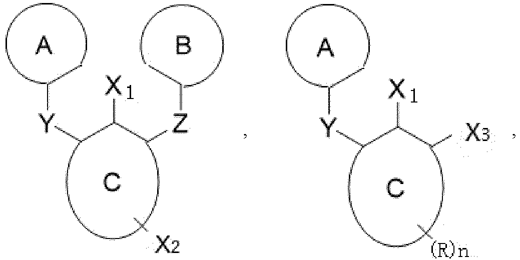 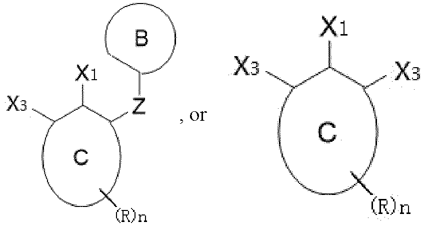 with a compound 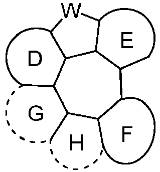 to obtain an intermediate 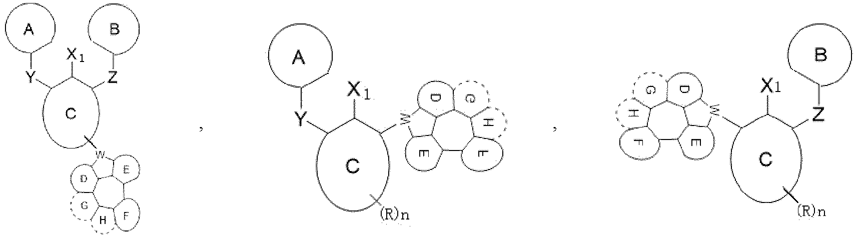 , or 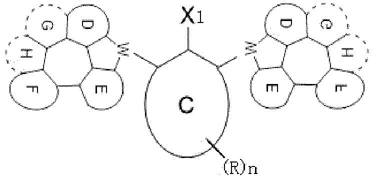 , wherein X₁, X₂, and X₃ are each independently selected from a halogen atom;
a ring A, a ring B, a ring C, a ring D, a ring E, a ring F, a ring G, and a ring H are each independently selected from substituted or unsubstituted aryl, substituted or unsubstituted heteroaryl, or a substituted or unsubstituted fused-ring structure;
W is selected from C(R₁, R₂), N-R₃, P, P=O, or Al;
Y and Z are each independently selected from C=O, N-R₄, O, S, Se, P, P=O, or P=S, and when Y and Z are both present, at least one of Y and Z is N-R₄;
R and R₁ to R₄ are each independently selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms; and
n is an integer greater than or equal to 0; and
reacting the intermediate 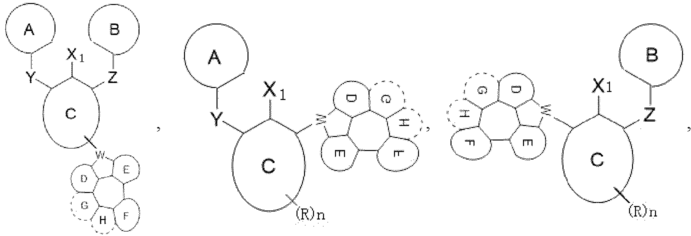 or 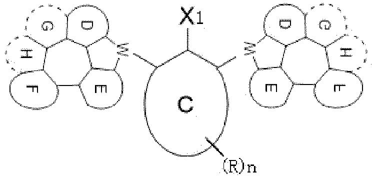 with an X-containing compound to obtain the organic compound 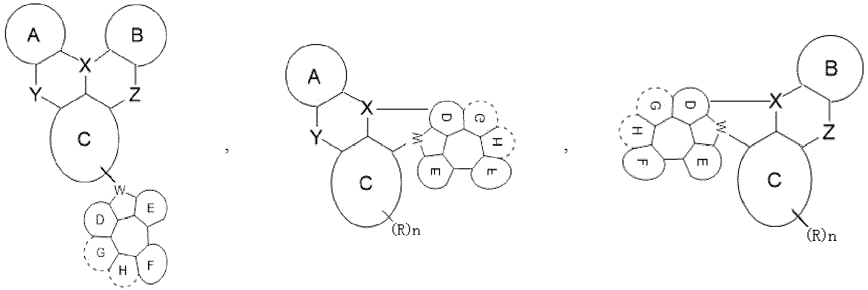 , or 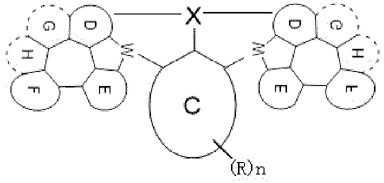 , wherein X is selected from B, N, P, P=O, or Al.

13. A method for preparing an organic compound, comprising the following steps: substituting X₂ and X₃ in a compound 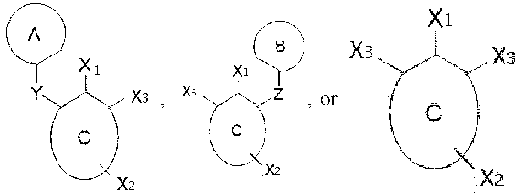 with a compound 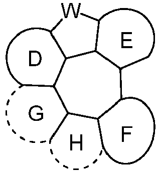 to obtain an intermediate 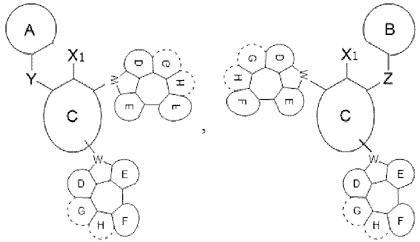 , or 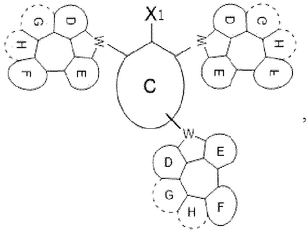 wherein X₁, X₂, and X₃ are each independently selected from a halogen atom;
a ring A, a ring B, a ring C, a ring D, a ring E, a ring F, a ring G, and a ring H are each independently selected from substituted or unsubstituted aryl, substituted or unsubstituted heteroaryl, or a substituted or unsubstituted fused-ring structure;
W is selected from C(R₁, R₂), N-R₃, P, P=O, or Al;
Y and Z are each independently selected from C=O, N-R₄, O, S, Se, P, P=O, or P=S, and when Y and Z are both present, at least one of Y and Z is N-R₄; and
R and R₁ to R₄ are each independently selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms; and reacting the intermediate 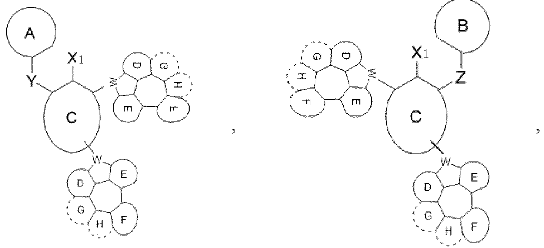 or 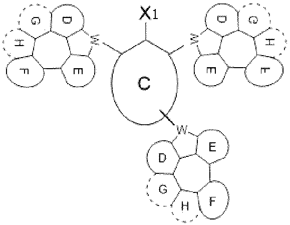 with an X-containing compound to obtain the organic compound 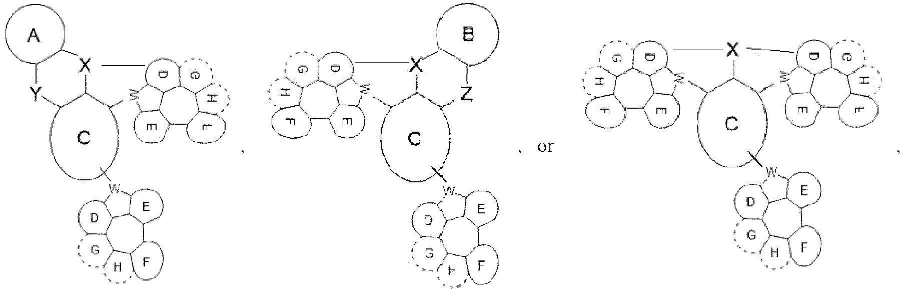 wherein X is selected from B, N, P, P=O, or Al.

14. A polymer, wherein the polymer comprises at least one first repeating unit, and the first repeating unit is derived from at least one organic compound according to any one of claims 1 to 11 or the organic compound prepared by using the method for preparing the organic compound according to claim 12 or 13.

15. The polymer according to claim 14, wherein the polymer further comprises at least one second repeating unit different from the first repeating unit.

16. The polymer according to claim 15, wherein the second repeating unit is selected from at least one of the following repeating units: 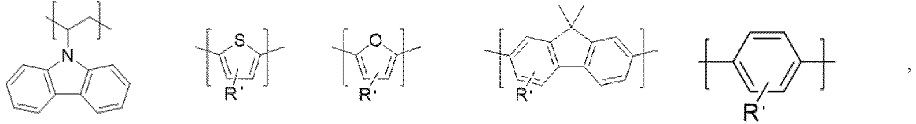 wherein
R' is selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms.

17. A polymer, wherein the polymer comprises a polymer molecule main chain and a branched chain linked to the polymer molecule main chain, and the branched chain is derived from at least one organic compound according to any one of claims 1 to 11 or the organic compound prepared by using the method for preparing the organic compound according to claim 12 or 13.

18. The polymer according to claim 17, wherein the polymer comprises at least one of the following repeating units: 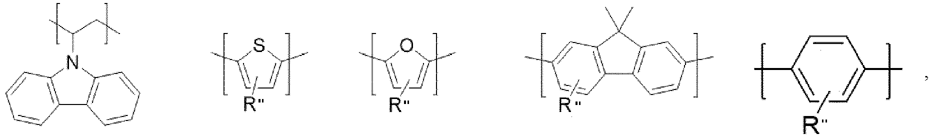 wherein
R" is selected from one or a combination of the following: H, D, C1-C20 linear alkyl, C1-C20 alkoxy, C1-C20 thioalkoxy, C3-C20 branched alkyl, C3-C20 cycloalkyl, C3-C20 branched alkoxy, C3-C20 cycloalkoxy, C3-C20 branched thioalkoxy, C3-C20 cyclic thioalkoxy, methylsilyl, C1-C20 keto, C2-C20 alkoxycarbonyl, C7-C20 aryloxycarbonyl, cyano, carbamoyl, haloformyl, formyl, isocyano, isocyanate, thiocyanate, isothiocyanate, hydroxyl, nitro, CF₃, Cl, Br, F, I, a crosslinkable group, substituted aryl containing 5 to 60 ring atoms, unsubstituted aryl containing 5 to 60 ring atoms, substituted heteroaryl containing 5 to 60 ring atoms, unsubstituted heteroaryl containing 5 to 60 ring atoms, aryloxy containing 5 to 60 ring atoms, and heteroaryloxy containing 5 to 60 ring atoms.

19. A composition, comprising an organic solvent and at least one organic compound according to any one of claims 1 to 11, or the organic compound prepared by using the method for preparing the organic compound according to claim 12 or 13, or at least one polymer according to any one of claims 14 to 18.

20. A mixture, comprising an organic functional material and at least one organic compound according to any one of claims 1 to 11, or the organic compound prepared by using the method for preparing the organic compound according to claim 12 or 13, or at least one polymer according to any one of claims 14 to 18, or at least one composition according to claim 19, wherein the organic functional material is selected from at least one of a hole injection material, a hole transport material, an electron transport material, an electron injection material, an electron blocking material, a hole blocking material, a light-emitting material, and a host material.

21. A device, comprising at least one functional layer and two electrodes, wherein the at least one functional layer is located between the two electrodes, and the at least one functional layer comprises at least one organic compound according to any one of claims 1 to 11, or the organic compound prepared by using the method for preparing the organic compound according to claim 12 or 13, or at least one polymer according to any one of claims 14 to 18, or at least one composition according to claim 19, or at least one mixture according to claim 20.

22. The device according to claim 21, wherein the device is an organic light-emitting device, the at least one functional layer comprises a light-emitting layer, and a guest material of the light-emitting layer comprises at least one organic compound according to any one of claims 1 to 11, or the organic compound prepared by using the method for preparing the organic compound according to claim 12 or 13, or at least one polymer according to any one of claims 14 to 18.

23. A display apparatus, comprising a control system and at least one device according to claim 21 or 22, wherein the control system is configured to control the device.

24. An electronic device, comprising a housing and the display apparatus according to claim 23 disposed on the housing.
